# EUROPEAN PATENT APPLICATION

(11) **EP 4 066 842 A1**
(43) Date of publication of application: **05.10.2022**
(21) Application number: 20894563.4
(22) Date of filing: 29.11.2020
(51) Int. Cl.: A61K 35/17, C12N 5/10, C07K 19/00, A61P 35/00

(54) **APPLICATION OF CAR T-CELLS IN PREPARING DRUG FOR TREATING CANCER**

(30) Priority: 29.11.2019 CN 201911201134; 29.11.2019 CN 201911202857
(71) Applicant: Suzhou Nova Therapeutics Co. Ltd, Suzhou, Jiangsu 215000 (CN)
(72) Inventor: WANG, Feng, Suzhou, Jiangsu 215000 (CN); GUO, Hui, Suzhou, Jiangsu 215000 (CN); LIU, Yan, Suzhou, Jiangsu 215000 (CN); ZHANG, Jay, Suzhou, Jiangsu 215000 (CN)
(74) Representative: Biggi, Cristina
(86) International application number: PCT/CN2020/132559
(87) International publication number: WO 2021/104511

(57) **Abstract**

Provided is an application of a chimeric antigen receptor (CAR)-modified T (CART) cells in preparing drugs for cancer treatment, the CART cells contain an artificially-introduced costimulatory signal transduction domain, and the CART cell does not contain an artificially-introduced first signal transduction domain.

## Description

### Technical Field

The present disclosure belongs to the field of cell therapies, and particularly relates to application of chimeric antigen receptor (CAR)-modified T (CART) cells in preparing drugs for cancer treatment, the CART cells contain artificially-introduced costimulatory signal transduction domains and do not contain an artificially-introduced activating signal first signal domain.

### Background

Chimeric antigen receptor T cells (CART cells) therapy is currently one of the hottest fields of anti-tumor immunotherapy, and is also a major breakthrough in medical immunology in the past decade. CART cells are generally made from patient-derived T-lymphocytes, which are reengineered in the laboratory to express a tumor antigen-recognizing chimeric receptor together with costimulatory molecules. CART cells are then expanded in vitro and infused back to the patients for tumor targeting and attacking.

Since the development of the first generation of CAR-T in 1989, CAR-T-based cancer immunotherapy has made significant progress. In August 2017, Novartis' CAR-T cell Kymriah was approved by Food and Drug Administration (FDA) of the United States for treatment of relapsed or refractory acute lymphoblastic leukemia (ALL) in patients aged up to 25 years, becoming the world's first autologous cell therapy and marking a milestone in the development history of the CAR-T cell therapy. Two months later, Yescarta, the second CAR-T therapy developed by Kite Pharma Company (acquired by Gilead), was also approved for marketing. It was the first CART therapy approved for the treatment of specific types of non-Hodgkin lymphoma (NHL).

Although the first-generation CD3ζ chain-based CART cells exhibit anti-tumor cytotoxicity by T cell activation, the lack of costimulatory signal, the poor in vivo persistence, and limited secretion of cytokines dampened the long-term anti-tumor efficacy. The second-generation CAR incorporates intracellular domain of a costimulatory signal molecules (CD28 or 4-1BB) on the basis of the first-generation CAR, providing two signals for T cell activation., The co-stimulation of B7/CD28 or 4-1BBL/CD137 in the intracellular signal causes continuous proliferation of T lymphocytes, elevated secretion of IL-2 and IFN-γ, resulting in increased in vivo persistence as well as enhanced anti-tumor efficacy [Dotti G. et al. CD28 costimulation improves expansion and persistence of chimeric antigen receptor modified T cells in lymphoma patients. J Clin Invest, 2011, 121(5): 1822-1826.]. Based on the second-generation CAR, the third-generation CAR further incorporates an additional intracellular domain of costimulatory signal molecule, for example a secondary signal molecule 4-1BB fused between costimulatory domain CD28 and an ITAM signal chain. The third-generation CAR-engineered T cells exhibit better effector function and in vivo persistence. Different from the previous three generations, the fourth generation is additionally engineered to secrete a transgenic cytokine like IL-12 on the basis of the second generation, also known as T cells redirected for antigen-unrestricted cytokine-initiated killing (TRUCKs for short). TRUCKs not only enhance T cell activation, but also attract and activate innate immune cells, thereby destroying antigen-negative cancer cells. TRUCKs may also be used for therapy of viral infections, metabolic diseases, autoimmune diseases and the like.

The CART cells that are disclosed so far all contain an artificially introduced CD3-mediated first signal. From the second-generation CAR to the fourth-generation CAR, two signals required for T cell activation are combined, thereby bypassing the obstacle that T cells are unable to activate due to the lack of second signal such as B7 on tumor cells, greatly enhancing T cell activation, proliferation, killing ability, and therapeutic efficacy. However, increased costimulatory signals in CAR may reduce the threshold required for effector cell activation, leading to the activation of genetically modified T lymphocytes even in conditions of a low level of antigens or even without antigen triggering, which could result in the release of high level cytokines (cytokine storm). In order to solve the risk, researchers introduce suicide systems such as HSV-TK or CD20-Rituximab into the CAR-modified T cells as a break to avoid excessive proliferation of T cells. However, the "waterfall" effect triggered by the off-target activation of CART cells is so fast that these suicide systems may not be able to work in time. Although the scheme of reducing the infusion dosage of CART cells reduced the risk to a certain extent, it also leads to a decline in therapeutic efficacy. Therefore, there is an urgent need in the field to optimize the components of the CAR system to improve the clinical safety of CAR-mediated adoptive cell therapy for tumors.

### Summary

It is generally acknowledged that T cell activation requires the stimulation of two signals, namely two signals related to T cell activation. Among them, the TCR-CD3 complex on the surface of T cells binds to the antigen peptide-major histocompatibility complex (MHC) molecule to provide the first signal of T cell activation, while the costimulatory molecule (such as CD28) on the surface of T cells binds to its corresponding ligand (such as B7), providing the second signal for T cell activation, which is essential for T cell proliferation and survival. Other costimulatory molecules such as 4-1BB, OX40, GITR, CD40, ICOS, CD152 (CTLA4), CD223(LAG3), CD273(PD-L2), CD274(PD-L1), NKG2C, NKG2D, SLAMF7, and B7-H3 are also considered to have similar functions to that of CD28. The prevailing view that the loss or decreased expression of the first signal stimulation source (such as MHC) molecules on tumor cells may not effectively provide signals required for T cell activation, resulting in the inability of T cells to effectively activate and proliferate.

Tumor marker recognizing TCR-CD3 complexes naturally occurring on the surface of tumor infiltrating T cells would provide natural primary signal (signal 1) for T cell activation, while these T cells cannot always exert killing activity efficiently due to the immunosuppressive microenvironment within tumor tissues. These tumor-recognizing T cells are also found in peripheral blood. Current CARs of CART cells all contain artificially introduced first signal molecules such as scFv-CD3zeta, and the artificially introduced costimulatory molecule signal domains such as scFv-4-1BB-CD3zeta or scFv-CD28. These tumor markers recognizing CART cells are activated by the first and the second signal, thereby exerting efficient killing activity, meanwhile, the proliferation and survival of the CART cells will further exert the killing activity. However, this mechanism may also cause CART to kill other normal cells expressing the same marker.

It is found by the inventors that: when T cells from peripheral blood of healthy donors are artificially incorporated with CAR containing a costimulatory signal conduction domain (4-1 BB) while without a first signal conduction domain (CD3zeta), these signal 1 lacking CART cells could secret T cell activating factors such as IL-2 and IFN-γ upon co-incubation with tumor cells in vitro, which further support the proliferation and survival of CART cells, indicating that the costimulatory signal of CART cells are activated. In a further experiment with CART cells harboring inducible cytokines expression element, not only secrete cell activating factors like IL-2 and IFN- γ , but also the inducible cytokines were detected in the supernatant. It is inferred that tumor recognition molecules on CAR may activate the costimulatory signal after binding to the tumor antigen, namely the activation of the CART costimulatory signal may be independent of the existence of the first signal.

T cells in peripheral blood from cancer patients contain a small population of T cells that naturally recognize tumor antigens, some of which are derived from tumor infiltrating lymphocytes (TIL). It is further found by the inventors that, when the peripheral blood T cells are artificially engineered with CAR containing the costimulatory signal conduction domain while without the artificially introduced first signal conduction domain, the natural TCR-CD3 complex on the surface of the TIL-derived CART cells specifically recognizes tumor cells (specificity and safety), meanwhile, tumor recognition molecules on CAR could also bind to tumor cells, leading to activation of downstream engineered costimulatory signals, which produce efficient tumor killing and maintain the proliferation and survival of CART cells (effectiveness) in cooperation with the first signal. This design effectively solves the problem of hyperactivation with traditional CART cells (all containing the first signal of T cell activation, namely all or part of the signal domain of CD3, such as CD3zeta, and tumor recognition molecules carried by CAR, such as scFv) to normal tissues which express low levels of tumor related antigen due to the persistence of the first signal and the second signal. In addition to that, although most circulating CART cells that do not recognize the tumor antigens do not possess the first signal required for T cell activation, the tumor recognition molecules carried on its CAR bind to the tumor cells and thus activate the artificially incorporated costimulatory signal. When activated, these CART cells secrete T cell activating factors such as IL-2 and IFN-r which maintain the proliferation and survival of CART cells and further induce the expression of downstream secreted polypeptides (such as aPD-L1 scfv), which could further exert immunomodulatory functions (such as breaking immune tolerance).

Therefore, CART cells of the present disclosure have very important clinical application values: 1) As a delivery carrier that specifically targeting the tumor antigens, CART cells expressed secretory polypeptides to exert immune modulation in the tumor microenvironment; and 2) They effectively solves the overactive immune response of traditional CART cells (all containing the first signal of T cell activation, namely all or part of the signal domain of CD3, such as CD3zeta, and tumor recognition molecules carried by CAR, such as scFv) to normal tissues which express low level of tumor related antigens due to the persistence of the first signal and the second signal..

In one aspect, the present disclosure provides the application of CAR-modified T cells in preparing drugs for cancer treatment. The CART cells include a first CAR, the first CAR includes an antigen binding domain, a transmembrane domain, and an intracellular domain, wherein the intracellular domain is an artificially incorporated costimulatory signal conduction domain, wherein the CART cell does not contain the artificially incorporated first signal conduction domain.

In some embodiments, the T cells are selected from one or more subsets of specific T cells, such as tumor infiltrating lymphocytes (TIL), cytotoxic T lymphocytes (CTL), natural killer T (NKT) cells, δ T cells, or regulatory T cells.

In some embodiments, the CAR includes an antigen binding domain, a transmembrane domain, and an intracellular domain, herein the intracellular domain is the artificially incorporated costimulatory signal transduction domain. In some embodiments, the artificially incorporated costimulatory signal transduction domain is selected from the intracellular signaling domains of one or more costimulatory proteins: 4-1BB (CD137), CD28, CD27, CD30, OX40 , GITR, CD40, ICOS, BAFFR, HVEM, ICAM-1, LCK, CD278(ICOS), CD150(SLAMF1), CD152(CTLA4), CD223(LAG3), CD270(HVEM), CD273(PD-L2), CD274(PD-L1), LAT, NKD2CSLP76, TRIM, ZAP70, DAP-10, DAP-12, LFA-1, CD2, CDS, CD7, CD287, LIGHT, NKG2C, NKG2D, SLAMF7, NKp80, NKp30, NKp44, NKp46, CD160, B7-H3 or ligands that specifically bind to CD83 and the like.

In some embodiments, the artificially introduced costimulatory signal conduction domain is selected from the intracellular signal transduction domain of costimulatory protein 4-1BB. In some embodiments, the artificially introduced costimulatory signal conduction domain is selected from the intracellular signal conduction domain of costimulatory protein OX40. In some embodiments, the artificially introduced costimulatory signal conduction domain is selected from intracellular signal transduction domains of 4-1BB and OX40. In some embodiments, the artificially introduced costimulatory signal conduction domain is selected from intracellular signal conduction domains of 4-1BB and CD28. In some embodiments, the artificially introduced costimulatory signal conduction domain is selected from intracellular signal conduction domains of 4-1BB and LAG3. In some embodiments, the artificially introduced costimulatory signal conduction domain is selected from intracellular signal conduction domains of 4-1BB, CD28 and OX40.

In some embodiments, the intracellular signal transduction domain of 4-1BB has a nucleic acid sequence as shown in SEQ ID NO: 13 or an amino acid sequence as shown in SEQ ID NO: 14. In some embodiments, the intracellular signal transduction domain of OX40 has a nucleic acid sequence as shown in SEQ ID NO: 60 or an amino acid sequence as shown in SEQ ID NO: 61. In some embodiments, the intracellular signal transduction domain of CD28 has a nucleic acid sequence as shown in SEQ ID NO: 145 or an amino acid sequence as shown in SEQ ID NO: 146. In some embodiments, the intracellular signal transduction domain of LAG3 has a nucleic acid sequence as shown in SEQ ID NO: 147 or an amino acid sequence as shown in SEQ ID NO: 148.

In some embodiments, the transmembrane domain of the first CAR is derived from the transmembrane region of CD8. In some embodiments, the transmembrane region of CD8 has a nucleic acid sequence as shown in SEQ ID NO: 9 or an amino acid sequence as shown in SEQ ID NO: 10.

In some embodiments, the antigen binding domain of the first CAR binds to disease-associated cell surface antigens.

In some embodiments, the disease-associated cell surface antigen is selected from immune checkpoint proteins.

In some embodiments, the immune checkpoint protein is selected from: PD-1, PD-L1, CTLA-4, LAG-3, OX40, CD28, CD40, CD47, CD70, CD80, CD122, GTIR, A2AR, B7-H3(CD276), B7-H4, IDO, KIR, Tim-3 or 4-1BB(CD137).

In some embodiments, the immune checkpoint protein is PD-1. In some embodiments, the immune checkpoint protein is PD-L1.

In some embodiments, the antigen binding domain targeting the immune checkpoint protein PD-1 has HCDR1 of an amino acid sequence as shown in SEQ ID NO: 62, HCDR2 of an amino acid sequence as shown in SEQ ID NO: 63, HCDR3 of an amino acid sequence as shown in SEQ ID NO: 64, LCDR1 of an amino acid sequence as shown in SEQ ID NO: 65, LCDR2 of an amino acid sequence as shown in SEQ ID NO: 66, and LCDR3 of an amino acid sequence as shown in SEQ ID NO: 67. In some embodiments, the antigen binding domain targeting the immune checkpoint protein PD-1 has a heavy chain variable region (VH) as shown in SEQ ID NO: 109 and a light chain variable region (VL) as shown in SEQ ID NO: 110. In some embodiments, the antigen binding domain that binds to the immune checkpoint protein PD-1 has a nucleic acid sequence as shown in SEQ ID NO: 25 or an amino acid sequence as shown in SEQ ID NO: 26.

In some embodiments, the antigen binding domain targeting the immune checkpoint protein PD-L1 has HCDR1 of an amino acid sequence as shown in SEQ ID NO: 68, HCDR2 of an amino acid sequence as shown in SEQ ID NO: 69, HCDR3 of an amino acid sequence as shown in SEQ ID NO: 70, LCDR1 of an amino acid sequence as shown in SEQ ID NO: 71, LCDR2 of an amino acid sequence as shown in SEQ ID NO: 72, and LCDR3 of an amino acid sequence as shown in SEQ ID NO: 73. In some embodiments, the antigen binding domain targeting the immune checkpoint protein PD-1 has VH as shown in SEQ ID NO: 111 and VL as shown in SEQ ID NO: 112. In some embodiments, the antigen binding domain that binds to the immune checkpoint protein PD-L1 has a nucleic acid sequence as shown in SEQ ID NO: 27 or an amino acid sequence as shown in SEQ ID NO: 28.

In some embodiments, the disease-associated cell surface antigen is selected from tumor antigens. In some embodiments, the tumor antigen is selected from: epidermal growth factor receptor family (EGFR, HER2, HER3, and HER4), PD-1, PD-L1, CTLA-4, 4-1BB(CD137), OX40, CD28, CD40, CD47, CD70, CD80, CD122, GTIR, A2AR, B7-H3(CD276), B7-H4, IDO, KIR, Tim-3, NY-ESO-1, GPC3, CLL-1, BCMA, mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, and MUC20), CD19, CD20, CD22, CD30, CD33, CD52, chemistry chemokine receptor family (CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, and CXCR6), PSMA, CEA, HDAC6, EpCAM, Mesothelin, TERT, TLR, TLR9, TLR4, CD33, GITR, Survivin, CD123, TIGIT, TIM-3, CD73, fibroblast growth factor receptor (FGFR), vascular endothelial growth factor receptor (FLT1, KDR/Flk-1, and VEGFR-3), hepatocyte growth factor receptor (HGFR), nerve growth factor receptor (NGFR), insulin-like growth factor receptor (IGFR), platelet-derived growth factor receptor (PDGFR) or hormone receptor (melanocortin 1 receptor (MC1R, and MSHR)), and the like.

In some embodiments, the tumor antigen is CD19. In some embodiments, the tumor antigen is GPC3. In some embodiments, the tumor antigen is PSMA. In some embodiments, the tumor antigen is MUC16. In some embodiments, the tumor antigen is FAP.

In some embodiments, the antigen binding domain targeting the tumor antigen CD19 has HCDR1 of an amino acid sequence as shown in SEQ ID NO: 86, HCDR2 of an amino acid sequence as shown in SEQ ID NO: 87, HCDR3 of an amino acid sequence as shown in SEQ ID NO: 88, LCDR1 of an amino acid sequence as shown in SEQ ID NO: 89, LCDR2 of an amino acid sequence as shown in SEQ ID NO: 90, and LCDR3 of an amino acid sequence as shown in SEQ ID NO: 91. In some embodiments, the antigen binding domain targeting the tumor antigen CD19 has VH as shown in SEQ ID NO: 113 and VL as shown in SEQ ID NO: 114. In some embodiments, the antigen binding domain targeting the tumor antigen CD19 has a nucleic acid sequence as shown in SEQ ID NO: 52 or an amino acid sequence as shown in SEQ ID NO: 53.

In some embodiments, the antigen binding domain targeting the tumor antigen GPC3 has HCDR1 of an amino acid sequence as shown in SEQ ID NO: 80, HCDR2 of an amino acid sequence as shown in SEQ ID NO: 81, HCDR3 of an amino acid sequence as shown in SEQ ID NO: 82, LCDR1 of an amino acid sequence as shown in SEQ ID NO: 83, LCDR2 of an amino acid sequence as shown in SEQ ID NO: 84, and LCDR3 of an amino acid sequence as shown in SEQ ID NO: 85. In some embodiments, the antigen binding domain targeting the tumor antigen GPC3 has VH as shown in SEQ ID NO: 105 and VL as shown in SEQ ID NO: 106. In some embodiments, the antigen binding domain targeting the tumor antigen GPC3 has a nucleic acid sequence as shown in SEQ ID NO: 23 or an amino acid sequence as shown in SEQ ID NO: 24. In some embodiments, the CAR targeting the tumor antigen GPC3 has a nucleic acid sequence as shown in SEQ ID NO: 41 or an amino acid sequence as shown in SEQ ID NO:42.

In some embodiments, the antigen binding domain targeting the tumor antigen PSMA has HCDR1 of an amino acid sequence as shown in SEQ ID NO: 74, HCDR2 of an amino acid sequence as shown in SEQ ID NO: 75, HCDR3 of an amino acid sequence as shown in SEQ ID NO: 76, LCDR1 of an amino acid sequence as shown in SEQ ID NO: 77, LCDR2 of an amino acid sequence as shown in SEQ ID NO: 78, and LCDR3 of an amino acid sequence as shown in SEQ ID NO: 79. In some embodiments, the antigen binding domain targeting the tumor antigen PSMA has VH as shown in SEQ ID NO: 107 and VL as shown in SEQ ID NO: 108. In some embodiments, the antigen binding domain targeting the tumor antigen PSMA has a nucleic acid sequence as shown in SEQ ID NO: 32 or an amino acid sequence as shown in SEQ ID NO: 33. In some embodiments, the CAR targeting the tumor antigen GPC3 has the nucleic acid sequence as shown in SEQ ID NO: 32 or the amino acid sequence as shown in SEQ ID NO: 33.

In some embodiments, the antigen binding domain targeting the tumor antigen MUC16 has HCDR1 of an amino acid sequence as shown in SEQ ID NO: 93, HCDR2 of an amino acid sequence as shown in SEQ ID NO: 94, HCDR3 of an amino acid sequence as shown in SEQ ID NO: 95, LCDR1 of an amino acid sequence as shown in SEQ ID NO: 96, LCDR2 of an amino acid sequence as shown in SEQ ID NO: 97, and LCDR3 of an amino acid sequence as shown in SEQ ID NO: 98. In some embodiments, the antigen binding domain targeting the tumor antigen MUC16 has VH as shown in SEQ ID NO: 115 and VL as shown in SEQ ID NO: 116. In some embodiments, the antigen binding domain targeting the tumor antigen MUC16 has a nucleic acid sequence as shown in SEQ ID NO: 56 or an amino acid sequence as shown in SEQ ID NO: 57.

In some embodiments, the antigen binding domain targeting the tumor antigen FAP has HCDR1 of an amino acid sequence as shown in SEQ ID NO: 99, HCDR2 of an amino acid sequence as shown in SEQ ID NO: 100, HCDR3 of an amino acid sequence as shown in SEQ ID NO: 101, LCDR1 of an amino acid sequence as shown in SEQ ID NO: 102, LCDR2 of an amino acid sequence as shown in SEQ ID NO: 103, and LCDR3 of an amino acid sequence as shown in SEQ ID NO: 104. In some embodiments, the antigen binding domain targeting the tumor antigen FAP has VH as shown in SEQ ID NO: 117 and VL as shown in SEQ ID NO: 118. In some embodiments, the antigen binding domain targeting the tumor antigen FAP has a nucleic acid sequence as shown in SEQ ID NO: 58 or an amino acid sequence as shown in SEQ ID NO: 59.

In some embodiments, the antigen binding domain targeting the tumor antigen EGFR has HCDR1 of an amino acid sequence as shown in SEQ ID NO: 133, HCDR2 of an amino acid sequence as shown in SEQ ID NO: 134, HCDR3 of an amino acid sequence as shown in SEQ ID NO: 135, LCDR1 of an amino acid sequence as shown in SEQ ID NO: 136, LCDR2 of an amino acid sequence as shown in SEQ ID NO: 137, and LCDR3 of an amino acid sequence as shown in SEQ ID NO: 138. In some embodiments, the antigen binding domain targeting the tumor antigen EGFR has VH as shown in SEQ ID NO: 119 and VL as shown in SEQ ID NO: 120.

In some embodiments, the antigen binding domain targeting the tumor antigen HER2 has HCDR1 of an amino acid sequence as shown in SEQ ID NO: 139, HCDR2 of an amino acid sequence as shown in SEQ ID NO: 140, HCDR3 of an amino acid sequence as shown in SEQ ID NO: 141, LCDR1 of an amino acid sequence as shown in SEQ ID NO: 142, LCDR2 of an amino acid sequence as shown in SEQ ID NO: 143, and LCDR3 of an amino acid sequence as shown in SEQ ID NO: 144. In some embodiments, the antigen binding domain targeting the tumor antigen HER2 has VH as shown in SEQ ID NO: 121 and VL as shown in SEQ ID NO: 122.

In some embodiments, the antigen binding domain targeting the tumor antigen CD3 has HCDR1 of an amino acid sequence as shown in SEQ ID NO: 127, HCDR2 of an amino acid sequence as shown in SEQ ID NO: 128, HCDR3 of an amino acid sequence as shown in SEQ ID NO: 129, LCDR1 of an amino acid sequence as shown in SEQ ID NO: 130, LCDR2 of an amino acid sequence as shown in SEQ ID NO: 131, and LCDR3 of an amino acid sequence as shown in SEQ ID NO: 132. In some embodiments, the antigen binding domain targeting the tumor antigen CD3 has VH as shown in SEQ ID NO: 123 and VL as shown in SEQ ID NO: 124.

In some embodiments, the CAR-modified T cell of the present disclosure further express a second CAR, wherein the second CAR includes an antigen binding domain and a transmembrane domain.

In some embodiments, the antigen binding domain of the second CAR binds to a disease-associated cell surface antigen. In some embodiments, the disease-associated cell surface antigen is a tumor antigen. In some embodiments, the tumor antigen is selected from: epidermal growth factor receptor family (EGFR, HER2, HER3, and HER4), PD-1, PD-L1, CTLA-4, 4-1BB(CD137), OX40, CD28, CD40, CD47, CD70, CD80, CD122, GTIR, A2AR, B7-H3(CD276), B7-H4, IDO, KIR, Tim-3, NY-ESO-1, GPC3, CLL-1, BCMA, mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, and MUC20), CD19, CD20, CD22, CD30, CD33, CD52, chemistry chemokine receptor family (CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, and CXCR6), PSMA, CEA, HDAC6, EpCAM, Mesothelin, TERT, TLR, TLR9, TLR4, CD33, GITR, Survivin, CD123, TIGIT, TIM-3, CD73, FGFR, vascular endothelial growth factor receptor (FLT1, KDR/Flk-1, and VEGFR-3), HGFR, NGFR, IGFR, PDGFR or hormone receptor (melanocortin 1 receptor (MC1R, and MSHR)). In some embodiments, the chimeric antigen receptor 2 binds to the tumor antigen EGFR.

In some embodiments, the CAR-modified T cells of the present disclosure further express secreted polypeptides selected from but not limited to: one or two of immune function modulatory factors, antibodies specifically targeting the tumor antigens, and antibodies specifically targeting the immune checkpoints.

In some embodiments, the secreted polypeptide is an immune function regulatory factor. In some embodiments, the immune function regulatory factor is selected from IL-2, IL-12, IL-7, IL-15, IL-18, IL-21, IL-24, 4-1BBL, PD-1 extracellular region, PD-L1 extracellular region, CCL19, MIP-1α, GM-CSF, IFN-α, IFN-β, IFN-γ, TNF-α, M-CSF, TGF-β or TRAIL, etc.

In some embodiments, the secreted polypeptide is an immune function regulatory factor IL-2. In some embodiments, the secreted polypeptide is an immune function regulatory factor IL-12. In some embodiments, the secreted polypeptide is an immune function regulatory factor IL-15. In some embodiments, the secreted polypeptide is an immune function regulatory factor IL-18. In some embodiments, the secreted polypeptide is an immune function regulatory factor PD-1 mutant polypeptide. In some embodiments, the immune function regulatory factor IL-2 has a nucleic acid sequence as shown in SEQ ID NO: 15 or an amino acid sequence as shown in SEQ ID NO: 16. In some embodiments, the immune function regulatory factor IL-12 has a nucleic acid sequence as shown in SEQ ID NO: 17 or an amino acid sequence as shown in SEQ ID NO: 18. In some embodiments, the immune function regulatory factor IL-15 has a nucleic acid sequence as shown in SEQ ID NO: 19 or an amino acid sequence as shown in SEQ ID NO: 20. In some embodiments, the immune function regulatory factor IL-2 has a nucleic acid sequence as shown in SEQ ID NO: 21 or an amino acid sequence as shown in SEQ ID NO:22.

In some embodiments, the secreted polypeptide is an antibody specifically targeting a tumor antigen. In some embodiments, the tumor antigen is selected from: epidermal growth factor receptor family (EGFR, HER2, HER3, and HER4), PD-1, PD-L1, CTLA-4, 4-1BB(CD137), OX40, CD28, CD40, CD47, CD70, CD80, CD122, GTIR, A2AR, B7-H3(CD276), B7-H4, IDO, KIR, Tim-3, NY-ESO-1, GPC3, CLL-1, BCMA, mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, and MUC20), CD19, CD20, CD22, CD30, CD33, CD52, chemistry chemokine receptor family (CCR1, CCR2, CCR3, CCR4, CCR5, CCR6, CCR7, CCR8, CCR9, CCR10, CCL27, CCL28, CX3CR1, CXCR1, CXCR2, CXCR3, CXCR4, CXCR5, and CXCR6), PSMA, CEA, HDAC6, EpCAM, Mesothelin, TERT, TLR, TLR9, TLR4, CD33, GITR, Survivin, CD123, TIGIT, TIM-3, CD73, FGFR, vascular endothelial growth factor receptor (FLT1, KDR/Flk-1, and VEGFR-3), HGFR, NGFR, IGFR, PDGFR or hormone receptor (melanocortin 1 receptor (MC1R, and MSHR)) and the like. In some embodiments, the immune checkpoint protein is selected from: PD-1, PD-L1, CTLA-4, LAG-3, OX40, CD28, CD40, CD47, CD70, CD80, CD122, GTIR, A2AR, B7-H3 (CD276), B7-H4, IDO, KIR, Tim-3, or 4-1BB (CD137). In some embodiments, the secreted polypeptide is a bispecific antibody. In some embodiments, the secreted polypeptide is a bispecific antibody targeting FAP and CD3. In some embodiments, the secreted polypeptide targeting FAP and CD3 has an amino acid sequence as shown in SEQ ID NO: 125. In some embodiments, the secreted polypeptide is a bispecific antibody targeting HER2 and CD3. In some embodiments, the secreted polypeptide targeting HER2 and CD3 has an amino acid sequence as shown in SEQ ID NO: 126. In some embodiments, the secreted polypeptide is a bispecific antibody targeting CD19 and CD3.

In some embodiments, the secreted polypeptide is an antibody specifically targeting an immune checkpoint. In some embodiments, the immune checkpoint protein is selected from: PD-1, PD-L1, CTLA-4, LAG-3, OX40, CD28, CD40, CD47, CD70, CD80, CD122, GTIR, A2AR, B7-H3(CD276), B7-H4, IDO, KIR, Tim-3 or 4-1BB(CD137). In some embodiments, the immune checkpoint protein is PD-1. In some embodiments, the immune checkpoint protein is PD-L1. In some embodiments, the antibody targeting the immune checkpoint protein PD-1 has a nucleic acid sequence as shown in SEQ ID NO: 25 or an amino acid sequence as shown in SEQ ID NO: 26. In some embodiments, the antigen binding domain that binds to the immune checkpoint protein PD-L1 has a nucleic acid sequence as shown in SEQ ID NO: 27 or an amino acid sequence as shown in SEQ ID NO: 28.

In some embodiments, the secreted polypeptide is constitutively expressed. In some embodiments, the constitutively expressed secreted polypeptide is on polypeptide chain of the first or second CAR. In some embodiments, the constitutively expressed secreted polypeptide is linked to the first or second CAR by a self-cleaving peptide. In some embodiments, the self-cleaving peptide is selected from P2A or T2A. In some embodiments, the self-cleaving peptide P2A has a nucleic acid sequence as shown in SEQ ID NO: 29 or an amino acid sequence as shown in SEQ ID NO: 30.

In some embodiments, the first CAR targets a tumor antigen, and the constitutively expressed secreted polypeptide is an immune function regulatory factor. In some embodiments, the first CAR targets the tumor antigen PSMA, and the constitutively expressed secreted polypeptide is immune function regulatory factor IL-12. In some embodiments, the CAR targeting PSMA and constitutively expressing IL-12 has a nucleic acid sequence as shown in SEQ ID NO: 40.

In some embodiments, the first CAR targets a tumor antigen, and the constitutively expressed secreted polypeptide is an antibody specifically targeting an immune checkpoint. In some embodiments, the first CAR targets tumor antigen GPC3, and the constitutively expressed secreted polypeptide is an antibody specifically targeting immune checkpoint PD-1. In some embodiments, the CAR targeting GPC3 and expressing the PD-1 antibody has a nucleic acid sequence as shown in SEQ ID NO: 47. In some embodiments, the CAR targeting GPC3 and expressing the PD-1 antibody has a nucleic acid sequence as shown in SEQ ID NO: 48.

In some embodiments, the first CAR targets a tumor antigen, and the constitutively expressed secreted polypeptides include an immune function regulatory factor and an antibody specifically targeting an immune checkpoint protein. In some embodiments, the first CAR targets tumor antigen GPC3, and the constitutively expressed secreted polypeptide includes immune function regulatory factor IL-12 and an antibody specifically targeting immune checkpoint PD-1. In some embodiments, the CAR targeting GPC3 and expressing IL-12 and specifically targeting the PD-1 antibody has a nucleic acid sequence as shown in SEQ ID NO: 50.

In some embodiments, the secreted polypeptide is inducibly expressed. In some embodiments, the inducibly secretory polypeptide is expressed after activation by an inducible promoter. In some embodiments, the inducible promoter is 6XNFAT. In some embodiments, the 6XNFAT promoter has a sequence as shown in SEQ ID NO. 3. In some embodiments, the inducible promoter is NF-κb responsive promoter. Further, the NF-κb responsive promoter includes an NF-κb response element and a basal promoter. In some embodiments, the NF-κb response element has a sequence as shown in one of SEQ ID NO. 149 to SEQ ID NO. 152. In some embodiments, the NF-κb basal promoter has a sequence as shown in one of SEQ ID NO. 153 to SEQ ID NO. 155. In some embodiments, the NF-κb responsive promoter has a sequence as shown in SEQ ID NO. 156.

In some embodiments, the secreted polypeptide is on a different polypeptide chain from the first or second CAR. In some embodiments, the secreted polypeptide is on the first or second CAR polypeptide. In some embodiments, the secreted polypeptide is linked to the CAR by a self-cleaving peptide. In some embodiments, the self-cleaving peptide is selected from P2A or T2A. In some embodiments, the self-cleaving peptide P2A has a nucleic acid sequence as shown in SEQ ID NO: 29 or an amino acid sequence as shown in SEQ ID NO: 30. In some embodiments, the coding sequence of the inducibly expressed secretory polypeptide and the first or second CAR are linked by a promoter and a signal peptide coding sequence.

In some embodiments, the first CAR targets a tumor antigen, and the inducibly expressed secretory polypeptide is an immune function regulatory factor.

In some embodiments, the first CAR targets tumor antigen PSMA, and the inducibly expressed secreted polypeptide is immune function regulatory factor IL-2. In some embodiments, the first CAR targeting PSMA with inducibly expressed IL-2 has a nucleic acid sequence as shown in SEQ ID NO: 39. In some embodiments, the first CAR targets tumor antigen PSMA, and the inducibly expressed secreted polypeptide is immune function regulatory factor IL-12. In some embodiments, the CAR targeting PSMA with inducibly expressed IL-12 has a nucleic acid sequence as shown in SEQ ID NO: 34. In some embodiments, the first CAR targets tumor antigen PSMA, and the inducibly expressed secreted polypeptide is immune function regulatory factor IL-15. In some embodiments, the CAR targeting PSMA with inducibly expressed IL-15 has a nucleic acid sequence as shown in SEQ ID NO: 36. In some embodiments, the first CAR targets tumor antigen PSMA, and the inducibly expressed secreted polypeptide is immune function regulatory factor IL-18. In some embodiments, the CAR targeting PSMA with inducibly expressed IL-18 has a nucleic acid sequence as shown in SEQ ID NO: 36. In some embodiments, the first CAR targets tumor antigen GPC3, and the inducibly expressed secreted polypeptide is immune function regulatory factor IL-2. In some embodiments, the CAR targeting PSMA with inducibly expressed IL-2 has a nucleic acid sequence as shown in SEQ ID NO: 49.

In some embodiments, the first CAR targets a tumor antigen, and the inducibly expressed secreted polypeptide is an antibody that specifically binds to an immune checkpoint protein.

In some embodiments, the first CAR targets tumor antigen GPC3, and the inducibly expressed secretory polypeptide is an antibody that specifically binds to immune checkpoint protein PD-1. In some embodiments, the CAR targeting GPC3 with inducibly expressed the PD-1 antibody has a nucleic acid sequence as shown in SEQ ID NO: 43. In some embodiments, the first CAR targets tumor antigen GPC3, and the inducibly expressed secreted polypeptide is an antibody that specifically binds to immune checkpoint protein PD-L1. In some embodiments, the CAR targeting GPC3 with inducibly expressed the PD-L1 antibody has a nucleic acid sequence as shown in SEQ ID NO: 45.

In some embodiments, the first CAR targets a tumor antigen, and the inducibly expressed secreted polypeptide includes an immune function regulatory factor and an antibody that specifically binds to an immune checkpoint protein.

In some embodiments, the first CAR targets tumor antigen GPC3, and the inducibly expressed secreted polypeptide includes immune function regulatory factor IL-12 and an antibody that specifically targets immune checkpoint PD-1. In some embodiments, the CAR targeting GPC3 with inducibly express IL-2 and PD-1 antibodies has a nucleic acid sequence as shown in SEQ ID NO: 51.

In some embodiments, CAR containing costimulatory signal domains (such as a 4-1BB intracellular costimulatory signal, intracellular signal conduction domains of 4-1BB and OX40, intracellular costimulatory signal conduction domains of 4-1BB and CD28, intracellular signal conduction domains of 4-1BB and LAG3, or intracellular signal conduction domains of 4-1BB, CD28 and OX40), tumor recognition molecule aPSMA scfv, immune function regulatory factor (IL-2, IL-12, IL-15 or IL-18) but without the first signal is artificially introduced into tumor infiltrating lymphocyte (TIL). After entering tumor tissue by the specificity of aPSMA scfv and the tissue specificity of the natural TCR-CD3 complex on the cell surface, CART cells are activated by the first signal provided by the combination of TCR-CD3 complex and antigen peptide-MHC-I molecule, and their anti-tumor effects are enhanced by the secreted immune regulatory factors (such as IL-2, IL-12, IL-15 or IL-18). Tumor recognition and binding by aPSMA scfv as well as the activation of intracellular signal conduction of the engineered costimulatory signal molecule maintain the survival of the activated CART cells.

In some embodiments, CAR containing costimulatory signal domain (such as a 4-1BB intracellular costimulatory signal domain, intracellular signal conduction domains of 4-1BB and OX40, intracellular costimulatory signal conduction domains of 4-1BB and CD28, intracellular signal conduction domains of 4-1BB and LAG3, or intracellular signal conduction domains of 4-1BB, CD28 and OX40), tumor recognition molecule aGPC3 scfv, immune function regulatory factor (IL-12, IL-15, or IL-18) and/or scfvs targeting an immune checkpoint protein but without the first signal is artificially introduced into TIL. After entering the tumor tissue by the specificity of aGPC3 scfv on its CAR and the tissue specificity of the natural TCR-CD3 complex on the cell surface, CART cells are activated by the first signal provided by the combination of TCR-CD3 complex and antigen peptide-MHC-I molecule, and their anti-tumor effects are enhanced by the secreted immune regulatory factors (such as IL-12, IL-15 or IL-18), or by the breakdown of the immune tolerance microenvironment of the tumor tissue by the secreted scfv targeting the immune checkpoint protein (such as PD-1 or PD-L1) . Tumor recognition and binding by the tumor cell recognition molecule aGPC3 scfv as well as the activation of intracellular signal conduction of the engineered costimulatory signal molecule maintain the survival of the activated CART cells.

### Brief Description of the Drawings

Drawings that constitute the present application are used to provide further understanding of the present disclosure. The exemplary embodiments and descriptions of the present disclosure thereof are used to explain the present disclosure, and do not constitute improper limitation to the present disclosure. In the drawings:
Fig. 1 shows expression of CAR on the surface of CART cells targeting PSMA.
Fig. 2A-2J show expression of CAR on the surface of CARTs cell targeting GPC-3.
Fig. 3A-3D shows levels of IL-2, IFN-γ, IL-12 and IL-18 secreted by PSMA-targeted CART effector cells after co-incubation with target cells PC3, LnCAP or 22RV1 at a ratio of 1:1. Herein Mock is PBMC cells without genetic modification, 1 is aPSMA BBZ, 2 is aPSMA BBZ+iIL-12, 3 is aPSMA BBZ+iIL-15, 4 is aPSMA BB+iIL-2, 5 is aPSMA BB+iIL-18, and 6 is aPSMA BB+IL-12.

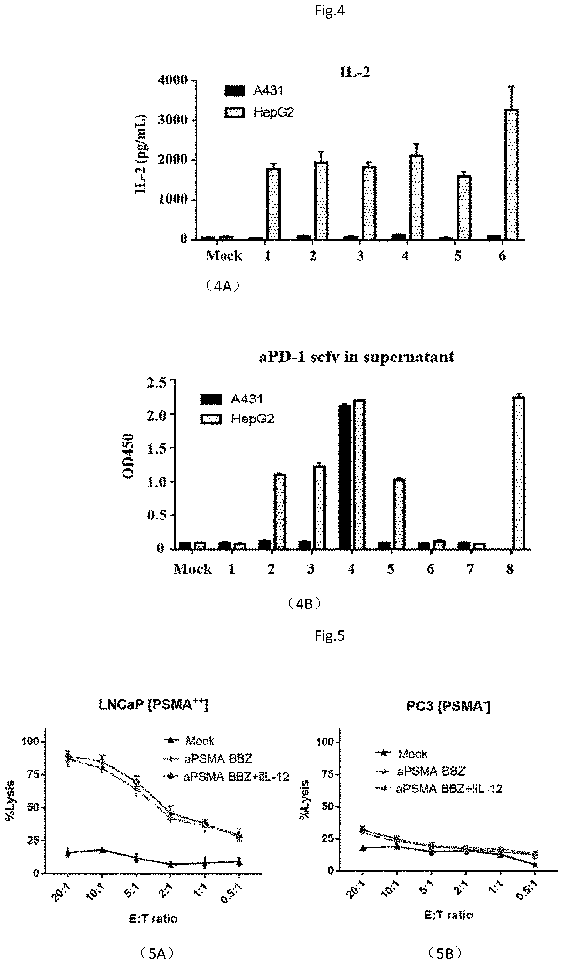

Fig. 4 shows the level of IL-2 or aPD-1 scfv secreted by GPC-3-targeted CART cells after co-incubation with target cells A431 or HepG2 at a ratio of 1:1. In Fig. 4A, Mock is PBMC cells without genetic modification, 1 is aGPC-3BBZ, 2 is aGPC-3 BB+iaPD-1, 3 is aGPC-3 BB+iaPD-L1, 4 is aGPC-3 BB-PGK+aPD-1, 5 is aGPC-3 BB-P2A+iaPD-1, and 6 is aGPC-3 BB+ilL-2; and in Fig. 4B, Mock is PBMC cells without genetic modification, 1 is aGPC-3BBZ, 2 is aGPC-3 BB+iaPD1, 3 is aGPC-3 BBZ+iaPD-1, 4 is aGPC-3 BB-P2A+aPD-1, 5 is aGPC-3 Z+iaPD-1, 6 is aGPC-3 BB+ ilL-2, 7 is aGPC-3 Z+ilL-2, and 8 is positive control nivolumab. 5 and 7 are CD3Z only CAR.
Fig. 5 shows killing activity of PMSA-targeted CART cells after co-incubation with target cells LNCaP or PC3 at different ratios, herein Mock is PBMC cells without genetic modification.
Fig. 6 shows killing activity of GPC-3-targeted CART cells after co-incubation with a target cell HepG2 or A431 at different ratios, herein Mock is PBMC cells without genetic modification.
Fig. 7 is a schematic diagram of CAR.
Fig. 8 shows the killing activity of CAR-T cells containing only costimulatory signal transduction domain with the first activation signal mediated by tumor-specific BiTE.
Fig. 9 shows the killing effect of patient derived CART cells expressing aPSMA BBZ towards tumor cells in vitro.
Fig. 10 shows the activation of NF-κb responsive promoter by TNFα, Phorbol myristate acetate (PMA) and 4-1bbL.
Fig. 11 shows that CART cells expressing aPSMA BBZ could effectively inhibit the growth ofa PSMA-positive solid tumors in mice.

### Detailed Description of the Embodiments

The present disclosure is described in detail herein by reference to the following definitions and embodiments. The contents of all patents and publications mentioned herein, including all sequences disclosed in these patents and publications, are expressly incorporated herein by reference.

The term "chimeric antigen receptor" or the term "CAR" is an artificially engineered receptor. From extracellular to intracellular, the "CAR" of the present disclosure includes: a) antigen binding domain; b) transmembrane domain; and c) intracellular costimulatory signal transduction domain., "CAR" of the present disclosure includes an artificially introduced costimulatory signal transduction domain, and does not contain an artificially introduced first signal transduction domain. The "chimeric antigen receptor-modified T (CART) cells" or "CAR-modified T cells" of the present disclosure have the following characteristics: the CART cells contain a natural, unmodified first signal transduction domain CD3zeta, and an artificially introduced costimulatory signal transduction domain, wherein the natural, unmodified first signal is provided by the natural TCR-CD3 complex on the surface of the CART cells.

The term "first signal" refers to T cell activation signal provided by the binding of the TCR-CD3 complex on the surface of the T cells to antigenic peptide-MHC molecules. Different from the first signal of traditional CART cells (the first to the fourth generation) provided by CD3zeta on CAR and TCR on T cells, the "first signal" of the chimeric antigen receptor-modified T cells of the present disclosure is provided by the natural TCR-CD3 complex on the surface of T cells.

The term "costimulatory signal" refers to the second signal provided by binding of costimulatory molecules (such as CD28) on the surface of T cell to its ligand (such as B7). The "second signal" of the chimeric antigen receptor-modified T cell of the present disclosure is provided by the artificially introduced costimulatory signal transduction domain on CAR.

The term "signal transduction domain" refers to a functional portion of a protein, and the functional portion exerts the following functions by transmitting information inside the cell: regulating cell activities by generating a second messenger or as an effector in response to such messengers.

The term "first signal transduction domain" refers to a signal transduction domain that regulates the primary activation of the TCR-CD3 complex in a stimulatory manner or in an inhibitory manner. The primary signal transduction domain that acts in the stimulatory manner may contain a signal transduction motif called immunoreceptor tyrosine-based activation motif (ITAM). Illustrative examples of ITAM containing the first signal transduction domain include, but are not limited to, those derived from FcRy, FcRβ, CD3γ, CD3ε, CD3δ, CD3zeta, CD22, CD79a, CD79b, and CD66d.

The term "intracellular signal transduction domain of costimulatory protein" may be the intracellular portion of a costimulatory protein, which may contain all the intracellular part or all the natural intracellular signal transduction domain of, or its functional fragment or derivative thereof. "Costimulatory protein" refers to some adhesion molecules on the surface of immune cells (such as T cells). These adhesion molecules bind to their ligands to activate the immune cells (such as T cells), thus enhancing the proliferation and cytokine secretion as well as persistence of the activated immune cells. "costimulatory proteins" include, but are not limited to: MHC class-I molecule, BTLA and Toll ligand receptor, and OX40, CD27, CD28, CDS, ICAM-1, LFA-1 (CD11a/CD18), ICOS(CD278), 4-1BB(CD137), GITR, BAFFR, HVEM(LIGHTR), SLAMF7, NKp80(KLRF1), NKp44, NKp30, NKp46, CD160, CD19, CD4, CD8α, CD8β, IL2Rβ, IL2Ry, IL7Rα, ITGA4, VLA1, CD49a , ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1 , ITGB7, NKG2D, NKG2C, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100( SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a and ligand that specifically binds to CD83.

The term "antigen binding domain" may have any structures as long as it binds to target antigen. The domain may include, for example, variable regions of antibody heavy and light chains (for example, 1 to 6 CDRs); a module about 35 amino acids called domain A, which is contained in Avimer (a cell membrane protein existing in vivo) (WO2004/044011 and WO2005/040229); 10Fn3 domain-containing Adnectin that binds to a protein in glycoprotein fibronectin expressed on the cell membrane (WO2002/032925); Affibody which has an IgG-binding scaffold (a triple-helix of 58 amino acids which constituting the protein A) (WO1995/001937); the designed Ankyrin repeat proteins (DARPins), which are exposed on the molecular surface of an ankyrin repeat (AR), wherein the AR has such a structure with repeat stack of subunits containing aa turn with 33 amino acid residues, two antiparallel helix, and a loop (WO2002/020565); Anticalins and the like, which are tetracyclic regions at one side of a centrally twisted barrel structure of eight antiparallel chains that are highly conserved in supporting molecule neutrophil gelatinase-associated lipocalin (NGAL) (WO2003/029462) and concave regions formed by a parallel sheet structure inside a horseshoeshaped structure formed by repeated stacking of a leucine-rich repeat (LRR) module of a variable lymphocyte receptor (VLR) (it does not have an immunoglobulin structure and is the system for acquired immunity in a jawless vertebrate such as lampreys and hagfish) (WO2008/016854). As a preferred example of the antigen binding domain of the present disclosure, it may be an antigen binding domain containing variable regions of heavy and light chains of an antibody. As an example of such an antigen binding domain, it may be "scFv (single-chain Fv)", "single-chain antibody", "Fv", "scFv2 (single-chain Fv2)", "Fab" or "F(ab')2" and the like.

The term "antibody" is used in the broadest sense as long as it exerts the desired biological activity, wherein it may include monoclonal antibodies (including full-length monoclonal antibodies), polyclonal antibodies, antibody variants, antibody fragments (including but not limited to Fab, Fab', F(ab')2, Fv fragment, scFv fragment, disulfide-linked Fvs (sdFv), Fd fragments consisting of VH and CH1 domain, linear antibodies, single domain antibodies such as sdAb (VL or VH), camelid VHH domains, multispecific antibodies or isolated CDRs or other epitope binding fragments formed by antibody fragments (for example, a bivalent fragment including two Fab fragments linked by disulfide bonds at the hinge region), multispecific antibodies (multiple specific antibodies) (for example, bispecific antibodies (dual specific antibodies), chimeric antibodies, humanized antibodies and other antibodies.

"Disease-associated cell surface antigen" of the present disclosure refers to cell surface antigens that are associated with disease treatment and diagnosis, expressed on the cell surface, and may be targeted by CAR. The "disease-associated cell surface antigen" of the present disclosure includes CD antigens, immune checkpoint antigen, tumor-associated antigens, tumor-specific antigens, virus-induced tumor antigens, and may be selected from but not limited to the following antigens: CD19, PD-1, PD-L1, HER2, STAT3, CTLA-4, IDO, NY-ESO-1, CD40, CSF1R, BCMA, MUC1, ADORA2A, CD20, GD2, TLR7, WT1, IFNAR1, CD47, Neoantigen, EGFR, LAG- 3. OX40, PSMA, Mesothelin, TERT, TLR, TLR9, 4-1BB, IL2R, TLR4, CD33, GITR, HPV E6, Survivin, CD123, TIGITTIM-3, CD73, HPV E7, TLR3, CD38, EBV, STING, CD22, GPC3, HDAC1, CXCR4, GMCSFR, CD30, CEACAM5, HDAC6, HPV, CD3, MAGE-A3, TNF, PSA, CD25, CEA, EPCAM, CMV, IL12, PRAME, IL12R, 5T4, Beta catenin, CCR2, PMEL, CXCL12, IGF1, CD46, CXCR1, GMCSF, IL15R, ROR1, TGFBR2, CCR4, FLT-3, FOLR1, GCSFR, ICOS, JAK2, KRAS, VISTA, CD133, CD27, CD39, CEACAM6, NKG2D, STAT5, TGFB1, TLR2, USP7,ANG1, ANG2, B7-H3, CLEC12A, IL13RA2, RIG-I, TRP2, VEGF, AFP, Alpha-Gal, COX-2, EPHA2, gp96, MUC16, p53, TGF-β, CD138, CDw136, CS1, CXCR2, EGFRvIII, Gelactin-3, Globo H, GR, IFNAR2, IFNGR1, IL6, JAK1, MLANA, RAS, SLAMF7, TDO, TGFB2, TLR8, ALK, Arginase, CCR1, CD56, CD70, FAP, GD3, IDH1, IL6R, IRAK4, MAGE-A4, MERTK, MIF, PSCA, PTGER4, SIRPA, TGFB, TGFBR1, ACPP, ADORA2B, AR, Brachyury, CA19-9, CD32, CEACAM1, Gastrin, HDAC, HPV L2, IFNAR, IFNGR, IGF1R, IGF2, IL15, IL17R, IL1B, IL7R, JAK, MAGE-A, MAGE-A1, MAGE-A6, P38, RORC, TLR5, VEGFR2, ADORA3, ATRT, B7-H4, c-KIT, CCR7, CD11b, CD135, CD171, CD174, CDH3, CX3CR1, Gelactin-1, GM3, HLA-A2, HSP70, IL10, IL17, IL2RB, JAK3, MDA5, NKG2A, PBF, PVRIG, SPAM1, URLC10, VEGFR1, ABCB5, ADABP, ADAM17, ADP, AEG1, Alpha-lactalbumin, AMHR2, ASPH, AXL, BCL2, BTE6-LX-8b, BTE6-X-15-7, Carbohydrate antigens, CCL20, CCL3, CCNB1, CD147, CD155, CD16, CD162, CD16a, CD200, CD21, CD28 , CD44, CD52, CD54, CD7, CD80, CD88, Claudin 18, cMET, COX2, CSF1, CTCFL, CXCR5, CXCR7, E1A, EIF2AK3, ERG, FGF2, FN1, GC, GM2, gpA33, HBV, Hemagglutinin, HER3, HILPDA, HLA-DR, HMW-MAA, HP59, HPV 16, HPV E6/7, HPV L1, HSP105, HSP65, HVEM, Hyaluronan, IL13RA1, IL2, IL21R, IL8, KIF20A, KIR2DL1, KIR2DL3, LXR, MAGE-A10 , MAGE-C2, MammaglobinA, MAPK, MICA, MiHA, MMP-11, MVP, Myeloblastin, N-Myc, NKp46, NLRP3, NR2F6, Oncofetal Antigen, P2RX7, RhoC, SIM-2, SSTR2, SSX2, STAT1, STn , TAG72, TAMA, TFDP3, TGFBR, TSA, TYK2, Tyrosinase, VEGFA, 5'Nucleotidase (Ecto 5'Nucleotidase or CD73 or NT5E or EC 3.1.3.5), ADAM9, AIM2, B7-H6, BAFF-R, BAI1, BARD1, BOB-1, CA9, Cancer testis antigen, CB2, CBLB, CC R9, CD13, CD130, CD150, CD160, CD200R1, CD267, CD29, CD3E, CD4, CD51, CD8, CGEN-XXXX, Claudin 6, CLEC2D, COX, COX-1, CPEB4, CPEG4, CRBN, CRLF2, CSPG4, CTA, CXCL1, CXCR3, Cytosine deaminase, DCK, DKK1, DLL3, DR3, DR5, EBNA3C, EGF, EGFR5, ELVAL4, EPHA3, EPS8, EVI1, FAIM-3, FasR, FCU1, FLT3, FOLR, FOXM1, FSHR, Galectin-3, GalNAc, GARP, Gelactin-9, Gelatcin-1/3/9, GLD18, GNRHR, GP160, GP73, H3.3K27M, HAGE, HDAC2, HDAC8, HPV16 E6, HPV16 E7, HSP, Hypoxia, ICAM, ICAM7, IDO1, IFNG, IFNGR2, IGF2R, IGFBP2, IGK, IL10RA, IL12RB1, IL13, IL13R, IL13Ralpha2, IL15RA, IL17A, IL17B, IL1A, IL1R1, IL1R3, IL21, IL22R, IL27R, IL2RA, IL35, IL9R, Integrin beta-7, IRAK1, ITGB5, Kappa myeloma antigen, KIR2DL2, Kynurenine, L1CAM, Lambda Myeloma Antigen, LAMP, LLO, LXRA, LXRB, Mas receptor, MG7, MHCI, MHCII, MIC, MOSPD2, MRP-3, MRP1, MRP3765, muGNTP01, MYB, MYBL2, NFAT, NGcGM3, Nrf2, p38 MAP Kinase (EC 2.7.11.24), P55, PAM4, PAP, PASD1, PCDH18, PD-L2, PI3K-delta, POTE, PPT, Protein tolemerase, PTGER2, RANKL, RBL001, RNF43, ROR2, S100A9, SEREX, SLAMF1, STAT, TACSTD2, TASTD2, TDO2, TEM, thymidine kinase, Thymidylate synthase, TIE2, TIMP3, TM4SF5, TOP1, TRBC1, TRBC2, TRIF, Tryptophan, TSHR, TWEAK, UTA2-1, VDBP, VRP, VSIG-4, XAGE1, XAGE1A, ZP1, ZP3.

"Immune function regulatory/modulatory factor" refers to any cytokine or molecule that can regulate the process or outcome of an immune response. The "immune function regulatory factor" includes immune function stimulating factors and immune function inhibiting factors. The "immune function regulatory factor" includes but is not limited to: IL-2, IL-12, IL-7, IL-15, IL-18, IL-21, IL-24, 4-1BBL, PD-1 extracellular region, PD-L1 extracellular region, CCL19, MIP-1α, GM-CSF, IFN-α, IFN-β, IFN-γ, TNF-α, M-CSF, TGF-β and TRAIL and the like.

"Expression vector" of the present disclosure refers to a vector containing a recombinant polynucleotide which contains an expression control sequence operably linked to a coding nucleotide sequence. The expression vector includes sufficient cis-acting element for expression, and other expression elements may be provided by host cells or in an in vitro expression system. The expression vector includes all those known in the art, including cosmids, plasmids (for example, naked or contained in a liposome) and viruses (for example, lentivirus, retrovirus, adenovirus and adeno-associated virus) that are incorporated with the recombinant polynucleotide.

The term "lentiviral vector" refers to a vector derived from at least a part of the lentiviral genome, and specifically includes a self-inactivating lentiviral vector as provided in Milone et al., Mol. Ther. 17(8): 1453-1464 (2009). Other examples of the lentiviral vector that may be used in clinical practice include, but are not limited to Lentivector^{®} gene delivery technology from Oxford BioMedica,and LLENTIMAX vector system from Lentigen. Non-clinical types of lentiviral vectors may also available and may be known to those skilled in the art.

"Constitutive" expression includes the state in which a gene product is produced in a living cell under most or all physiological conditions of the cell.

### Embodiment

It should be noted that, in the case without conflicting, the embodiments in the present application are merely illustrative, and are not intended to limit the present disclosure in any manner.

### Embodiment

### Embodiment 1: Construction of recombinant lentiviral vector of chimeric antigen receptor

Recombinant vectors containing the following sequences are constructed (expression framework from 5-end to 3-end):
1) Vector targeting PSMA:
   EF1a promoter-CD8α signal peptide-aPSMA scfv-CD8 hinge-CD8 transmembrane region-4-1BB (aPSMA BB)
   EF1a promoter-CD8α signal peptide-aPSMA scfv-CD8 hinge-CD8 transmembrane region-4-1 BB-CD3Z (aPSMA BBZ)
2) Vector targeting PSMA and inducibly secreting IL-12:
   EF1a promoter-CD8α signal peptide-aPSMA scfv-CD8 hinge-CD8 transmembrane region-4-1BB-6XNFAT promoter-IgG kappa signal peptide-IL-12(P40-35) (aPSMA BB+iIL-12)
   EF1a promoter-CD8α signal peptide-aPSMA scfv-CD8 hinge-CD8 transmembrane region-4-1BB-CD3Z-6XNFAT promoter-IgG kappa signal peptide-IL-12 (P40-35) (aPSMA BBZ+iIL-12)
3) Vector targeting PSMA and inducibly secreting IL-12:
   EF1a promoter-CD8α signal peptide-aPSMA scfv-CD8 hinge-CD8 transmembrane region-4-1BB-6XNFAT promoter-IgG kappa signal peptide-IL-15 (aPSMA BB+iIL-15)
   EF1a promoter-CD8α signal peptide-aPSMA scfv-CD8 hinge-CD8 transmembrane region-4-1BB-CD3Z-6XNFAT promoter-IgG kappa signal peptide-IL-15 (aPSMA BBZ+iIL-15)
4) Vector targeting PSMA and inducibly secreting IL-18:
   EF1a promoter-CD8α signal peptide-aPSMA BB-CD8 hinge-CD8 transmembrane region-4-1BB-6XNFAT promoter-IgG kappa signal peptide-IL-18 (aPSMA BB+iIL-18)
5) Vector targeting PSMA and inducibly secreting IL-2:
   EF1a promoter-CD8α signal peptide-aPSMA scfv-CD8 hinge-CD8 transmembrane region-4-1BB-6XNFAT promoter-IgG kappa signal peptide-IL-2 (aPSMA BB+iIL-2)
6) Vector targeting PSMA and constitutively secreting IL-12:
   EF1a promoter-CD8α signal peptide-aPSMA scfv-CD8 hinge-CD8 transmembrane region-4-1BB-PGK promoter-IgG kappa signal peptide-IL-12 (P40-35) (aPSMA BB-PGK+IL-12)
7) Vector targeting GPC3:
   EF1a promoter-CD8α signal peptide-aGPC3 scfv-CD8 hinge-CD8 transmembrane region-4-1BB (aGPC-3BB)
   EF1a promoter-CD8α signal peptide-aGPC3 scfv-CD8 hinge-CD8 transmembrane region-4-1BB-CD3Z (aGPC-3BBZ)
8) Vector targeting GPC3 and inducibly secreting aPD-1:
   EF1a promoter-CD8α signal peptide-aGPC3scfv-CD8 hinge-CD8 transmembrane region-4-1BB-6XNFAT promoter-IgG kappa signal peptide-aPD-1 scfv (aGPC-3BB+iaPD-1)
   EF1a promoter-CD8α signal peptide-aGPC3 scfv-CD8 hinge-CD8 transmembrane region-4-1BB-CD3Z-6XNFAT promoter-IgG kappa signal peptide-aPD-1 scfv (aGPC-3BBZ+iaPD-1)
9) Vector targeting GPC3 and inducibly secreting aPD-L1:
   EF1a promoter-CD8α signal peptide-aGPC3scfv-CD8 hinge-CD8 transmembrane region-4-1BB-6XNFAT promoter-IgG kappa signal peptide-aPD-L1 scfv (aGPC-3BB+iaPD-L1)
   EF1a promoter-CD8α signal peptide-aGPC3scfv-CD8 hinge-CD8 transmembrane region-4-1BB-CD3Z-6XNFAT promoter-IgG kappa signal peptide-aPD-L1 scfv (aGPC-3BBZ+iaPD-L1)
10) Vector targeting GPC3 and constitutively secreting aPD-1: EF1a promoter-CD8α signal peptide-aGPC3scfv-CD8hinge-CD8 transmembrane region-4-1BB-PGK promoter-IgG kappa signal peptide-aPD-1 scfv (aGPC-3BB-PGK+aPD-1)
11) Vector targeting GPC3 and constitutively secreting aPD-1:
   EF1a promoter-CD8α signal peptide-aGPC3scfv-CD8 hinge-CD8 transmembrane region-4-1BB-P2A-IgG kappa signal peptide-aPD-1 scfv (aGPC-3BB-P2A+aPD-1)
12) Vector targeting GPC3 and inducibly secreting IL-2: EF1a promoter-CD8α signal peptide-aGPC3scfv-CD8 hinge-CD8 transmembrane region-4-1BB-6XNFAT promoter-IgG kappa signal peptide-iIL-2 (aGPC-3BB+iIL-2)
13) Vector targeting GPC3 and constitutively secreting IL-12 (p40-35), and aPD-1:
   EF1a promoter-CD8α signal peptide-aGPC3scfv-CD8 hinge-CD8 transmembrane region-4-1BB-PGK promoter-IgG kappa signal peptide-IL-12(p40-35)-P2A-IgG kappa signal peptide-aPD-1 scfv (aGPC-3BB-PGK+IL-12-P2A+aPD-1)
14) Vector targeting GPC3 and inducibly secreting IL-12 (p40-35) and constitutively secreting aPD-1 :
   EF1a promoter-CD8α signal peptide-aGPC3scfv-CD8 hinge-CD8 transmembrane region-4-1 BB-6XNFAT promoter-IgG kappa signal peptide-IL-12(p40-35)-P2A-IgG kappa signal peptide-aPD-1 scfv (aGPC-3BB+iIL-12-P2A+aPD-1)

The above sequences are synthesized and cloned into the lentiviral expression plasmid pLenti, and verified by sequencing. The nucleic acid and amino acid sequences corresponding to each part are shown in the table below.

| | Nucleic acid sequence SEQ ID NO: | Amino acid sequence SEQ ID NO: |
|---|---|---|
| EF1 a promoter | 1 | / |
| PGK promoter | 2 | / |
| 6XNFAT promoter | 3 | / |
| IL-2 promoter | 4 | / |
| IgG kappa signal peptide | 5 | 6 |
| SP-Kappa 128477 | 7 | 8 |
| CD8 hinge region and transmembrane region | 9 | 10 |
| CD3Z signal domain | 11 | 12 |
| 4-1BB signal domain | 13 | 14 |
| IL-2 | 15 | 16 |
| IL-12(P40-35) | 17 | 18 |
| IL-15 | 19 | 20 |
| IL-18 | 21 | 22 |
| aGPC3scfv | 23 | 24 |
| aPD-1 scfv | 25 | 26 |
| aPD-L1 scfv | 27 | 28 |
| P2A | 29 | 30 |
| aPSMA scfv | 31 | / |
| aPSMA BB | 32 | / |
| aPSMA BBZ | 33 | / |
| aPSMA BB+iIL-12 | 34 | / |
| aPSMA BBZ+iIL-12 | 35 | / |
| aPSMA BB+iIL-15 | 36 | / |
| aPSMA BBZ+iIL-15 | 37 | / |
| aPSMA BB+iIL-18 | 38 | / |
| aPSMA BB-iIL-2 | 39 | / |
| aPSMA BB-IL-12 | 40 | / |
| aGPC-3BB | 41 | / |
| aGPC-3BBZ | 42 | / |
| aGPC-3BB+iaPD-1 | 43 | / |
| aGPC-3BBZ+iaPD-1 | 44 | / |
| aGPC-3 BB+iaPD-L1 | 45 | / |
| aGPC-3BBZ+iaPD-L1 | 46 | / |
| aGPC-3BB-PGK+aPD-1 | 47 | / |
| aGPC-3BB-P2A+aPD-1 | 48 | / |
| aGPC-3BB+iIL-2 | 49 | / |
| GPC-3BB-PGK+IL-12-P2A+aPD-1 | 50 | / |
| aGPC-3BB+iIL-12-P2A+aPD-1 | 51 | / |
| aCD19 scfv | 52 | 53 |
| aCD19 BBZ | 54 | 55 |
| aMUC16 scfv | 56 | 57 |
| aFAP scfv | 58 | 59 |
| OX40 | 60 | 61 |
| aPD-1 HCDR1 | / | 62 |
| aPD-1 HCDR2 | / | 63 |
| aPD-1 HCDR3 | / | 64 |
| aPD-1 LCDR1 | / | 65 |
| aPD-1 LCDR2 | / | 66 |
| aPD-1 LCDR3 | / | 67 |
| aPD-L1 HCDR1 | / | 68 |
| aPD-L1 HCDR2 | / | 69 |
| aPD-L1 HCDR3 | / | 70 |
| aPD-L1 LCDR1 | / | 71 |
| aPD-L1 LCDR2 | / | 72 |
| aPD-L1 LCDR3 | / | 73 |
| aPSMA HCDR1 | / | 74 |
| aPSMA HCDR2 | / | 75 |
| aPSMA HCDR3 | / | 76 |
| aPSMA LCDR1 | / | 77 |
| aPSMA LCDR2 | / | 78 |
| aPSMA LCDR3 | / | 79 |
| aGPC3 HCDR1 | / | 80 |
| aGPC3 HCDR2 | / | 81 |
| aGPC3 HCDR3 | / | 82 |
| aGPC3 LCDR1 | / | 83 |
| aGPC3 LCDR2 | / | 84 |
| aGPC3 LCDR3 | / | 85 |
| aCD19 HCDR1 | / | 86 |
| aCD19 HCDR2 | / | 87 |
| aCD19 HCDR3 | / | 88 |
| aCD19 HCDR4 | / | 89 |
| aCD19 LCDR1 | / | 90 |
| aCD19 LCDR2 | / | 91 |
| aCD19 LCDR3 | / | 92 |
| aMUC16 HCDR1 | / | 93 |
| aMUC16 HCDR2 | / | 94 |
| aMUC16 HCDR3 | / | 95 |
| aMUC16 LCDR1 | / | 96 |
| aMUC16 LCDR2 | / | 97 |
| aMUC16 LCDR3 | / | 98 |
| aFAP HCDR1 | / | 99 |
| aFAP HCDR2 | / | 100 |
| aFAP HCDR3 | / | 101 |
| aFAP LCDR1 | / | 102 |
| aFAP LCDR2 | / | 103 |
| aFAP LCDR3 | / | 104 |
| aGPC3 VH | / | 105 |
| aGPC3 VL | / | 106 |
| aPSMA VH | / | 107 |
| aPSMA VL | / | 108 |
| aPD-1 VH | / | 109 |
| aPD-1 VL | / | 110 |
| aPD-L1 VH | / | 111 |
| aPD-L1 VL | / | 112 |
| aCD19 VH | / | 113 |
| aCD19 VL | / | 114 |
| aMUC16 VH | / | 115 |
| aMUC16 VL | / | 116 |
| aFAP VH | / | 117 |
| aFAP VL | / | 118 |
| aEGFR VH | / | 119 |
| aEGFR VL | / | 120 |
| aHER2 VH | / | 121 |
| aHER2 VL | / | 122 |
| aCD3 VH | / | 123 |
| aCD3 VL | / | 124 |
| aFAP scfv-aCD3 | / | 125 |
| aHER2-aCD3 | / | 126 |
| aCD3 HCDR1 | / | 127 |
| aCD3 HCDR2 | / | 128 |
| aCD3 HCDR3 | / | 129 |
| aCD3 LCDR1 | / | 130 |
| aCD3 LCDR2 | / | 131 |
| aCD3 LCDR3 | / | 132 |
| aEGFR HCDR1 | / | 133 |
| aEGFR HCDR2 | / | 134 |
| aEGFR HCDR3 | / | 135 |
| aEGFR LCDR1 | / | 136 |
| aEGFR LCDR2 | / | 137 |
| aEGFR LCDR3 | / | 138 |
| aHER2 HCDR1 | / | 139 |
| aHER2 HCDR2 | / | 140 |
| aHER2 HCDR3 | / | 141 |
| aHER2 LCDR1 | / | 142 |
| aHER2 LCDR2 | / | 143 |
| aHER2 LCDR3 | / | 144 |
| CD28 intracellular signal transduction domain | 145 | 146 |
| LAG3 intracellular signal transduction domain | 147 | 148 |
| NF-_{κ}b response element.1 | 149 | / |
| NF-_{κ}b response element.2 | 150 | / |
| NF-_{κ}b response element.3 | 151 | / |
| NF-_{κ}b response element.4 | 152 | / |
| NF-_{κ}b basal promoter.1 | 153 | / |
| NF-_{κ}b basal promoter.2 | 154 | / |
| NF-_{κ}b basal promoter.3 | 155 | / |
| NF-_{κ}b response promoter | 156 | / |

### Embodiment 2: Lentiviral package and preparation of CAR-T cells

The Lentiviral packaging cell line 293T cells were seeded in a 10 cm cell culture dish pre-coated with poly-L-lysine, and transfected with the confluence rate of 70%-80% at 37°C, 5% CO₂. The lentiviral expression plasmid prepared in Embodiment 1 and helper plasmids psPax2 and pMD2.G were co-transfected into 293T cells with transfection reagent PEI in a mass ratio of 4:3:1, and further cultured at 37°C, 5% CO₂. After 8-12 hours, supernatant was removed and fresh medium added. Supernatant was collected after further 48 hours' culture and centrifuged at 4°C, 2000 rpm for 10 minutes to remove cell debris, the supernatant was further filtered with a 0.45 µm sterile filter and placed in a low-temperature refrigerator at -80°C for storage or directly used for T cell infection.

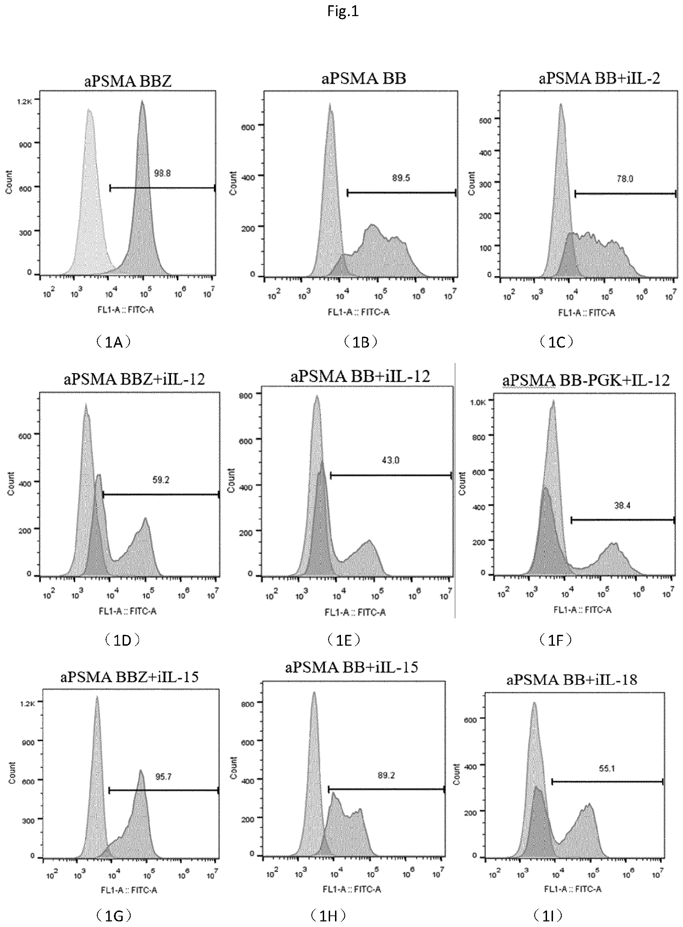

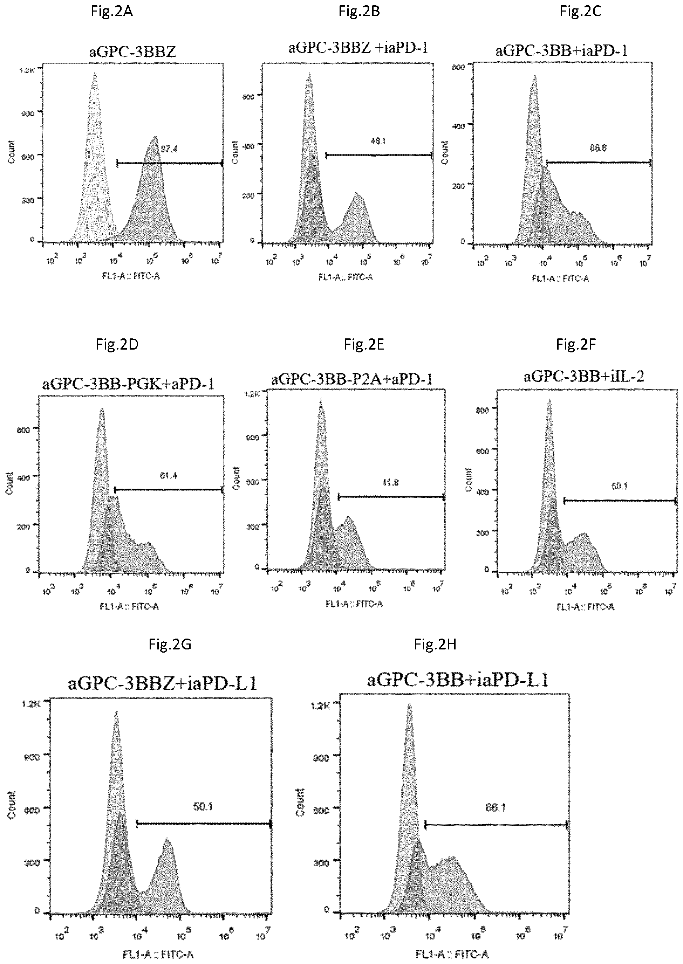

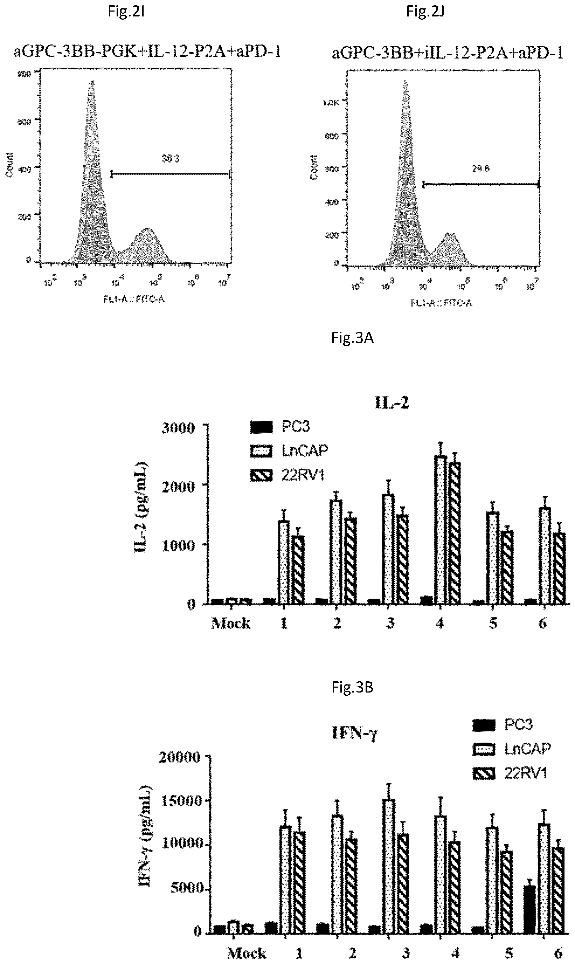

Peripheral blood mononuclear cells (PBMCs) were isolated from healthy donors with lymphocyte separation solution, transferred to a cell culture flask and cultured at 37°C,5% CO₂ for 1 hour to remove adherent cells. Suspended PBMCs were adjusted to 2×10⁶/mL and activated for 2 days with anti-CD3 antibody OKT-3 and anti-CD28 antibody 15E8. After activation, an appropriate number of cells were mixed with fresh or thawed virus solution as mention above as well as with protamine at a final concentration of 8 µg/mL. The mixed cells were subjected to centrifuge at 32°C, 1000×G for 1 hour in a horizontal rotor centrifuge (the centrifuge was set with increase speed of 9 and a decrease speed of 0). After discarding the supernatant, cells were further cultured for 8 hours and transferred to fresh medium containing 300 IU/mL IL-2. 72hours later, CAR expression on cells was detected by flow cytometry. Results were shown in Fig. 1 and Fig. 2A-2J.

Embodiment 3: In vitro killing activity of PSMA-targeting CART cells with secretory pro-inflammatory factor

### 3.1 Secretion of IL-2, IFN-γ, IL-12 and IL-18 by PSMA-targeting CAR-T cells

Effector cells (such as PSMA-targeted CART with secretory functions in the embodiment) and target cells (such as PC3, LnCAP or 22RV1 cells) are mixed in a 12-well plate at a ratio of 1:1( both the cell number of the effector cells and target cells in each well was 1*10⁶. After incubating at 37°C, 5% CO₂ for 24 hours, the culture supernatant was collected, and the levels of IL-2, IL-12, IFN-γ and IL-18 in the supernatant were detected with Human IL-2 ELISA MAX^{™} Standard (BioLegend), Human IL-12 ELISA MAX^{™} Standard (BioLegend), HUMAN IFN GAMMA ELISA RSG (eBioscience) and human interleukin 18 (IL-18) ELISA kits, respectively.

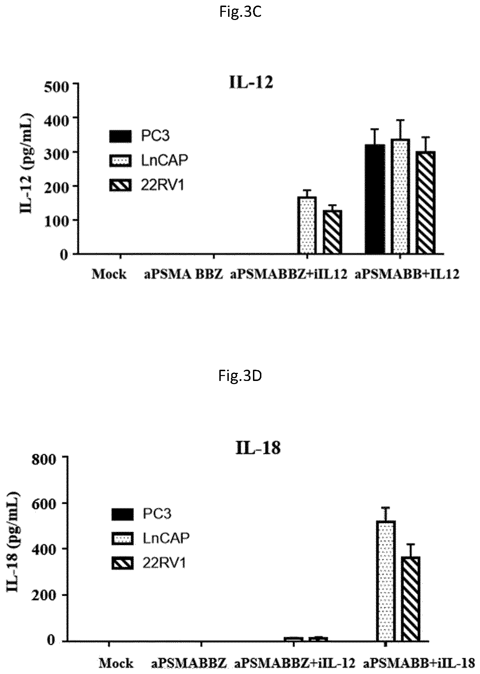

Results were shown in Fig. 3A-3D. aPSMA-expressing CART cells could effectively secret IL-2 and IFN-γ when incubated with PSMA-positive target cells LnCAP or 22RV1, while no IL-2 or IFN-γ secretion was detected when incubated with PSMA-negative target cells PC3. The cytokines secretion of the CART cells (cells expressing aPSMA BB+iIL-2, aPSMA BB+iIL-18 or aPSMA BB+IL-12) lacking an artificial first signal CD3Z were similar to that of CART cells (cells expressing aPSMA BBZ, aPSMA BBZ+iIL-12, aPSMA BBZ+iIL-15) containing the artificial first signal CD3Z (Figs. 3A-3D).

### 3.2 In vitro killing activity of PSMA-targeting CAR-T cells

The effector cells (such as PSMA-targeting CART cells with secretory cytokines in the embodiment) and target cells (such as PC3 or LnCAP cells) were incubated at 37°C, 5% CO₂ at different ratios (20:1, 10:1, 5:1, 2:1, 1:1, and 0.5:1). After 24 hours, cell supernatant was collected, and the levels of LDH was detected by CytoTox 96 Non-Radioactive Cytotoxicity Assay (Promega).

Results were shown in Fig. 5, CART cells expressing aPSMA BBZ or aPSMA BBZ+iIL-12 could effectively exert killing activity on PSMA-positive LnCAP cells while not on PMSA-negative PC3 cells.

### Embodiment 4: In vitro killing activity of GPC3-targeted CART cells with secretory immune-checkpoint inhibitors

### 4.1 Detection of IL-2 and aPD-1 scfv secreted by CAR-T cells

GPC3-targeting effector cells (such as CART cells) and target cells (such as A431 or HepG2 cells) were incubated at a ratio of 1:1 at 37°C, 5% CO₂. After 24 hours, cell supernatant was collected, and the level of IL-2 in the supernatant was detected with Human IL-2 ELISA MAX^{™} Standard (BioLegend).

The aPD-1 scfv expression was detected by sandwich ELISA: hPD-1 was coated on a 96-well plate, and incubated at 4°C overnight. After blocking with PBST (0.5% Tween-20 in phosphate buffered saline (PBS)) containing 2% fetal bovine serum (FBS) at room temperature for 1 hour, the cell supernatant above was added, and then incubated at room temperature for 2 h. After being washed for 4-5 times with PBST containing 2% FBS, goat anti-Human IgG(Fab')2(HRP) secondary antibody was added and incubated at room temperature for 1 h, followed by washing with PBST containing 2% nonfat dry milk for 4-5 times. Reading at OD450nm were recorded after TMB reagent (BioLegend, ) was added for color development.

Results were shown in Fig. 4. The level of IL-2 secretion by CART cells lacking an artificial first signal CD3Z (aGPC-3BB+iaPD1, aGPC-3BB+iaPD-L1, aGPC-3BB-PGK+aPD-1, aGPC-3BB-P2A+iaPD-1, and aGPC-3BB+iIL-2) was similar to that of IL-2 secreted by CART cells containing the artificial first signal CD3Z when incubating with GPC-3 positive target cells HepG2. CART cells lacking the artificial first signal CD3Z while constitutively expressing aPD-1 scfv showed significantly higher level of aPD-1 scfv expression (aGPC-3BB-PGK+aPD-1), compared to CART constructs with inducible aPD-1 scfv secretion (aGPC-3BB+iaPD1, aGPC-3BBZ+iaPD-1, and aGPC-3Z+iaPD-1).

### 4.2 In vitro killing activity of GPC-3-targeting CAR-T cells

Effector cells (such as GPC-3-targeting CART cells in the embodiment) and target cells (such as HepG2 or A431 cells) were co-incubated at 37°C, 5% CO₂ at different ratios (5:1, 3:1, 1:1, 1:3, and 1:5). After 24 hours, cell supernatant was collected, and the LDH level was detected with CytoTox 96 Non-Radioactive Cytotoxicity Assay (Promega).

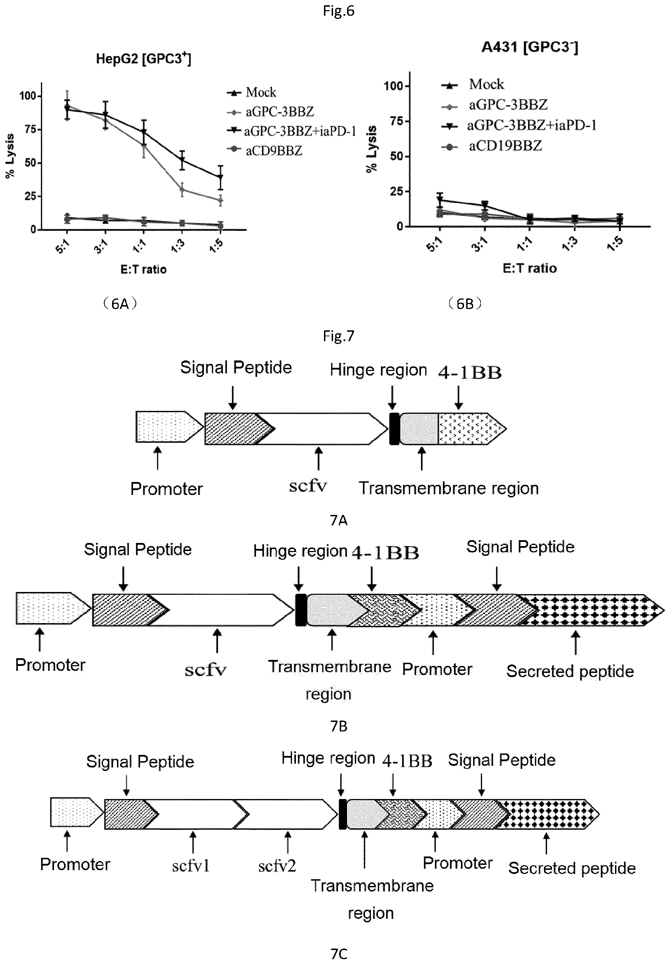

Results were shown in Fig. 6, CART cells expressing aGPC-3 BBZ or aGPC-3 BBZ+iaPD-1 could effectively exert killing activity on GPC-3 positive HepG2 cells while not on GPC-3 negative A431 cells.

### Embodiment 5: In vitro killing activity of PSMA-targeting CART cells

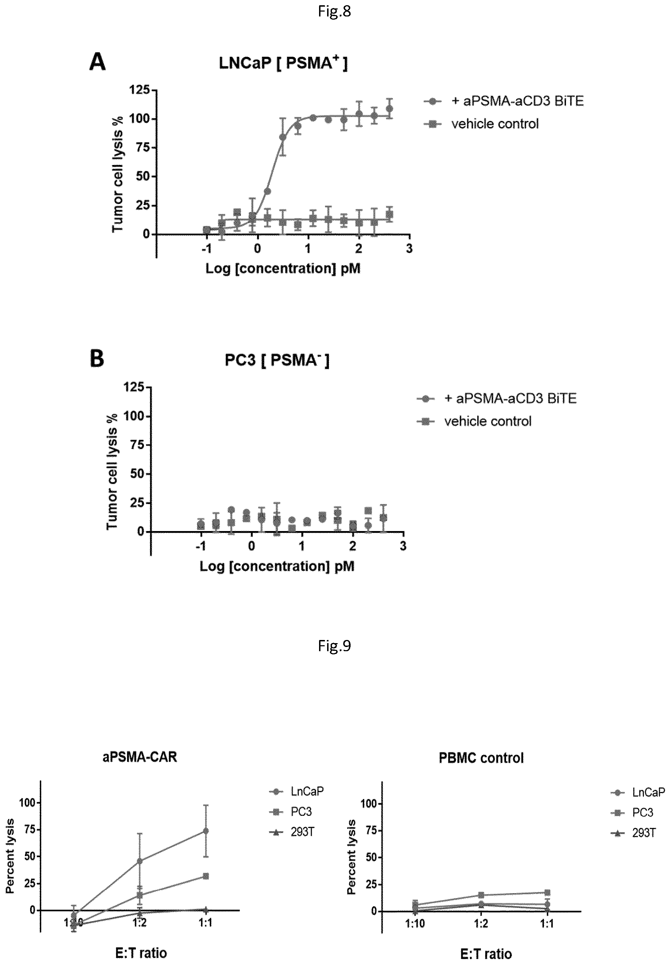

aPSMA BBZ CAR-T cells were incubated with two target cells (LNCaP and PC3) at a ratio of 1:1 (10⁴ cells each per well) in a 96-well plate (flat bottom), respectively. Bispecific antibody aPSMA-aCD3 BiTE pre-diluted in a two-fold gradient and PBS (as solvent control) were added, and incubated in a cell incubator (37°C, 5% CO₂) for 16 hours. CytoTox 96^{®} Non-Radioactive Cytotoxicity Assay (Promega^{™}) kit was used to detect the killing activity of CAR-T cells on target cells. As shown in Fig. 8, PSMA-specific CAR-T cells containing only costimulatory signal transduction domain showed specific killing activity on PSMA-positive tumor target cells LnCaP while not on PSMA-negative tumor target cells PC3 in the presence of first activation signal mediated by aPSMA-aCD3 BiTE molecules.

CAR-T effector cells derived from patient's PBMCs and target cells (such as tumor cells, PC3 cells or LnCAP cells) were mixed at different ratios (1:10, 1:2, 1:1), herein the number of the target cells was 10⁴ cells/well. After incubation at 37°C, 5% CO₂ for 24 hours, cell culture supernatant was collected, and LDH level was detected by CytoTox 96 Non-Radioactive Cytotoxicity Assay (Promega) kit. Results were shown in Fig. 9, patient-derived CART cells expressing aPSMA BBZ could specifically and effectively exert killing activity on PSMA-positive tumor cells in vitro. In contrast, activated T cells without CAR transfection exhibited no killing activity.

### Embodiment 6: NF-κb responsive promoter induced by costimulatory signal activation

Firefly luciferase reporter gene elements driven by NF-κb responsive promoter (from N-terminal to C-terminal: NF-κb responsive promoter-firefly luciferase reporter gene-PGK promoter-green fluorescent protein) were introduced into PBMC cells by lentiviral transduction. The prepared PBMC cells were plated in a 96-well plate at a density of 4×10⁴ cells/well, and stimulants (TNFα, Phorbol myristate acetate, 4-1 bbL) pre-diluted in a two-fold gradient and PBS (as solvent control) were further added. After incubation (37°C, 5% CO₂) for 6 hours, fluorescence in well was detected by Firefly Luc One-Step Glow Assay Kit (Pierce^{™}), and the induction of luciferase expression were calculated with the solvent control as the base line.

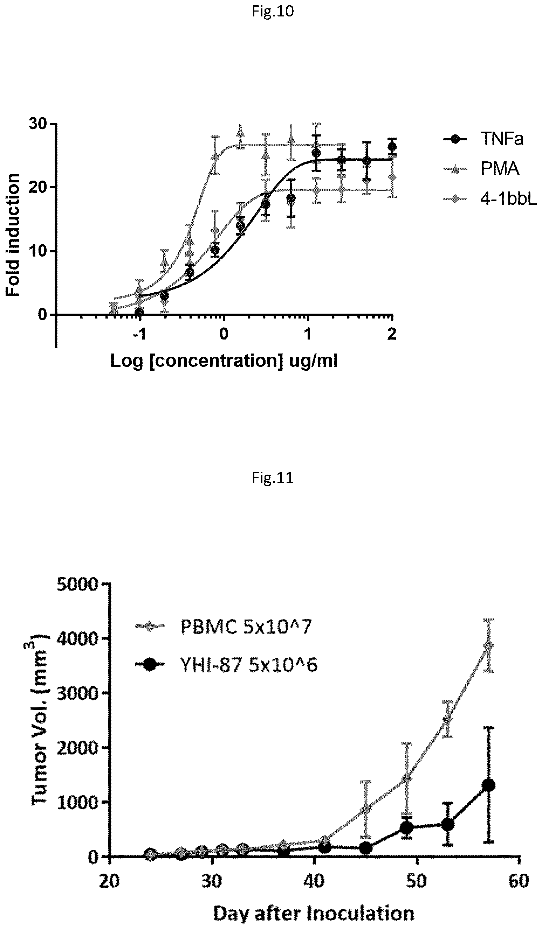

As shown in Fig. 10, TNFα, Phorbol myristate acetate (PMA) and 4-1bbL could all activate the NF-κb responsive promoter in PBMC cells and drive the expression of downstream genes.

### Embodiment 7: In vivo study with mouse model

Efficacy studies of PSMA-targeted CAR were performed in 6-week-old male NCG mice. 2×10⁶ LnCap cells were subcutaneously (SC) injected into the right flank of mice, and the size of tumor was measured with calipers three times a week. When the volume of the tumor reached 100-200 mm³, CART cells were intravenously injected into the tail (Day 0) (The experiments were divided into two groups: activated T cell injection group, and 5×10⁶ CART cell injection group). Tumor size and mouse body weight were measured weekly. The tumor volume was calculated by the following formula: tumor volume=width*length*height/2. Five weeks after CART injection, the mice are sacrificed, serum or plasma was collected for cytokine or immune factor analysis and mouse tissues were collected. CART cell population in TILs and PBMCs were analyzed by flow cytometry and immunohistochemistry. For CARTs with secretory function, the levels of the secreted proteins in tumor and blood were further analyzed.

As were shown in Fig. 11, CART cells expressing aPSMA BBZ exhibited efficient killing activity to PSMA-positive solid tumors in mice, and the activated T cells without CAR transfection at a dose up to 5×10⁷ showed no killing activity.

## Claims

1. Use of chimeric antigen receptor (CAR)-modified T (CART) cells in preparing a drug for cancer treatment, wherein the CART cells comprise a first CAR, wherein the first CAR comprises an antigen binding domain, a transmembrane domain and an intracellular domain, wherein the intracellular domain contains artificially introduced costimulatory signal transduction domains, wherein the CART cells do not contain an artificially introduced first signal transduction domain.

2. The use of the CART cells according to claim 1, wherein the artificially introduced costimulatory signal transduction domain is selected from one or more of the following intracellular signal transduction domains of costimulatory proteins: 4-1BB (CD137), CD28 , CD27, CD30, OX40, GITR, CD40, BAFFR, ICAM-1, LCK, CD278(ICOS), CD150(SLAMF1), CD152(CTLA4), CD223(LAG3), CD270(HVEM), CD273(PD-L2), CD274(PD-L1), LAT, NKD2CSLP76, TRIM, ZAP70, DAP-10, DAP-12, LFA-1, CD2, CDS, CD7, CD287, LIGHT, NKG2C, NKG2D, SLAMF7, NKp80, NKp30, NKp44, NKp46, CD160, B7-H3 or a ligand that specifically binds to CD83 and the like.

3. The use of the CART cells according to claim 2, wherein the artificially introduced costimulatory signal transduction domain is an intracellular signal transduction domain of 4-1BB or OX40.

4. The use of the CART cells according to any one of claims 1-3, wherein the antigen binding domain binds to disease-associated cell surface antigens.

5. The use of the CART cells according to claim 4, wherein the disease-associated cell surface antigen is selected from immune checkpoint proteins or tumor antigens.

6. The use of the CART cells according to any one of claims 1-5, wherein the CART cell further expresses a second CAR and/or secretory polypeptides, wherein the second CAR comprises an antigen binding domain and a transmembrane domain, wherein the secretory polypeptides are constitutively or inducibly expressed.

7. The use of the CART cells according to claim 6, wherein the antigen binding domain of the second CAR binds to disease-associated cell surface antigens, and preferably, the disease-associated cell surface antigen is a tumor antigen.

8. The use of the CART cells according to claim 7, wherein the secreted polypeptides are selected from but not limited to: one or two of the followings, immune function regulatory factors, antibodies specifically targeting the tumor antigen, or antibodies specifically targeting the immune checkpoint.

9. The use of the CART cells according to any one of claims 5-8, wherein the tumor antigen is independently selected from: epidermal growth factor receptor family (EGFR, HER2, HER3, HER4), PD-1, PD-L1 , CTLA-4, 4-1BB(CD137), OX40, CD28, CD40, CD47, CD70, CD80, CD122, GTIR, A2AR, B7-H3(CD276), B7-H4, IDO, KIR, Tim-3, NY-ESO-1, GPC3, CLL-1, BCMA, mucin family (MUC1, MUC2, MUC3A, MUC3B, MUC4, MUC5AC, MUC5B, MUC6, MUC7, MUC8, MUC12, MUC13, MUC15, MUC16, MUC17, MUC19, MUC20), CD19, CD20, CD22, CD30, CD33, CD52, chemokine receptor family (CCR1, CCR2, CCR3, CCR4, CCR5, CCR6, CCR7, CCR8, CCR9, CCR10, CCL27, CCL28, CX3CR1, CXCR1, CXCR2 , CXCR3, CXCR4, CXCR5, CXCR6), PSMA, CEA, HDAC6, EpCAM, Mesothelin, TERT, TLR, TLR9, TLR4, CD33, GITR, Survivin, CD123, TIGIT, TIM-3, CD73, fibroblast growth factor body (FGFR), vascular endothelial growth factor receptor (FLT1, KDR/Flk-1, VEGFR-3), hepatocyte growth factor receptor (HGFR), nerve growth factor receptor (NGFR), insulin-like growth factor receptor (IGFR), platelet-derived growth factor receptor (PDGFR) or hormone receptor (melanocortin 1 receptor (MC1R, MSHR)) and the like.

10. The use of the CART cells according to claim 9, wherein the tumor antigen is PSMA, GPC3, CD19, MUC16, EGFR, HER2, CD3 or FAP.

11. The use of the CART cells according to any one of claims 5-8, wherein the immune checkpoint protein is selected from: PD-1, PD-L1, CTLA-4, LAG-3, OX40, CD28, CD40, CD47, CD70, CD80, CD122, GTIR, A2AR, B7-H3 (CD276), B7-H4, IDO, KIR, Tim-3 or 4-1BB (CD137) and the like.

12. The use of the CART cells according to claim 11, wherein the immune checkpoint protein is PD-1 or PD-L1.

13. The use of the CART cells according to claim 8, wherein the immune function regulatory factor is selected from: IL-2, IL-12, IL-7, IL-15, IL-18, IL-21, IL-24, 4-1BBL, PD-1 extracellular region, PD-L1 extracellular region, PD-1 mutant, CCL19, MIP-1α, GM-CSF, IFN-α, IFN-β, IFN-γ, TNF-α, M-CSF, TGF-β or TRAIL and the like.

14. The use of the CART cells according to claim 13, wherein the immune function regulatory factor is IL-2, IL-12, IL-15, IL-18, or PD-1 mutant.

15. The use of the CART cells according to claim 6, wherein the secreted polypeptides are on the same peptide chain as the first or second CAR, and the secreted polypeptides are linked to the CAR by self-cleaving peptides, or the coding sequence of the secreted polypeptides are linked to the coding sequence of the CAR by promoter and signal peptide coding sequences.

16. The use of the CART cells according to claim 6, wherein the secreted polypeptides are on different peptide chains from the first or second CAR.

17. The use of the CART cells according to any one of claims 5-16, wherein the antigen binding domain is an antibody or an antibody fragment.

18. The use of the CART cells according to any one of claims 6-17, wherein the secreted polypeptides are proteins, antibodies, or antibody fragments.

19. The use of the CART cells according to any one of claims 1-18, wherein the T cells is selected from one or more subsets of specific T cells, such as a tumor-infiltrating lymphocyte (TIL), a cytotoxic T lymphocyte (CTL), a natural killer T (NKT) cell, a δ T cell or a regulatory T cell.


SEQUENCE LISTING

<110>  SUZHOU NOVA THERAPEUTICS CO.LTD
       WANG, Feng
      GUO, Hui
       LIU, Yan
       ZHANG, Jie

<120>  Application of CART cell in preparing drug for treating cancer

<160>  156

<170>  PatentIn version 3.5

<210>  1
<211>  1178
<212>  DNA
<213>  artificial sequence

<400>  1
gctccggtgc ccgtcagtgg gcagagcgca catcgcccac agtccccgag aagttggggg      60

gaggggtcgg caattgaacc ggtgcctaga gaaggtggcg cggggtaaac tgggaaagtg     120

atgtcgtgta ctggctccgc ctttttcccg agggtggggg agaaccgtat ataagtgcag     180

tagtcgccgt gaacgttctt tttcgcaacg ggtttgccgc cagaacacag gtaagtgccg     240

tgtgtggttc ccgcgggcct ggcctcttta cgggttatgg cccttgcgtg ccttgaatta     300

cttccacctg gctgcagtac gtgattcttg atcccgagct tcgggttgga agtgggtggg     360

agagttcgag gccttgcgct taaggagccc cttcgcctcg tgcttgagtt gaggcctggc     420

ctgggcgctg gggccgccgc gtgcgaatct ggtggcacct tcgcgcctgt ctcgctgctt     480

tcgataagtc tctagccatt taaaattttt gatgacctgc tgcgacgctt tttttctggc     540

aagatagtct tgtaaatgcg ggccaagatc tgcacactgg tatttcggtt tttggggccg     600

cgggcggcga cggggcccgt gcgtcccagc gcacatgttc ggcgaggcgg ggcctgcgag     660

cgcggccacc gagaatcgga cgggggtagt ctcaagctgg ccggcctgct ctggtgcctg     720

gcctcgcgcc gccgtgtatc gccccgccct gggcggcaag gctggcccgg tcggcaccag     780

ttgcgtgagc ggaaagatgg ccgcttcccg gccctgctgc agggagctca aaatggagga     840

cgcggcgctc gggagagcgg gcgggtgagt cacccacaca aaggaaaagg gcctttccgt     900

cctcagccgt cgcttcatgt gactccactg agtaccgggc gccgtccagg cacctcgatt     960

agttctcgag cttttggagt acgtcgtctt taggttgggg ggaggggttt tatgcgatgg    1020

agtttcccca cactgagtgg gtggagactg aagttaggcc agcttggcac ttgatgtaat    1080

tctccttgga atttgccctt tttgagtttg gatcttggtt cattctcaag cctcagacag    1140

tggttcaaag tttttttctt ccatttcagg tgtcgtga                            1178


<210>  2
<211>  507

<212>  DNA
<213>  artificial sequence

<400>  2
ctaccgggta ggggaggcgc ttttcccaag gcagtctgga gcatgcgctt tagcagcccc      60

gctgggcact tggcgctaca caagtggcct ctggcctcgc acacattcca catccaccgg     120

taggcgccaa ccggctccgt tctttggtgg ccccttcgcg ccaccttcta ctcctcccct     180

agtcaggaag ttcccccccg ccccgcagct cgcgtcgtgc aggacgtgac aaatggaagt     240

agcacgtctc actagtctcg tgcagatgga cagcaccgct gagcaatgga agcgggtagg     300

cctttggggc agcggccaat agcagctttg ctccttcgct ttctgggctc agaggctggg     360

aaggggtggg tccgggggcg ggctcagggg cgggctcagg ggcggggcgg gcgcccgaag     420

gtcctccgga ggcccggcat tctgcacgct tcaaaagcgc acgtctgccg cgctgttctc     480

ctcttcctca tctccgggcc tttcgac                                         507


<210>  3
<211>  266
<212>  DNA
<213>  artificial sequence

<400>  3
ggtatcgata agcttgatat cgaattagga ggaaaaactg tttcatacag aaggcgtcaa      60

ttaggaggaa aaactgtttc atacagaagg cgtcaattag gaggaaaaac tgtttcatac     120

agaaggcgtc aattggtccc atcgaattag gaggaaaaac tgtttcatac agaaggcgtc     180

aattaggagg aaaaactgtt tcatacagaa ggcgtcaatt aggaggaaaa actgtttcat     240

acagaaggcg tcaattggtc ccggga                                          266


<210>  4
<211>  113
<212>  DNA
<213>  artificial sequence

<400>  4
cattttgaca cccccataat atttttccag aattaacagt ataaattgca tctcttgttc      60

aagagttccc tatcactctc tttaatcact actcacagta acctcaactc ctg            113


<210>  5
<211>  66
<212>  DNA
<213>  artificial sequence

<400>  5
atggacatgc gagttcctgc ccaactgctt ggccttcttt tgctctggct gccaggagcc      60

aagtgc                                                                 66


<210>  6

<211>  22
<212>  PRT
<213>  artificial sequence

<400>  6

Met Asp Met Arg Val Pro Ala Gln Leu Leu Gly Leu Leu Leu Leu Trp
1               5                   10                  15

Leu Pro Gly Ala Lys Cys
            20

<210>  7
<211>  57
<212>  DNA
<213>  artificial sequence

<400>  7
atggagtttg ggctgagctg ggttttcctc gttgctcttt ttagaggtgt acagtgt          57

<210>  8
<211>  19
<212>  PRT
<213>  artificial sequence

<400>  8

Met Glu Phe Gly Leu Ser Trp Val Phe Leu Val Ala Leu Phe Arg Gly
1               5                   10                  15

Val Gln Cys

<210>  9
<211>  207
<212>  DNA
<213>  artificial sequence

<400>  9
accacgacgc cagcgccgcg accaccaaca ccggcgccca ccatcgcgtc gcagcccctg      60

tccctgcgcc cagaggcgtg ccggccagcg gcggggggcg cagtgcacac gagggggctg     120

gacttcgcct gtgatatcta catctgggcg cccttggccg ggacttgtgg ggtccttctc     180

ctgtcactgg ttatcaccct ttactgc                                         207

<210>  10
<211>  69
<212>  PRT
<213>  artificial sequence

<400>  10

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp Ile Tyr Ile
        35                  40                  45

Trp Ala Pro Leu Ala Gly Thr Cys Gly Val Leu Leu Leu Ser Leu Val
    50                  55                  60

Ile Thr Leu Tyr Cys
65

<210>  11
<211>  336
<212>  DNA
<213>  artificial sequence

<400>  11
agagtgaagt tcagcaggag cgcagacgcc cccgcgtaca agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgc                               336

<210>  12
<211>  112
<212>  PRT
<213>  artificial sequence

<400>  12

Arg Val Lys Phe Ser Arg Ser Ala Asp Ala Pro Ala Tyr Lys Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  13
<211>  126
<212>  DNA
<213>  artificial sequence

<400>  13
aaacggggca gaaagaaact cctgtatata ttcaaacaac catttatgag accagtacaa      60

actactcaag aggaagatgg ctgtagctgc cgatttccag aagaagaaga aggaggatgt     120

gaactg                                                                126

<210>  14
<211>  42
<212>  PRT
<213>  artificial sequence

<400>  14

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  15
<211>  399
<212>  DNA
<213>  artificial sequence

<400>  15
gccccaacca gctcaagtac taagaagaca cagctgcagc tggagcacct gctgctggac      60

ctgcagatga tcctgaacgg catcaacaac tacaagaacc ccaagctgac ccgcatgctg     120

accttcaagt tctacatgcc caagaaggcc accgagctga agcacctgca gtgcctggag     180

gaggagctga agcccctgga ggaggtgctg aacctggccc agagcaagaa cttccacctg     240

cgcccccgcg acctgatcag caacatcaac gtgatcgtgc tggagctgaa gggcagcgag     300

accaccttca tgtgcgagta cgccgacgag accgccacca tcgtggagtt cctgaaccgc     360

tggatcacct tctgccagag catcatcagc accctgacc                            399

<210>  16
<211>  133

<212>  PRT
<213>  artificial sequence

<400>  16

<210>  17
<211>  1530
<212>  DNA
<213>  artificial sequence

<400>  17
atctgggagc tgaagaagga cgtgtacgtg gtggagctgg actggtaccc cgacgccccc      60

ggcgagatgg tggtattaac ctgcgacacc ccagaggaag acggcatcac ctggaccctg     120

gaccagtcaa gcgaggtgct gggtagcggc aagaccctga ccatccaggt gaaggagttc     180

ggcgacgccg gccagtacac ctgccacaag ggcggcgagg tgctgagcca cagcctactg     240

cttctccaca agaaggagga cggcatctgg agcaccgaca tcctgaagga ccagaaggag     300

cccaagaaca agaccttcct gcgctgcgag gccaagaatt actcaggacg attcacctgc     360

tggtggctga ccaccatcag caccgacctg accttcagcg tgaagagcag ccgcggcagc     420

agcgaccccc agggcgtgac ctgcggcgcc gccaccctga gcgccgagcg cgtgcgcggc     480

gacaacaagg agtacgagta cagcgtggag tgccaggagg acagcgcctg ccccgccgcc 540

gaggagagcc tgcccatcga ggtgatggtg gacgccgtgc acaagctgaa gtacgagaac 600

tacaccagca gcttcttcat ccgcgacatc atcaagcccg acccccccaa gaacctgcag 660

ctgaagcccc tgaagaacag ccgccaggtg gaggtgagct gggagtaccc cgacacctgg 720

agcacccccc acagctactt cagcctgacc ttctgcgtgc aggtgcaggg caagagcaag 780

cgcgagaaga aggaccgcgt gttcaccgac aagaccagcg ccaccgtgat ctgccgcaag 840

aatgcgagca tcagcgtgcg cgcccaggac cgctactaca gcagcagctg gagcgagtgg 900

gccagcgtgc cctgcagcgg aggtggcggt ggaggcagcc gcaacctgcc cgtggccacc 960

cccgaccccg gcatgttccc ctgcctgcac cacagccaga acctgctgcg cgccgtgagc 1020

aacatgctgc agaaggcccg ccagaccctg gagttctacc cctgcaccag cgaggagatc 1080

gaccacgagg acatcaccaa ggacaagacc agcaccgtgg aggcctgcct gcccctggag 1140

ctgaccaaga acgagagctg tctgaacagc agagagacta gcttcatcac taacggcagc 1200

tgcctagcca gccgcaagac cagcttcatg atggccctgt gcctgagcag catctacgag 1260

gacctgaaga tgtaccaggt ggagttcaag accatgaacg ccaagctgct gatggacccc 1320

aagcgccaga tcttcctgga ccagaacatg ctggccgtga tcgacgagct gatgcaggcc 1380

ctgaacttca acagcgagac cgtgccccag aagagcagcc tggaggagcc cgacttctac 1440

aagaccaaga tcaagctgtg catcctgctg cacgccttcc gcatccgcgc cgtgaccatc 1500

gaccgcgtga tgagctacct gaacgcatca 1530

<210> 18
<211> 510
<212> PRT
<213> artificial sequence

<400> 18

Ile Trp Glu Leu Lys Lys Asp Val Tyr Val Val Glu Leu Asp Trp Tyr
1               5                   10                  15

Pro Asp Ala Pro Gly Glu Met Val Val Leu Thr Cys Asp Thr Pro Glu
            20                  25                  30

Glu Asp Gly Ile Thr Trp Thr Leu Asp Gln Ser Ser Glu Val Leu Gly
        35                  40                  45

Ser Gly Lys Thr Leu Thr Ile Gln Val Lys Glu Phe Gly Asp Ala Gly
    50                  55                  60

Gln Tyr Thr Cys His Lys Gly Gly Glu Val Leu Ser His Ser Leu Leu
65                  70                  75                  80

Leu Leu His Lys Lys Glu Asp Gly Ile Trp Ser Thr Asp Ile Leu Lys
                85                  90                  95

Asp Gln Lys Glu Pro Lys Asn Lys Thr Phe Leu Arg Cys Glu Ala Lys
            100                 105                 110

Asn Tyr Ser Gly Arg Phe Thr Cys Trp Trp Leu Thr Thr Ile Ser Thr
        115                 120                 125

Asp Leu Thr Phe Ser Val Lys Ser Ser Arg Gly Ser Ser Asp Pro Gln
    130                 135                 140

Gly Val Thr Cys Gly Ala Ala Thr Leu Ser Ala Glu Arg Val Arg Gly
145                 150                 155                 160

Asp Asn Lys Glu Tyr Glu Tyr Ser Val Glu Cys Gln Glu Asp Ser Ala
                165                 170                 175

Cys Pro Ala Ala Glu Glu Ser Leu Pro Ile Glu Val Met Val Asp Ala
            180                 185                 190

Val His Lys Leu Lys Tyr Glu Asn Tyr Thr Ser Ser Phe Phe Ile Arg
        195                 200                 205

Asp Ile Ile Lys Pro Asp Pro Pro Lys Asn Leu Gln Leu Lys Pro Leu
    210                 215                 220

Lys Asn Ser Arg Gln Val Glu Val Ser Trp Glu Tyr Pro Asp Thr Trp
225                 230                 235                 240

Ser Thr Pro His Ser Tyr Phe Ser Leu Thr Phe Cys Val Gln Val Gln
                245                 250                 255

Gly Lys Ser Lys Arg Glu Lys Lys Asp Arg Val Phe Thr Asp Lys Thr
            260                 265                 270

Ser Ala Thr Val Ile Cys Arg Lys Asn Ala Ser Ile Ser Val Arg Ala
        275                 280                 285

Gln Asp Arg Tyr Tyr Ser Ser Ser Trp Ser Glu Trp Ala Ser Val Pro
    290                 295                 300

Cys Ser Gly Gly Gly Gly Gly Gly Ser Arg Asn Leu Pro Val Ala Thr
305                 310                 315                 320

Pro Asp Pro Gly Met Phe Pro Cys Leu His His Ser Gln Asn Leu Leu
                325                 330                 335

Arg Ala Val Ser Asn Met Leu Gln Lys Ala Arg Gln Thr Leu Glu Phe
            340                 345                 350

Tyr Pro Cys Thr Ser Glu Glu Ile Asp His Glu Asp Ile Thr Lys Asp
        355                 360                 365

Lys Thr Ser Thr Val Glu Ala Cys Leu Pro Leu Glu Leu Thr Lys Asn
    370                 375                 380

Glu Ser Cys Leu Asn Ser Arg Glu Thr Ser Phe Ile Thr Asn Gly Ser
385                 390                 395                 400

Cys Leu Ala Ser Arg Lys Thr Ser Phe Met Met Ala Leu Cys Leu Ser
                405                 410                 415

Ser Ile Tyr Glu Asp Leu Lys Met Tyr Gln Val Glu Phe Lys Thr Met
            420                 425                 430

Asn Ala Lys Leu Leu Met Asp Pro Lys Arg Gln Ile Phe Leu Asp Gln
        435                 440                 445

Asn Met Leu Ala Val Ile Asp Glu Leu Met Gln Ala Leu Asn Phe Asn
    450                 455                 460

Ser Glu Thr Val Pro Gln Lys Ser Ser Leu Glu Glu Pro Asp Phe Tyr
465                 470                 475                 480

Lys Thr Lys Ile Lys Leu Cys Ile Leu Leu His Ala Phe Arg Ile Arg
                485                 490                 495

Ala Val Thr Ile Asp Arg Val Met Ser Tyr Leu Asn Ala Ser
            500                 505                 510

<210>  19
<211>  342
<212>  DNA
<213>  artificial sequence

<400>  19
aactgggtga acgtgatcag cgacctgaag aagatcgagg acctgatcca gagcatgcac      60

atcgacgcca ccctgtacac cgagagcgac gtgcacccca gctgcaaggt gaccgccatg     120

aagtgcttcc tgctggagct gcaggtgatc agcctggaga gcggcgacgc cagcatccac     180

gacaccgtgg agaacctgat catcctggcc aacaacagcc tgagcagcaa cggcaacgtg     240

accgagagcg gctgcaagga gtgcgaggag ctggaggaga agaacatcaa ggagttcctg     300

cagagcttcg tgcacatcgt gcagatgttc atcaacacca gc                        342

<210>  20
<211>  114
<212>  PRT
<213>  artificial sequence

<400>  20

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

<210>  21
<211>  471
<212>  DNA
<213>  artificial sequence

<400>  21
tacttcggca agctggagag caagctgagc gtgatccgca acctgaacga ccaggtgctg      60

ttcatcgacc agggcaaccg ccccctgttc gaggacatga ccgacagcga ctgccgcgac      120

aacgcccccc gcaccatctt catcatcagc atgtacaagg acagccagcc ccgcggcatg      180

gccgtgacca tcagcgtgaa gtgcgagaag atcagcaccc tgagctgcga gaacaagatc      240

atcagcttca aggagatgaa cccccccgac aacatcaagg acaccaagag cgacatcatc      300

ttcttccagc gcagcgtgcc cggccacgac aacaagatgc agttcgagag cagcagctac      360

gagggctact tcctggcctg cgagaaggag cgcgacctgt tcaagctgat cctgaagaag      420

gaggacgagc tgggcgaccg cagcatcatg ttcaccgtgc agaacgagga c               471

<210>  22
<211>  157
<212>  PRT
<213>  artificial sequence

<400>  22

Tyr Phe Gly Lys Leu Glu Ser Lys Leu Ser Val Ile Arg Asn Leu Asn
1               5                   10                  15

Asp Gln Val Leu Phe Ile Asp Gln Gly Asn Arg Pro Leu Phe Glu Asp
            20                  25                  30

Met Thr Asp Ser Asp Cys Arg Asp Asn Ala Pro Arg Thr Ile Phe Ile
        35                  40                  45

Ile Ser Met Tyr Lys Asp Ser Gln Pro Arg Gly Met Ala Val Thr Ile
    50                  55                  60

Ser Val Lys Cys Glu Lys Ile Ser Thr Leu Ser Cys Glu Asn Lys Ile
65                  70                  75                  80

Ile Ser Phe Lys Glu Met Asn Pro Pro Asp Asn Ile Lys Asp Thr Lys
                85                  90                  95

Ser Asp Ile Ile Phe Phe Gln Arg Ser Val Pro Gly His Asp Asn Lys
            100                 105                 110

Met Gln Phe Glu Ser Ser Ser Tyr Glu Gly Tyr Phe Leu Ala Cys Glu
        115                 120                 125

Lys Glu Arg Asp Leu Phe Lys Leu Ile Leu Lys Lys Glu Asp Glu Leu
    130                 135                 140

Gly Asp Arg Ser Ile Met Phe Thr Val Gln Asn Glu Asp
145                 150                 155

<210>  23
<211>  726
<212>  DNA
<213>  artificial sequence

<400>  23
gacgtggtga tgacccagag ccccctgagc ctgcccgtga cccccggcga gcccgccagc      60

atcagctgcc gcagcagcca gagcctggtg cacagcaacc gcaacaccta cctgcactgg     120

tacctgcaga agcccggcca gagcccccag ctgctgatct acaaggtgag caaccgcttc     180

agcggcgtgc ccgaccgctt cagcggcagc ggcagcggca ccgacttcac cctgaagatc     240

agccgcgtgg aggccgagga cgtgggcgtg tactactgca gccagaacac ccacgtgccc     300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagc 726

<210> 24
<211> 242
<212> PRT
<213> artificial sequence

<400> 24

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Gly Gly Gly Gly Ser Gly Gly Gly Gly Ser Gly Gly Gly Gly Ser Gln
        115                 120                 125

Val Gln Leu Val Gln Ser Gly Ala Glu Val Lys Lys Pro Gly Ala Ser
    130                 135                 140

Val Lys Val Ser Cys Lys Ala Ser Gly Tyr Thr Phe Thr Asp Tyr Glu
145                 150                 155                 160

Met His Trp Val Arg Gln Ala Pro Gly Gln Gly Leu Glu Trp Met Gly
                165                 170                 175

Ala Leu Asp Pro Lys Thr Gly Asp Thr Ala Tyr Ser Gln Lys Phe Lys
            180                 185                 190

Gly Arg Val Thr Leu Thr Ala Asp Lys Ser Thr Ser Thr Ala Tyr Met
        195                 200                 205

Glu Leu Ser Ser Leu Thr Ser Glu Asp Thr Ala Val Tyr Tyr Cys Thr
    210                 215                 220

Arg Phe Tyr Ser Tyr Thr Tyr Trp Gly Gln Gly Thr Leu Val Thr Val
225                 230                 235                 240

Ser Ser

<210>  25
<211>  705
<212>  DNA
<213>  artificial sequence

<400>  25
caggtgcagc tggtggagag tggaggtggc gtggtacagc ccggccgcag cctgcgcctg      60

gactgcaagg ccagcggcat caccttcagc aacagcggca tgcactgggt gcgccaggcc     120

cccggcaagg gcctggagtg ggtggccgtg atctggtacg acggcagcaa gcgctactac     180

gccgacagcg tgaagggccg cttcaccatc agccgcgaca acagcaagaa caccctgttc     240

ctgcagatga acagcctgcg cgccgaggac accgccgtgt actactgcgc caccaacgac     300

gactactggg gccagggcac cctggtgacc gtgagcagcg gtggcggtgg ctcgggcggt     360

ggtgggtcgg gtggcggcgg atctgagatc gtgctgaccc agagccccgc caccctgagc     420

ctgagccccg gcgagcgcgc caccctgagc tgccgcgcca gccagagcgt gagcagctac     480

ctggcctggt accagcagaa gccaggacag gctccacgac tgctaatcta tgacgccagc     540

aaccgcgcca ccggcatccc cgcccgcttc agcggcagcg gcagcggcac cgacttcacc     600

ctgaccatca gcagcctgga gcccgaggac ttcgccgtgt actactgcca gcagagcagc     660

aactggcccc gcaccttcgg ccagggcacc aaggtggaga tcaag                     705

<210>  26
<211>  235
<212>  PRT
<213>  artificial sequence

<400>  26

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Gly Gly Gly Gly Ser Gly Gly Gly Gly Ser Gly Gly Gly Gly Ser
        115                 120                 125

Glu Ile Val Leu Thr Gln Ser Pro Ala Thr Leu Ser Leu Ser Pro Gly
    130                 135                 140

Glu Arg Ala Thr Leu Ser Cys Arg Ala Ser Gln Ser Val Ser Ser Tyr
145                 150                 155                 160

Leu Ala Trp Tyr Gln Gln Lys Pro Gly Gln Ala Pro Arg Leu Leu Ile
                165                 170                 175

Tyr Asp Ala Ser Asn Arg Ala Thr Gly Ile Pro Ala Arg Phe Ser Gly
            180                 185                 190

Ser Gly Ser Gly Thr Asp Phe Thr Leu Thr Ile Ser Ser Leu Glu Pro
        195                 200                 205

Glu Asp Phe Ala Val Tyr Tyr Cys Gln Gln Ser Ser Asn Trp Pro Arg
    210                 215                 220

Thr Phe Gly Gln Gly Thr Lys Val Glu Ile Lys
225                 230                 235

<210>  27
<211>  720

<212>  DNA
<213>  artificial sequence

<400>  27
gaggtgcagc tggtggagag cggaggtgga ctagtacagc ctggtggcag cctacgactg      60

agttgcgccg ccagcggctt caccttcagc gacagctgga tccactgggt gcgccaggcc     120

cccggcaagg gcctggagtg ggtggcctgg atcagcccct acggcggcag cacctactac     180

gccgacagcg tgaagggccg cttcaccatc agcgccgaca ccagcaagaa caccgcctac     240

ctgcagatga acagcctgcg cgccgaggac accgccgtgt actactgcgc ccgccgccac     300

tggcccggcg gcttcgacta ctggggccag ggcaccctgg tgaccgtgag cagcggaggc     360

gggggaagcg gcggcggagg gtctggagga gggggcagtg acatccagat gacccagagc     420

cccagcagcc tgagcgccag cgtgggcgac cgcgtgacca tcacctgccg cgccagccag     480

gacgtgagca ccgccgtggc ctggtaccag cagaagcccg gcaaggcccc caagctgctg     540

atctacagcg ccagcttcct gtacagcggc gtgcccagcc gcttcagcgg cagcggcagc     600

ggcaccgact tcaccctgac catcagcagc ctgcagcccg aggacttcgc cacctactac     660

tgccagcagt acctgtacca ccccgccacc ttcggccagg gcaccaaggt ggagatcaag     720

<210>  28
<211>  240
<212>  PRT
<213>  artificial sequence

<400>  28

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Asp Ile Gln Met Thr Gln Ser Pro Ser Ser Leu
    130                 135                 140

Ser Ala Ser Val Gly Asp Arg Val Thr Ile Thr Cys Arg Ala Ser Gln
145                 150                 155                 160

Asp Val Ser Thr Ala Val Ala Trp Tyr Gln Gln Lys Pro Gly Lys Ala
                165                 170                 175

Pro Lys Leu Leu Ile Tyr Ser Ala Ser Phe Leu Tyr Ser Gly Val Pro
            180                 185                 190

Ser Arg Phe Ser Gly Ser Gly Ser Gly Thr Asp Phe Thr Leu Thr Ile
        195                 200                 205

Ser Ser Leu Gln Pro Glu Asp Phe Ala Thr Tyr Tyr Cys Gln Gln Tyr
    210                 215                 220

Leu Tyr His Pro Ala Thr Phe Gly Gln Gly Thr Lys Val Glu Ile Lys
225                 230                 235                 240

<210>  29
<211>  57
<212>  DNA
<213>  artificial sequence

<400>  29
gccaccaact tcagcctgct gaagcaggcc ggcgacgtgg aggagaaccc cggcccc          57

<210>  30
<211>  19
<212>  PRT
<213>  artificial sequence

<400>  30

Ala Thr Asn Phe Ser Leu Leu Lys Gln Ala Gly Asp Val Glu Glu Asn
1               5                   10                  15

Pro Gly Pro

<210>  31
<211>  711
<212>  DNA
<213>  artificial sequence

<400>  31

gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccccagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag c 711

<210> 32
<211> 1044
<212> DNA
<213> artificial sequence

<400> 32
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actg 1044

<210> 33
<211> 1380
<212> DNA
<213> artificial sequence

<400> 33
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actgagagtg aagttcagca ggagcgcaga cgcccccgcg 1080

tacaagcagg gccagaacca gctctataac gagctcaatc taggacgaag agaggagtac 1140

gatgttttgg acaagagacg tggccgggac cctgagatgg ggggaaagcc gagaaggaag 1200

aaccctcagg aaggcctgta caatgaactg cagaaagata agatggcgga ggcctacagt 1260

gagattggga tgaaaggcga gcgccggagg ggcaaggggc acgatggcct ttaccagggt 1320

ctcagtacag ccaccaagga cacctacgac gcccttcaca tgcaggccct gccccctcgc 1380

<210> 34
<211> 3264
<212> DNA
<213> artificial sequence

<400> 34
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actgtaagct agccgactgt gccttctagt tgccagccat 1080

ctgttgtttg cccctccccc gtgccttcct tgaccctgga aggtgccact cccactgtcc 1140

tttcctaata aaatgaggaa attgcatcgc attgtctgag taggtgtcat tctattctgg 1200

ggggtggggt ggggcaggac agcaaggggg aggattggga agacaatagc aggcatgctg 1260

gggatgcggt gggctctatg gcatatgggt atcgataagc ttgatatcga attaggagga 1320

aaaactgttt catacagaag gcgtcaatta ggaggaaaaa ctgtttcata cagaaggcgt 1380

caattaggag gaaaaactgt ttcatacaga aggcgtcaat tggtcccatc gaattaggag 1440

gaaaaactgt ttcatacaga aggcgtcaat taggaggaaa aactgtttca tacagaaggc 1500

gtcaattagg aggaaaaact gtttcataca gaaggcgtca attggtcccg ggacattttg 1560

acacccccat aatatttttc cagaattaac agtataaatt gcatctcttg ttcaagagtt 1620

ccctatcact ctctttaatc actactcaca gtaacctcaa ctcctgtcta gaccaccatg 1680

gagtttgggc tgagctgggt tttcctcgtt gctcttttta gaggtgtaca gtgtatctgg 1740

gagctgaaga aggacgtgta cgtggtggag ctggactggt accccgacgc ccccggcgag 1800

atggtggtat taacctgcga caccccagag gaagacggca tcacctggac cctggaccag 1860

tcaagcgagg tgctgggtag cggcaagacc ctgaccatcc aggtgaagga gttcggcgac 1920

gccggccagt acacctgcca caagggcggc gaggtgctga gccacagcct actgcttctc 1980

cacaagaagg aggacggcat ctggagcacc gacatcctga aggaccagaa ggagcccaag 2040

aacaagacct tcctgcgctg cgaggccaag aattactcag gacgattcac ctgctggtgg 2100

ctgaccacca tcagcaccga cctgaccttc agcgtgaaga gcagccgcgg cagcagcgac 2160

ccccagggcg tgacctgcgg cgccgccacc ctgagcgccg agcgcgtgcg cggcgacaac 2220

aaggagtacg agtacagcgt ggagtgccag gaggacagcg cctgccccgc cgccgaggag 2280

agcctgccca tcgaggtgat ggtggacgcc gtgcacaagc tgaagtacga gaactacacc 2340

agcagcttct tcatccgcga catcatcaag cccgaccccc ccaagaacct gcagctgaag 2400

cccctgaaga acagccgcca ggtggaggtg agctgggagt accccgacac ctggagcacc 2460

ccccacagct acttcagcct gaccttctgc gtgcaggtgc agggcaagag caagcgcgag 2520

aagaaggacc gcgtgttcac cgacaagacc agcgccaccg tgatctgccg caagaatgcg 2580

agcatcagcg tgcgcgccca ggaccgctac tacagcagca gctggagcga gtgggccagc 2640

gtgccctgca gcggaggtgg cggtggaggc agccgcaacc tgcccgtggc cacccccgac 2700

cccggcatgt tcccctgcct gcaccacagc cagaacctgc tgcgcgccgt gagcaacatg 2760

ctgcagaagg cccgccagac cctggagttc tacccctgca ccagcgagga gatcgaccac 2820

gaggacatca ccaaggacaa gaccagcacc gtggaggcct gcctgcccct ggagctgacc 2880

aagaacgaga gctgtctgaa cagcagagag actagcttca tcactaacgg cagctgccta 2940

gccagccgca agaccagctt catgatggcc ctgtgcctga gcagcatcta cgaggacctg 3000

aagatgtacc aggtggagtt caagaccatg aacgccaagc tgctgatgga ccccaagcgc 3060

cagatcttcc tggaccagaa catgctggcc gtgatcgacg agctgatgca ggccctgaac 3120

ttcaacagcg agaccgtgcc ccagaagagc agcctggagg agcccgactt ctacaagacc 3180

aagatcaagc tgtgcatcct gctgcacgcc ttccgcatcc gcgccgtgac catcgaccgc 3240

gtgatgagct acctgaacgc atca 3264

<210> 35
<211> 3600
<212> DNA
<213> artificial sequence

<400> 35
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actgagagtg aagttcagca ggagcgcaga cgcccccgcg 1080

tacaagcagg gccagaacca gctctataac gagctcaatc taggacgaag agaggagtac 1140

gatgttttgg acaagagacg tggccgggac cctgagatgg ggggaaagcc gagaaggaag 1200

aaccctcagg aaggcctgta caatgaactg cagaaagata agatggcgga ggcctacagt 1260

gagattggga tgaaaggcga gcgccggagg ggcaaggggc acgatggcct ttaccagggt 1320

ctcagtacag ccaccaagga cacctacgac gcccttcaca tgcaggccct gccccctcgc 1380

taagctagcc gactgtgcct tctagttgcc agccatctgt tgtttgcccc tcccccgtgc 1440

cttccttgac cctggaaggt gccactccca ctgtcctttc ctaataaaat gaggaaattg 1500

catcgcattg tctgagtagg tgtcattcta ttctgggggg tggggtgggg caggacagca 1560

agggggagga ttgggaagac aatagcaggc atgctgggga tgcggtgggc tctatggcat 1620

atgggtatcg ataagcttga tatcgaatta ggaggaaaaa ctgtttcata cagaaggcgt 1680

caattaggag gaaaaactgt ttcatacaga aggcgtcaat taggaggaaa aactgtttca 1740

tacagaaggc gtcaattggt cccatcgaat taggaggaaa aactgtttca tacagaaggc 1800

gtcaattagg aggaaaaact gtttcataca gaaggcgtca attaggagga aaaactgttt 1860

catacagaag gcgtcaattg gtcccgggac attttgacac ccccataata tttttccaga 1920

attaacagta taaattgcat ctcttgttca agagttccct atcactctct ttaatcacta 1980

ctcacagtaa cctcaactcc tgtctagacc accatggagt ttgggctgag ctgggttttc 2040

ctcgttgctc tttttagagg tgtacagtgt atctgggagc tgaagaagga cgtgtacgtg 2100

gtggagctgg actggtaccc cgacgccccc ggcgagatgg tggtattaac ctgcgacacc 2160

ccagaggaag acggcatcac ctggaccctg gaccagtcaa gcgaggtgct gggtagcggc 2220

aagaccctga ccatccaggt gaaggagttc ggcgacgccg gccagtacac ctgccacaag 2280

ggcggcgagg tgctgagcca cagcctactg cttctccaca agaaggagga cggcatctgg 2340

agcaccgaca tcctgaagga ccagaaggag cccaagaaca agaccttcct gcgctgcgag 2400

gccaagaatt actcaggacg attcacctgc tggtggctga ccaccatcag caccgacctg 2460

accttcagcg tgaagagcag ccgcggcagc agcgaccccc agggcgtgac ctgcggcgcc 2520

gccaccctga gcgccgagcg cgtgcgcggc gacaacaagg agtacgagta cagcgtggag 2580

tgccaggagg acagcgcctg ccccgccgcc gaggagagcc tgcccatcga ggtgatggtg 2640

gacgccgtgc acaagctgaa gtacgagaac tacaccagca gcttcttcat ccgcgacatc 2700

atcaagcccg acccccccaa gaacctgcag ctgaagcccc tgaagaacag ccgccaggtg 2760

gaggtgagct gggagtaccc cgacacctgg agcacccccc acagctactt cagcctgacc 2820

ttctgcgtgc aggtgcaggg caagagcaag cgcgagaaga aggaccgcgt gttcaccgac 2880

aagaccagcg ccaccgtgat ctgccgcaag aatgcgagca tcagcgtgcg cgcccaggac 2940

cgctactaca gcagcagctg gagcgagtgg gccagcgtgc cctgcagcgg aggtggcggt 3000

ggaggcagcc gcaacctgcc cgtggccacc cccgaccccg gcatgttccc ctgcctgcac 3060

cacagccaga acctgctgcg cgccgtgagc aacatgctgc agaaggcccg ccagaccctg 3120

gagttctacc cctgcaccag cgaggagatc gaccacgagg acatcaccaa ggacaagacc 3180

agcaccgtgg aggcctgcct gcccctggag ctgaccaaga acgagagctg tctgaacagc 3240

agagagacta gcttcatcac taacggcagc tgcctagcca gccgcaagac cagcttcatg 3300

atggccctgt gcctgagcag catctacgag gacctgaaga tgtaccaggt ggagttcaag 3360

accatgaacg ccaagctgct gatggacccc aagcgccaga tcttcctgga ccagaacatg 3420

ctggccgtga tcgacgagct gatgcaggcc ctgaacttca acagcgagac cgtgccccag 3480

aagagcagcc tggaggagcc cgacttctac aagaccaaga tcaagctgtg catcctgctg 3540

cacgccttcc gcatccgcgc cgtgaccatc gaccgcgtga tgagctacct gaacgcatca 3600

<210> 36
<211> 2076
<212> DNA
<213> artificial sequence

<400> 36
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccagagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actgtaagct agccgactgt gccttctagt tgccagccat 1080

ctgttgtttg cccctccccc gtgccttcct tgaccctgga aggtgccact cccactgtcc 1140

tttcctaata aaatgaggaa attgcatcgc attgtctgag taggtgtcat tctattctgg 1200

ggggtggggt ggggcaggac agcaaggggg aggattggga agacaatagc aggcatgctg 1260

gggatgcggt gggctctatg gcatatgggt atcgataagc ttgatatcga attaggagga 1320

aaaactgttt catacagaag gcgtcaatta ggaggaaaaa ctgtttcata cagaaggcgt 1380

caattaggag gaaaaactgt ttcatacaga aggcgtcaat tggtcccatc gaattaggag 1440

gaaaaactgt ttcatacaga aggcgtcaat taggaggaaa aactgtttca tacagaaggc 1500

gtcaattagg aggaaaaact gtttcataca gaaggcgtca attggtcccg ggacattttg 1560

acacccccat aatatttttc cagaattaac agtataaatt gcatctcttg ttcaagagtt 1620

ccctatcact ctctttaatc actactcaca gtaacctcaa ctcctgtcta gaccaccatg 1680

gagtttgggc tgagctgggt tttcctcgtt gctcttttta gaggtgtaca gtgtaactgg 1740

gtgaacgtga tcagcgacct gaagaagatc gaggacctga tccagagcat gcacatcgac 1800

gccaccctgt acaccgagag cgacgtgcac cccagctgca aggtgaccgc catgaagtgc 1860

ttcctgctgg agctgcaggt gatcagcctg gagagcggcg acgccagcat ccacgacacc 1920

gtggagaacc tgatcatcct ggccaacaac agcctgagca gcaacggcaa cgtgaccgag 1980

agcggctgca aggagtgcga ggagctggag gagaagaaca tcaaggagtt cctgcagagc 2040

ttcgtgcaca tcgtgcagat gttcatcaac accagc 2076

<210>  37
<211>  2412
<212>  DNA
<213>  artificial sequence

<400>  37
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc      300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag caccacgacg     720

ccagcgccgc gaccaccaac accggcgccc accatcgcgt cgcagcccct gtccctgcgc     780

ccagaggcgt gccggccagc ggcggggggc gcagtgcaca cgagggggct ggacttcgcc     840

tgtgatatct acatctgggc gcccttggcc gggacttgtg gggtccttct cctgtcactg     900

gttatcaccc tttactgcaa acggggcaga aagaaactcc tgtatatatt caaacaacca     960

tttatgagac cagtacaaac tactcaagag gaagatggct gtagctgccg atttccagaa    1020

gaagaagaag gaggatgtga actgagagtg aagttcagca ggagcgcaga cgcccccgcg    1080

tacaagcagg gccagaacca gctctataac gagctcaatc taggacgaag agaggagtac    1140

gatgttttgg acaagagacg tggccgggac cctgagatgg ggggaaagcc gagaaggaag    1200

aaccctcagg aaggcctgta caatgaactg cagaaagata agatggcgga ggcctacagt    1260

gagattggga tgaaaggcga gcgccggagg ggcaaggggc acgatggcct ttaccagggt    1320

ctcagtacag ccaccaagga cacctacgac gcccttcaca tgcaggccct gccccctcgc    1380

taagctagcc gactgtgcct tctagttgcc agccatctgt tgtttgcccc tcccccgtgc    1440

cttccttgac cctggaaggt gccactccca ctgtcctttc ctaataaaat gaggaaattg    1500

catcgcattg tctgagtagg tgtcattcta ttctgggggg tggggtgggg caggacagca    1560

agggggagga ttgggaagac aatagcaggc atgctgggga tgcggtgggc tctatggcat    1620

atgggtatcg ataagcttga tatcgaatta ggaggaaaaa ctgtttcata cagaaggcgt    1680

caattaggag gaaaaactgt ttcatacaga aggcgtcaat taggaggaaa aactgtttca 1740

tacagaaggc gtcaattggt cccatcgaat taggaggaaa aactgtttca tacagaaggc 1800

gtcaattagg aggaaaaact gtttcataca gaaggcgtca attaggagga aaaactgttt 1860

catacagaag gcgtcaattg gtcccgggac attttgacac ccccataata tttttccaga 1920

attaacagta taaattgcat ctcttgttca agagttccct atcactctct ttaatcacta 1980

ctcacagtaa cctcaactcc tgtctagacc accatggagt ttgggctgag ctgggttttc 2040

ctcgttgctc tttttagagg tgtacagtgt aactgggtga acgtgatcag cgacctgaag 2100

aagatcgagg acctgatcca gagcatgcac atcgacgcca ccctgtacac cgagagcgac 2160

gtgcacccca gctgcaaggt gaccgccatg aagtgcttcc tgctggagct gcaggtgatc 2220

agcctggaga gcggcgacgc cagcatccac gacaccgtgg agaacctgat catcctggcc 2280

aacaacagcc tgagcagcaa cggcaacgtg accgagagcg gctgcaagga gtgcgaggag 2340

ctggaggaga agaacatcaa ggagttcctg cagagcttcg tgcacatcgt gcagatgttc 2400

atcaacacca gc 2412

<210> 38
<211> 2205
<212> DNA
<213> artificial sequence

<400> 38
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccccagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actgtaagct agccgactgt gccttctagt tgccagccat 1080

ctgttgtttg cccctccccc gtgccttcct tgaccctgga aggtgccact cccactgtcc 1140

tttcctaata aaatgaggaa attgcatcgc attgtctgag taggtgtcat tctattctgg 1200

ggggtggggt ggggcaggac agcaaggggg aggattggga agacaatagc aggcatgctg 1260

gggatgcggt gggctctatg gcatatgggt atcgataagc ttgatatcga attaggagga 1320

aaaactgttt catacagaag gcgtcaatta ggaggaaaaa ctgtttcata cagaaggcgt 1380

caattaggag gaaaaactgt ttcatacaga aggcgtcaat tggtcccatc gaattaggag 1440

gaaaaactgt ttcatacaga aggcgtcaat taggaggaaa aactgtttca tacagaaggc 1500

gtcaattagg aggaaaaact gtttcataca gaaggcgtca attggtcccg ggacattttg 1560

acacccccat aatatttttc cagaattaac agtataaatt gcatctcttg ttcaagagtt 1620

ccctatcact ctctttaatc actactcaca gtaacctcaa ctcctgtcta gaccaccatg 1680

gagtttgggc tgagctgggt tttcctcgtt gctcttttta gaggtgtaca gtgttacttc 1740

ggcaagctgg agagcaagct gagcgtgatc cgcaacctga acgaccaggt gctgttcatc 1800

gaccagggca accgccccct gttcgaggac atgaccgaca gcgactgccg cgacaacgcc 1860

ccccgcacca tcttcatcat cagcatgtac aaggacagcc agccccgcgg catggccgtg 1920

accatcagcg tgaagtgcga gaagatcagc accctgagct gcgagaacaa gatcatcagc 1980

ttcaaggaga tgaacccccc cgacaacatc aaggacacca agagcgacat catcttcttc 2040

cagcgcagcg tgcccggcca cgacaacaag atgcagttcg agagcagcag ctacgagggc 2100

tacttcctgg cctgcgagaa ggagcgcgac ctgttcaagc tgatcctgaa gaaggaggac 2160

gagctgggcg accgcagcat catgttcacc gtgcagaacg aggac 2205

<210> 39
<211> 2133
<212> DNA
<213> artificial sequence

<400> 39
gacatccaga tgacccagag ccccagcagc ctgagcacca gcgtgggcga ccgcgtgacc 60

ctgacctgca aggccagcca ggacgtgggc accgccgtgg actggtacca gcagaagccc 120

ggccccagcc ccaagctgct gatctactgg gccagcaccc gccacaccgg catccccagc 180

cgcttcagcg gcagcggcag cggcaccgac ttcaccctga ccatcagcag cctgcagccc 240

gaggacttcg ccgactacta ctgccagcag tacaacagct accccctgac cttcggcccc 300

ggcaccaagg tggacatcaa gggtggcggt ggctcgggcg gtggtgggtc gggtggcggc 360

ggatctgagg tgcagctggt gcagagcggc cccgaggtga agaagcccgg cgccaccgtg 420

aagatcagct gcaagaccag cggctacacc ttcaccgagt acaccatcca ctgggtgaag 480

caggcccccg gcaagggcct ggagtggatc ggcaacatca accccaacaa cggcggcacc 540

acctacaacc agaagttcga ggacaaggcc accctgaccg tggacaagag caccgacacc 600

gcctacatgg agctgagcag cctgcgcagc gaggacaccg ccgtgtacta ctgcgccgcc 660

ggctggaact tcgactactg gggccagggc accctactta cagtaagtag caccacgacg 720

ccagcgccgc gaccaccaac accggcgccc accatcgcgt cgcagcccct gtccctgcgc 780

ccagaggcgt gccggccagc ggcggggggc gcagtgcaca cgagggggct ggacttcgcc 840

tgtgatatct acatctgggc gcccttggcc gggacttgtg gggtccttct cctgtcactg 900

gttatcaccc tttactgcaa acggggcaga aagaaactcc tgtatatatt caaacaacca 960

tttatgagac cagtacaaac tactcaagag gaagatggct gtagctgccg atttccagaa 1020

gaagaagaag gaggatgtga actgtaagct agccgactgt gccttctagt tgccagccat 1080

ctgttgtttg cccctccccc gtgccttcct tgaccctgga aggtgccact cccactgtcc 1140

tttcctaata aaatgaggaa attgcatcgc attgtctgag taggtgtcat tctattctgg 1200

ggggtggggt ggggcaggac agcaaggggg aggattggga agacaatagc aggcatgctg 1260

gggatgcggt gggctctatg gcatatgggt atcgataagc ttgatatcga attaggagga 1320

aaaactgttt catacagaag gcgtcaatta ggaggaaaaa ctgtttcata cagaaggcgt 1380

caattaggag gaaaaactgt ttcatacaga aggcgtcaat tggtcccatc gaattaggag 1440

gaaaaactgt ttcatacaga aggcgtcaat taggaggaaa aactgtttca tacagaaggc 1500

gtcaattagg aggaaaaact gtttcataca gaaggcgtca attggtcccg ggacattttg 1560

acacccccat aatatttttc cagaattaac agtataaatt gcatctcttg ttcaagagtt 1620

ccctatcact ctctttaatc actactcaca gtaacctcaa ctcctgtcta gaccaccatg 1680

gagtttgggc tgagctgggt tttcctcgtt gctcttttta gaggtgtaca gtgtgcccca 1740

accagctcaa gtactaagaa gacacagctg cagctggagc acctgctgct ggacctgcag 1800

atgatcctga acggcatcaa caactacaag aaccccaagc tgacccgcat gctgaccttc 1860

aagttctaca tgcccaagaa ggccaccgag ctgaagcacc tgcagtgcct ggaggaggag 1920

ctgaagcccc tggaggaggt gctgaacctg gcccagagca agaacttcca cctgcgcccc 1980

cgcgacctga tcagcaacat caacgtgatc gtgctggagc tgaagggcag cgagaccacc 2040

ttcatgtgcg agtacgccga cgagaccgcc accatcgtgg agttcctgaa ccgctggatc 2100

accttctgcc agagcatcat cagcaccctg acc 2133

<210> 40
<211> 3392

<212>  DNA
<213>  artificial sequence

<400>  40
gacatccaga tgacccagag ccccagcagc ctgagcacca gcgtgggcga ccgcgtgacc      60

ctgacctgca aggccagcca ggacgtgggc accgccgtgg actggtacca gcagaagccc     120

ggccagagcc ccaagctgct gatctactgg gccagcaccc gccacaccgg catccccagc     180

cgcttcagcg gcagcggcag cggcaccgac ttcaccctga ccatcagcag cctgcagccc     240

gaggacttcg ccgactacta ctgccagcag tacaacagct accccctgac cttcggcccc     300

ggcaccaagg tggacatcaa gggtggcggt ggctcgggcg gtggtgggtc gggtggcggc     360

ggatctgagg tgcagctggt gcagagcggc cccgaggtga agaagcccgg cgccaccgtg     420

aagatcagct gcaagaccag cggctacacc ttcaccgagt acaccatcca ctgggtgaag     480

caggcccccg gcaagggcct ggagtggatc ggcaacatca accccaacaa cggcggcacc     540

acctacaacc agaagttcga ggacaaggcc accctgaccg tggacaagag caccgacacc     600

gcctacatgg agctgagcag cctgcgcagc gaggacaccg ccgtgtacta ctgcgccgcc     660

ggctggaact tcgactactg gggccagggc accctactta cagtaagtag caccacgacg     720

ccagcgccgc gaccaccaac accggcgccc accatcgcgt cgcagcccct gtccctgcgc     780

ccagaggcgt gccggccagc ggcggggggc gcagtgcaca cgagggggct ggacttcgcc     840

tgtgatatct acatctgggc gcccttggcc gggacttgtg gggtccttct cctgtcactg     900

gttatcaccc tttactgcaa acggggcaga aagaaactcc tgtatatatt caaacaacca     960

tttatgagac cagtacaaac tactcaagag gaagatggct gtagctgccg atttccagaa    1020

gaagaagaag gaggatgtga actgtaagct agccgactgt gccttctagt tgccagccat    1080

ctgttgtttg cccctccccc gtgccttcct tgaccctgga aggtgccact cccactgtcc    1140

tttcctaata aaatgaggaa attgcatcgc attgtctgag taggtgtcat tctattctgg    1200

ggggtggggt ggggcaggac agcaaggggg aggattggga agacaatagc aggcatgctg    1260

gggatgcggt gggctctatg gcatatgcta ccgggtaggg gaggcgcttt tcccaaggca    1320

gtctggagca tgcgctttag cagccccgct gggcacttgg cgctacacaa gtggcctctg    1380

gcctcgcaca cattccacat ccaccggtag gcgccaaccg gctccgttct ttggtggccc    1440

cttcgcgcca ccttctactc ctcccctagt caggaagttc ccccccgccc cgcagctcgc    1500

gtcgtgcagg acgtgacaaa tggaagtagc acgtctcact agtctcgtgc agatggacag    1560

caccgctgag caatggaagc gggtaggcct ttggggcagc ggccaatagc agctttgctc    1620

cttcgctttc tgggctcaga ggctgggaag gggtgggtcc gggggcgggc tcaggggcgg    1680

gctcaggggc ggggcgggcg cccgaaggtc ctccggaggc ccggcattct gcacgcttca    1740

aaagcgcacg tctgccgcgc tgttctcctc ttcctcatct ccgggccttt cgactctaga    1800

ccaccatgga gtttgggctg agctgggttt tcctcgttgc tctttttaga ggtgtacagt 1860

gtatctggga gctgaagaag gacgtgtacg tggtggagct ggactggtac cccgacgccc 1920

ccggcgagat ggtggtatta acctgcgaca ccccagagga agacggcatc acctggaccc 1980

tggaccagtc aagcgaggtg ctgggtagcg gcaagaccct gaccatccag gtgaaggagt 2040

tcggcgacgc cggccagtac acctgccaca agggcggcga ggtgctgagc cacagcctac 2100

tgcttctcca caagaaggag gacggcatct ggagcaccga catcctgaag gaccagaagg 2160

agcccaagaa caagaccttc ctgcgctgcg aggccaagaa ttactcagga cgattcacct 2220

gctggtggct gaccaccatc agcaccgacc tgaccttcag cgtgaagagc agccgcggca 2280

gcagcgaccc ccagggcgtg acctgcggcg ccgccaccct gagcgccgag cgcgtgcgcg 2340

gcgacaacaa ggagtacgag tacagcgtgg agtgccagga ggacagcgcc tgccccgccg 2400

ccgaggagag cctgcccatc gaggtgatgg tggacgccgt gcacaagctg aagtacgaga 2460

actacaccag cagcttcttc atccgcgaca tcatcaagcc cgaccccccc aagaacctgc 2520

agctgaagcc cctgaagaac agccgccagg tggaggtgag ctgggagtac cccgacacct 2580

ggagcacccc ccacagctac ttcagcctga ccttctgcgt gcaggtgcag ggcaagagca 2640

agcgcgagaa gaaggaccgc gtgttcaccg acaagaccag cgccaccgtg atctgccgca 2700

agaatgcgag catcagcgtg cgcgcccagg accgctacta cagcagcagc tggagcgagt 2760

gggccagcgt gccctgcagc ggaggtggcg gtggaggcag ccgcaacctg cccgtggcca 2820

cccccgaccc cggcatgttc ccctgcctgc accacagcca gaacctgctg cgcgccgtga 2880

gcaacatgct gcagaaggcc cgccagaccc tggagttcta cccctgcacc agcgaggaga 2940

tcgaccacga ggacatcacc aaggacaaga ccagcaccgt ggaggcctgc ctgcccctgg 3000

agctgaccaa gaacgagagc tgtctgaaca gcagagagac tagcttcatc actaacggca 3060

gctgcctagc cagccgcaag accagcttca tgatggccct gtgcctgagc agcatctacg 3120

aggacctgaa gatgtaccag gtggagttca agaccatgaa cgccaagctg ctgatggacc 3180

ccaagcgcca gatcttcctg gaccagaaca tgctggccgt gatcgacgag ctgatgcagg 3240

ccctgaactt caacagcgag accgtgcccc agaagagcag cctggaggag cccgacttct 3300

acaagaccaa gatcaagctg tgcatcctgc tgcacgcctt ccgcatccgc gccgtgacca 3360

tcgaccgcgt gatgagctac ctgaacgcat ca 3392

<210> 41
<211> 1059
<212> DNA
<213> artificial sequence

<400> 41
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactg 1059

<210> 42
<211> 1395
<212> DNA
<213> artificial sequence

<400> 42
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactga gagtgaagtt cagcaggagc 1080

gcagacgccc ccgcgtacaa gcagggccag aaccagctct ataacgagct caatctagga 1140

cgaagagagg agtacgatgt tttggacaag agacgtggcc gggaccctga gatgggggga 1200

aagccgagaa ggaagaaccc tcaggaaggc ctgtacaatg aactgcagaa agataagatg 1260

gcggaggcct acagtgagat tgggatgaaa ggcgagcgcc ggaggggcaa ggggcacgat 1320

ggcctttacc agggtctcag tacagccacc aaggacacct acgacgccct tcacatgcag 1380

gccctgcccc ctcgc 1395

<210> 43
<211> 2463
<212> DNA
<213> artificial sequence

<400> 43
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactgt aagctagccg actgtgcctt 1080

ctagttgcca gccatctgtt gtttgcccct cccccgtgcc ttccttgacc ctggaaggtg 1140

ccactcccac tgtcctttcc taataaaatg aggaaattgc atcgcattgt ctgagtaggt 1200

gtcattctat tctggggggt ggggtggggc aggacagcaa gggggaggat tgggaagaca 1260

atagcaggca tgctggggat gcggtgggct ctatggcata tgggtatcga taagcttgat 1320

atcgaattag gaggaaaaac tgtttcatac agaaggcgtc aattaggagg aaaaactgtt 1380

tcatacagaa ggcgtcaatt aggaggaaaa actgtttcat acagaaggcg tcaattggtc 1440

ccatcgaatt aggaggaaaa actgtttcat acagaaggcg tcaattagga ggaaaaactg 1500

tttcatacag aaggcgtcaa ttaggaggaa aaactgtttc atacagaagg cgtcaattgg 1560

tcccgggaca ttttgacacc cccataatat ttttccagaa ttaacagtat aaattgcatc 1620

tcttgttcaa gagttcccta tcactctctt taatcactac tcacagtaac ctcaactcct 1680

gtctagacca ccatggacat gcgagttcct gcccaactgc ttggccttct tttgctctgg 1740

ctgccaggag ccaagtgcca ggtgcagctg gtggagagtg gaggtggcgt ggtacagccc 1800

ggccgcagcc tgcgcctgga ctgcaaggcc agcggcatca ccttcagcaa cagcggcatg 1860

cactgggtgc gccaggcccc cggcaagggc ctggagtggg tggccgtgat ctggtacgac 1920

ggcagcaagc gctactacgc cgacagcgtg aagggccgct tcaccatcag ccgcgacaac 1980

agcaagaaca ccctgttcct gcagatgaac agcctgcgcg ccgaggacac cgccgtgtac 2040

tactgcgcca ccaacgacga ctactggggc cagggcaccc tggtgaccgt gagcagcggt 2100

ggcggtggct cgggcggtgg tgggtcgggt ggcggcggat ctgagatcgt gctgacccag 2160

agccccgcca ccctgagcct gagccccggc gagcgcgcca ccctgagctg ccgcgccagc 2220

cagagcgtga gcagctacct ggcctggtac cagcagaagc caggacaggc tccacgactg 2280

ctaatctatg acgccagcaa ccgcgccacc ggcatccccg cccgcttcag cggcagcggc 2340

agcggcaccg acttcaccct gaccatcagc agcctggagc ccgaggactt cgccgtgtac 2400

tactgccagc agagcagcaa ctggccccgc accttcggcc agggcaccaa ggtggagatc 2460

aag 2463

<210> 44
<211> 2799
<212> DNA
<213> artificial sequence

<400> 44
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactga gagtgaagtt cagcaggagc 1080

gcagacgccc ccgcgtacaa gcagggccag aaccagctct ataacgagct caatctagga 1140

cgaagagagg agtacgatgt tttggacaag agacgtggcc gggaccctga gatgggggga 1200

aagccgagaa ggaagaaccc tcaggaaggc ctgtacaatg aactgcagaa agataagatg 1260

gcggaggcct acagtgagat tgggatgaaa ggcgagcgcc ggaggggcaa ggggcacgat 1320

ggcctttacc agggtctcag tacagccacc aaggacacct acgacgccct tcacatgcag 1380

gccctgcccc ctcgctaagc tagccgactg tgccttctag ttgccagcca tctgttgttt 1440

gcccctcccc cgtgccttcc ttgaccctgg aaggtgccac tcccactgtc ctttcctaat 1500

aaaatgagga aattgcatcg cattgtctga gtaggtgtca ttctattctg gggggtgggg 1560

tggggcagga cagcaagggg gaggattggg aagacaatag caggcatgct ggggatgcgg 1620

tgggctctat ggcatatggg tatcgataag cttgatatcg aattaggagg aaaaactgtt 1680

tcatacagaa ggcgtcaatt aggaggaaaa actgtttcat acagaaggcg tcaattagga 1740

ggaaaaactg tttcatacag aaggcgtcaa ttggtcccat cgaattagga ggaaaaactg 1800

tttcatacag aaggcgtcaa ttaggaggaa aaactgtttc atacagaagg cgtcaattag 1860

gaggaaaaac tgtttcatac agaaggcgtc aattggtccc gggacatttt gacaccccca 1920

taatattttt ccagaattaa cagtataaat tgcatctctt gttcaagagt tccctatcac 1980

tctctttaat cactactcac agtaacctca actcctgtct agaccaccat ggacatgcga 2040

gttcctgccc aactgcttgg ccttcttttg ctctggctgc caggagccaa gtgccaggtg 2100

cagctggtgg agagtggagg tggcgtggta cagcccggcc gcagcctgcg cctggactgc 2160

aaggccagcg gcatcacctt cagcaacagc ggcatgcact gggtgcgcca ggcccccggc 2220

aagggcctgg agtgggtggc cgtgatctgg tacgacggca gcaagcgcta ctacgccgac 2280

agcgtgaagg gccgcttcac catcagccgc gacaacagca agaacaccct gttcctgcag 2340

atgaacagcc tgcgcgccga ggacaccgcc gtgtactact gcgccaccaa cgacgactac 2400

tggggccagg gcaccctggt gaccgtgagc agcggtggcg gtggctcggg cggtggtggg 2460

tcgggtggcg gcggatctga gatcgtgctg acccagagcc ccgccaccct gagcctgagc 2520

cccggcgagc gcgccaccct gagctgccgc gccagccaga gcgtgagcag ctacctggcc 2580

tggtaccagc agaagccagg acaggctcca cgactgctaa tctatgacgc cagcaaccgc 2640

gccaccggca tccccgcccg cttcagcggc agcggcagcg gcaccgactt caccctgacc 2700

atcagcagcc tggagcccga ggacttcgcc gtgtactact gccagcagag cagcaactgg 2760

ccccgcacct tcggccaggg caccaaggtg gagatcaag 2799

<210> 45
<211> 2478
<212> DNA
<213> artificial sequence

<400> 45
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactgt aagctagccg actgtgcctt 1080

ctagttgcca gccatctgtt gtttgcccct cccccgtgcc ttccttgacc ctggaaggtg 1140

ccactcccac tgtcctttcc taataaaatg aggaaattgc atcgcattgt ctgagtaggt 1200

gtcattctat tctggggggt ggggtggggc aggacagcaa gggggaggat tgggaagaca 1260

atagcaggca tgctggggat gcggtgggct ctatggcata tgggtatcga taagcttgat 1320

atcgaattag gaggaaaaac tgtttcatac agaaggcgtc aattaggagg aaaaactgtt 1380

tcatacagaa ggcgtcaatt aggaggaaaa actgtttcat acagaaggcg tcaattggtc 1440

ccatcgaatt aggaggaaaa actgtttcat acagaaggcg tcaattagga ggaaaaactg 1500

tttcatacag aaggcgtcaa ttaggaggaa aaactgtttc atacagaagg cgtcaattgg 1560

tcccgggaca ttttgacacc cccataatat ttttccagaa ttaacagtat aaattgcatc 1620

tcttgttcaa gagttcccta tcactctctt taatcactac tcacagtaac ctcaactcct 1680

gtctagacca ccatggacat gcgagttcct gcccaactgc ttggccttct tttgctctgg 1740

ctgccaggag ccaagtgcga ggtgcagctg gtggagagcg gaggtggact agtacagcct 1800

ggtggcagcc tacgactgag ttgcgccgcc agcggcttca ccttcagcga cagctggatc 1860

cactgggtgc gccaggcccc cggcaagggc ctggagtggg tggcctggat cagcccctac 1920

ggcggcagca cctactacgc cgacagcgtg aagggccgct tcaccatcag cgccgacacc 1980

agcaagaaca ccgcctacct gcagatgaac agcctgcgcg ccgaggacac cgccgtgtac 2040

tactgcgccc gccgccactg gcccggcggc ttcgactact ggggccaggg caccctggtg 2100

accgtgagca gcggaggcgg gggaagcggc ggcggagggt ctggaggagg gggcagtgac 2160

atccagatga cccagagccc cagcagcctg agcgccagcg tgggcgaccg cgtgaccatc 2220

acctgccgcg ccagccagga cgtgagcacc gccgtggcct ggtaccagca gaagcccggc 2280

aaggccccca agctgctgat ctacagcgcc agcttcctgt acagcggcgt gcccagccgc 2340

ttcagcggca gcggcagcgg caccgacttc accctgacca tcagcagcct gcagcccgag 2400

gacttcgcca cctactactg ccagcagtac ctgtaccacc ccgccacctt cggccagggc 2460

accaaggtgg agatcaag 2478

<210> 46
<211> 2814
<212> DNA
<213> artificial sequence

<400> 46
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactga gagtgaagtt cagcaggagc 1080

gcagacgccc ccgcgtacaa gcagggccag aaccagctct ataacgagct caatctagga 1140

cgaagagagg agtacgatgt tttggacaag agacgtggcc gggaccctga gatgggggga 1200

aagccgagaa ggaagaaccc tcaggaaggc ctgtacaatg aactgcagaa agataagatg 1260

gcggaggcct acagtgagat tgggatgaaa ggcgagcgcc ggaggggcaa ggggcacgat 1320

ggcctttacc agggtctcag tacagccacc aaggacacct acgacgccct tcacatgcag 1380

gccctgcccc ctcgctaagc tagccgactg tgccttctag ttgccagcca tctgttgttt 1440

gcccctcccc cgtgccttcc ttgaccctgg aaggtgccac tcccactgtc ctttcctaat 1500

aaaatgagga aattgcatcg cattgtctga gtaggtgtca ttctattctg gggggtgggg 1560

tggggcagga cagcaagggg gaggattggg aagacaatag caggcatgct ggggatgcgg 1620

tgggctctat ggcatatggg tatcgataag cttgatatcg aattaggagg aaaaactgtt 1680

tcatacagaa ggcgtcaatt aggaggaaaa actgtttcat acagaaggcg tcaattagga 1740

ggaaaaactg tttcatacag aaggcgtcaa ttggtcccat cgaattagga ggaaaaactg 1800

tttcatacag aaggcgtcaa ttaggaggaa aaactgtttc atacagaagg cgtcaattag 1860

gaggaaaaac tgtttcatac agaaggcgtc aattggtccc gggacatttt gacaccccca 1920

taatattttt ccagaattaa cagtataaat tgcatctctt gttcaagagt tccctatcac 1980

tctctttaat cactactcac agtaacctca actcctgtct agaccaccat ggacatgcga 2040

gttcctgccc aactgcttgg ccttcttttg ctctggctgc caggagccaa gtgcgaggtg 2100

cagctggtgg agagcggagg tggactagta cagcctggtg gcagcctacg actgagttgc 2160

gccgccagcg gcttcacctt cagcgacagc tggatccact gggtgcgcca ggcccccggc 2220

aagggcctgg agtgggtggc ctggatcagc ccctacggcg gcagcaccta ctacgccgac 2280

agcgtgaagg gccgcttcac catcagcgcc gacaccagca agaacaccgc ctacctgcag 2340

atgaacagcc tgcgcgccga ggacaccgcc gtgtactact gcgcccgccg ccactggccc 2400

ggcggcttcg actactgggg ccagggcacc ctggtgaccg tgagcagcgg aggcggggga 2460

agcggcggcg gagggtctgg aggagggggc agtgacatcc agatgaccca gagccccagc 2520

agcctgagcg ccagcgtggg cgaccgcgtg accatcacct gccgcgccag ccaggacgtg 2580

agcaccgccg tggcctggta ccagcagaag cccggcaagg cccccaagct gctgatctac 2640

agcgccagct tcctgtacag cggcgtgccc agccgcttca gcggcagcgg cagcggcacc 2700

gacttcaccc tgaccatcag cagcctgcag cccgaggact tcgccaccta ctactgccag 2760

cagtacctgt accaccccgc caccttcggc cagggcacca aggtggagat caag 2814

<210> 47
<211> 2591
<212> DNA
<213> artificial sequence

<400> 47
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactgt aagctagccg actgtgcctt 1080

ctagttgcca gccatctgtt gtttgcccct cccccgtgcc ttccttgacc ctggaaggtg 1140

ccactcccac tgtcctttcc taataaaatg aggaaattgc atcgcattgt ctgagtaggt 1200

gtcattctat tctggggggt ggggtggggc aggacagcaa gggggaggat tgggaagaca 1260

atagcaggca tgctggggat gcggtgggct ctatggcata tgctaccggg taggggaggc 1320

gcttttccca aggcagtctg gagcatgcgc tttagcagcc ccgctgggca cttggcgcta 1380

cacaagtggc ctctggcctc gcacacattc cacatccacc ggtaggcgcc aaccggctcc 1440

gttctttggt ggccccttcg cgccaccttc tactcctccc ctagtcagga agttcccccc 1500

cgccccgcag ctcgcgtcgt gcaggacgtg acaaatggaa gtagcacgtc tcactagtct 1560

cgtgcagatg gacagcaccg ctgagcaatg gaagcgggta ggcctttggg gcagcggcca 1620

atagcagctt tgctccttcg ctttctgggc tcagaggctg ggaaggggtg ggtccggggg 1680

cgggctcagg ggcgggctca ggggcggggc gggcgcccga aggtcctccg gaggcccggc 1740

attctgcacg cttcaaaagc gcacgtctgc cgcgctgttc tcctcttcct catctccggg 1800

cctttcgact ctagaccacc atggacatgc gagttcctgc ccaactgctt ggccttcttt 1860

tgctctggct gccaggagcc aagtgccagg tgcagctggt ggagagtgga ggtggcgtgg 1920

tacagcccgg ccgcagcctg cgcctggact gcaaggccag cggcatcacc ttcagcaaca 1980

gcggcatgca ctgggtgcgc caggcccccg gcaagggcct ggagtgggtg gccgtgatct 2040

ggtacgacgg cagcaagcgc tactacgccg acagcgtgaa gggccgcttc accatcagcc 2100

gcgacaacag caagaacacc ctgttcctgc agatgaacag cctgcgcgcc gaggacaccg 2160

ccgtgtacta ctgcgccacc aacgacgact actggggcca gggcaccctg gtgaccgtga 2220

gcagcggtgg cggtggctcg ggcggtggtg ggtcgggtgg cggcggatct gagatcgtgc 2280

tgacccagag ccccgccacc ctgagcctga gccccggcga gcgcgccacc ctgagctgcc 2340

gcgccagcca gagcgtgagc agctacctgg cctggtacca gcagaagcca ggacaggctc 2400

cacgactgct aatctatgac gccagcaacc gcgccaccgg catccccgcc cgcttcagcg 2460

gcagcggcag cggcaccgac ttcaccctga ccatcagcag cctggagccc gaggacttcg 2520

ccgtgtacta ctgccagcag agcagcaact ggccccgcac cttcggccag ggcaccaagg 2580

tggagatcaa g                                                          2591

<210>  48
<211>  1920
<212>  DNA
<213>  artificial sequence

<400>  48
gacgtggtga tgacccagag ccccctgagc ctgcccgtga cccccggcga gcccgccagc      60

atcagctgcc gcagcagcca gagcctggtg cacagcaacc gcaacaccta cctgcactgg     120

tacctgcaga agcccggcca gagcccccag ctgctgatct acaaggtgag caaccgcttc     180

agcggcgtgc ccgaccgctt cagcggcagc ggcagcggca ccgacttcac cctgaagatc     240

agccgcgtgg aggccgagga cgtgggcgtg tactactgca gccagaacac ccacgtgccc     300

cccaccttcg gccagggcac caagctggag atcaagggtg gcggtggctc gggaggtgga     360

gggtcgggtg gcggcggatc tcaggtgcag ctggtgcaga gcggcgccga ggtgaagaag     420

cccggcgcca gcgtgaaggt gagctgcaag gccagcggct acaccttcac cgactacgag     480

atgcactggg tgcgccaggc ccccggccag ggcctggagt ggatgggcgc cctggacccc     540

aagaccggcg acaccgccta cagccagaag ttcaagggcc gcgtgaccct gaccgccgac     600

aagagcacca gcaccgccta catggagctg agcagcctga ccagcgagga caccgccgtg     660

tactactgca cccgcttcta cagctacacc tactggggcc agggcaccct ggtgaccgtg     720

agcagcacca cgacgccagc gccgcgacca ccaacaccgg cgcccaccat cgcgtcgcag     780

cccctgtccc tgcgcccaga ggcgtgccgg ccagcggcgg ggggcgcagt gcacacgagg     840

gggctggact tcgcctgtga tatctacatc tgggcgccct tggccgggac ttgtggggtc     900

cttctcctgt cactggttat caccctttac tgcaaacggg gcagaaagaa actcctgtat     960

atattcaaac aaccatttat gagaccagta caaactactc aagaggaaga tggctgtagc    1020

tgccgatttc cagaagaaga agaaggagga tgtgaactgg ctagccatat gggcagcggc    1080

gccaccaact tcagcctgct gaagcaggcc ggcgacgtgg aggagaaccc cggcccctct    1140

agagccacca tggacatgcg agttcctgcc caactgcttg gccttctttt gctctggctg    1200

ccaggagcca agtgccaggt gcagctggtg gagagtggag gtggcgtggt acagcccggc    1260

cgcagcctgc gcctggactg caaggccagc ggcatcacct tcagcaacag cggcatgcac    1320

tgggtgcgcc aggcccccgg caagggcctg gagtgggtgg ccgtgatctg gtacgacggc    1380

agcaagcgct actacgccga cagcgtgaag ggccgcttca ccatcagccg cgacaacagc    1440

aagaacaccc tgttcctgca gatgaacagc ctgcgcgccg aggacaccgc cgtgtactac    1500

tgcgccacca acgacgacta ctggggccag ggcaccctgg tgaccgtgag cagcggtggc    1560

ggtggctcgg gcggtggtgg gtcgggtggc ggcggatctg agatcgtgct gacccagagc    1620

cccgccaccc tgagcctgag ccccggcgag cgcgccaccc tgagctgccg cgccagccag 1680

agcgtgagca gctacctggc ctggtaccag cagaagccag gacaggctcc acgactgcta 1740

atctatgacg ccagcaaccg cgccaccggc atccccgccc gcttcagcgg cagcggcagc 1800

ggcaccgact tcaccctgac catcagcagc ctggagcccg aggacttcgc cgtgtactac 1860

tgccagcaga gcagcaactg gccccgcacc ttcggccagg gcaccaaggt ggagatcaag 1920

<210> 49
<211> 2148
<212> DNA
<213> artificial sequence

<400> 49
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactgt aagctagccg actgtgcctt 1080

ctagttgcca gccatctgtt gtttgcccct cccccgtgcc ttccttgacc ctggaaggtg 1140

ccactcccac tgtcctttcc taataaaatg aggaaattgc atcgcattgt ctgagtaggt 1200

gtcattctat tctggggggt ggggtggggc aggacagcaa gggggaggat tgggaagaca 1260

atagcaggca tgctggggat gcggtgggct ctatggcata tgggtatcga taagcttgat 1320

atcgaattag gaggaaaaac tgtttcatac agaaggcgtc aattaggagg aaaaactgtt 1380

tcatacagaa ggcgtcaatt aggaggaaaa actgtttcat acagaaggcg tcaattggtc 1440

ccatcgaatt aggaggaaaa actgtttcat acagaaggcg tcaattagga ggaaaaactg 1500

tttcatacag aaggcgtcaa ttaggaggaa aaactgtttc atacagaagg cgtcaattgg 1560

tcccgggaca ttttgacacc cccataatat ttttccagaa ttaacagtat aaattgcatc 1620

tcttgttcaa gagttcccta tcactctctt taatcactac tcacagtaac ctcaactcct 1680

gtctagacca ccatggagtt tgggctgagc tgggttttcc tcgttgctct ttttagaggt 1740

gtacagtgtg ccccaaccag ctcaagtact aagaagacac agctgcagct ggagcacctg 1800

ctgctggacc tgcagatgat cctgaacggc atcaacaact acaagaaccc caagctgacc 1860

cgcatgctga ccttcaagtt ctacatgccc aagaaggcca ccgagctgaa gcacctgcag 1920

tgcctggagg aggagctgaa gcccctggag gaggtgctga acctggccca gagcaagaac 1980

ttccacctgc gcccccgcga cctgatcagc aacatcaacg tgatcgtgct ggagctgaag 2040

ggcagcgaga ccaccttcat gtgcgagtac gccgacgaga ccgccaccat cgtggagttc 2100

ctgaaccgct ggatcacctt ctgccagagc atcatcagca ccctgacc 2148

<210> 50
<211> 4263
<212> DNA
<213> artificial sequence

<400> 50
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactgt aagctagccg actgtgcctt 1080

ctagttgcca gccatctgtt gtttgcccct cccccgtgcc ttccttgacc ctggaaggtg 1140

ccactcccac tgtcctttcc taataaaatg aggaaattgc atcgcattgt ctgagtaggt 1200

gtcattctat tctggggggt ggggtggggc aggacagcaa gggggaggat tgggaagaca 1260

atagcaggca tgctggggat gcggtgggct ctatggcata tgctaccggg taggggaggc 1320

gcttttccca aggcagtctg gagcatgcgc tttagcagcc ccgctgggca cttggcgcta 1380

cacaagtggc ctctggcctc gcacacattc cacatccacc ggtaggcgcc aaccggctcc 1440

gttctttggt ggccccttcg cgccaccttc tactcctccc ctagtcagga agttcccccc 1500

cgccccgcag ctcgcgtcgt gcaggacgtg acaaatggaa gtagcacgtc tcactagtct 1560

cgtgcagatg gacagcaccg ctgagcaatg gaagcgggta ggcctttggg gcagcggcca 1620

atagcagctt tgctccttcg ctttctgggc tcagaggctg ggaaggggtg ggtccggggg 1680

cgggctcagg ggcgggctca ggggcggggc gggcgcccga aggtcctccg gaggcccggc 1740

attctgcacg cttcaaaagc gcacgtctgc cgcgctgttc tcctcttcct catctccggg 1800

cctttcgaca tctagaccac catggagttt gggctgagct gggttttcct cgttgctctt 1860

tttagaggtg tacagtgtat ctgggagctg aagaaggacg tgtacgtggt ggagctggac 1920

tggtaccccg acgcccccgg cgagatggtg gtattaacct gcgacacccc agaggaagac 1980

ggcatcacct ggaccctgga ccagtcaagc gaggtgctgg gtagcggcaa gaccctgacc 2040

atccaggtga aggagttcgg cgacgccggc cagtacacct gccacaaggg cggcgaggtg 2100

ctgagccaca gcctactgct tctccacaag aaggaggacg gcatctggag caccgacatc 2160

ctgaaggacc agaaggagcc caagaacaag accttcctgc gctgcgaggc caagaattac 2220

tcaggacgat tcacctgctg gtggctgacc accatcagca ccgacctgac cttcagcgtg 2280

aagagcagcc gcggcagcag cgacccccag ggcgtgacct gcggcgccgc caccctgagc 2340

gccgagcgcg tgcgcggcga caacaaggag tacgagtaca gcgtggagtg ccaggaggac 2400

agcgcctgcc ccgccgccga ggagagcctg cccatcgagg tgatggtgga cgccgtgcac 2460

aagctgaagt acgagaacta caccagcagc ttcttcatcc gcgacatcat caagcccgac 2520

ccccccaaga acctgcagct gaagcccctg aagaacagcc gccaggtgga ggtgagctgg 2580

gagtaccccg acacctggag caccccccac agctacttca gcctgacctt ctgcgtgcag 2640

gtgcagggca agagcaagcg cgagaagaag gaccgcgtgt tcaccgacaa gaccagcgcc 2700

accgtgatct gccgcaagaa tgcgagcatc agcgtgcgcg cccaggaccg ctactacagc 2760

agcagctgga gcgagtgggc cagcgtgccc tgcagcggag gtggcggtgg aggcagccgc 2820

aacctgcccg tggccacccc cgaccccggc atgttcccct gcctgcacca cagccagaac 2880

ctgctgcgcg ccgtgagcaa catgctgcag aaggcccgcc agaccctgga gttctacccc 2940

tgcaccagcg aggagatcga ccacgaggac atcaccaagg acaagaccag caccgtggag 3000

gcctgcctgc ccctggagct gaccaagaac gagagctgtc tgaacagcag agagactagc 3060

ttcatcacta acggcagctg cctagccagc cgcaagacca gcttcatgat ggccctgtgc 3120

ctgagcagca tctacgagga cctgaagatg taccaggtgg agttcaagac catgaacgcc 3180

aagctgctga tggaccccaa gcgccagatc ttcctggacc agaacatgct ggccgtgatc 3240

gacgagctga tgcaggccct gaacttcaac agcgagaccg tgccccagaa gagcagcctg 3300

gaggagcccg acttctacaa gaccaagatc aagctgtgca tcctgctgca cgccttccgc 3360

atccgcgccg tgaccatcga ccgcgtgatg agctacctga acgcatcagt taacggcagc 3420

ggcgccacca acttcagcct gctgaagcag gccggcgacg tggaggagaa ccccggcccc 3480

tacgtagcca ccatggacat gcgagttcct gcccaactgc ttggccttct tttgctctgg 3540

ctgccaggag ccaagtgcca ggtgcagctg gtggagagtg gaggtggcgt ggtacagccc 3600

ggccgcagcc tgcgcctgga ctgcaaggcc agcggcatca ccttcagcaa cagcggcatg 3660

cactgggtgc gccaggcccc cggcaagggc ctggagtggg tggccgtgat ctggtacgac 3720

ggcagcaagc gctactacgc cgacagcgtg aagggccgct tcaccatcag ccgcgacaac 3780

agcaagaaca ccctgttcct gcagatgaac agcctgcgcg ccgaggacac cgccgtgtac 3840

tactgcgcca ccaacgacga ctactggggc cagggcaccc tggtgaccgt gagcagcggt 3900

ggcggtggct cgggcggtgg tgggtcgggt ggcggcggat ctgagatcgt gctgacccag 3960

agccccgcca ccctgagcct gagccccggc gagcgcgcca ccctgagctg ccgcgccagc 4020

cagagcgtga gcagctacct ggcctggtac cagcagaagc caggacaggc tccacgactg 4080

ctaatctatg acgccagcaa ccgcgccacc ggcatccccg cccgcttcag cggcagcggc 4140

agcggcaccg acttcaccct gaccatcagc agcctggagc ccgaggactt cgccgtgtac 4200

tactgccagc agagcagcaa ctggccccgc accttcggcc agggcaccaa ggtggagatc 4260

aag 4263

<210> 51
<211> 4134
<212> DNA
<213> artificial sequence

<400> 51
gacgtggtga tgacccagag ccccctgagc ctgcccgtga cccccggcga gcccgccagc 60

atcagctgcc gcagcagcca gagcctggtg cacagcaacc gcaacaccta cctgcactgg 120

tacctgcaga agcccggcca gagcccccag ctgctgatct acaaggtgag caaccgcttc 180

agcggcgtgc ccgaccgctt cagcggcagc ggcagcggca ccgacttcac cctgaagatc 240

agccgcgtgg aggccgagga cgtgggcgtg tactactgca gccagaacac ccacgtgccc 300

cccaccttcg gccagggcac caagctggag atcaagggtg gcggtggctc gggaggtgga 360

gggtcgggtg gcggcggatc tcaggtgcag ctggtgcaga gcggcgccga ggtgaagaag 420

cccggcgcca gcgtgaaggt gagctgcaag gccagcggct acaccttcac cgactacgag 480

atgcactggg tgcgccaggc ccccggccag ggcctggagt ggatgggcgc cctggacccc 540

aagaccggcg acaccgccta cagccagaag ttcaagggcc gcgtgaccct gaccgccgac 600

aagagcacca gcaccgccta catggagctg agcagcctga ccagcgagga caccgccgtg 660

tactactgca cccgcttcta cagctacacc tactggggcc agggcaccct ggtgaccgtg 720

agcagcacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactgt aagctagccg actgtgcctt 1080

ctagttgcca gccatctgtt gtttgcccct cccccgtgcc ttccttgacc ctggaaggtg 1140

ccactcccac tgtcctttcc taataaaatg aggaaattgc atcgcattgt ctgagtaggt 1200

gtcattctat tctggggggt ggggtggggc aggacagcaa gggggaggat tgggaagaca 1260

atagcaggca tgctggggat gcggtgggct ctatggcata tgggtatcga taagcttgat 1320

atcgaattag gaggaaaaac tgtttcatac agaaggcgtc aattaggagg aaaaactgtt 1380

tcatacagaa ggcgtcaatt aggaggaaaa actgtttcat acagaaggcg tcaattggtc 1440

ccatcgaatt aggaggaaaa actgtttcat acagaaggcg tcaattagga ggaaaaactg 1500

tttcatacag aaggcgtcaa ttaggaggaa aaactgtttc atacagaagg cgtcaattgg 1560

tcccgggaca ttttgacacc cccataatat ttttccagaa ttaacagtat aaattgcatc 1620

tcttgttcaa gagttcccta tcactctctt taatcactac tcacagtaac ctcaactcct 1680

gtctagacca ccatggagtt tgggctgagc tgggttttcc tcgttgctct ttttagaggt 1740

gtacagtgta tctgggagct gaagaaggac gtgtacgtgg tggagctgga ctggtacccc 1800

gacgcccccg gcgagatggt ggtattaacc tgcgacaccc cagaggaaga cggcatcacc 1860

tggaccctgg accagtcaag cgaggtgctg ggtagcggca agaccctgac catccaggtg 1920

aaggagttcg gcgacgccgg ccagtacacc tgccacaagg gcggcgaggt gctgagccac 1980

agcctactgc ttctccacaa gaaggaggac ggcatctgga gcaccgacat cctgaaggac 2040

cagaaggagc ccaagaacaa gaccttcctg cgctgcgagg ccaagaatta ctcaggacga 2100

ttcacctgct ggtggctgac caccatcagc accgacctga ccttcagcgt gaagagcagc 2160

cgcggcagca gcgaccccca gggcgtgacc tgcggcgccg ccaccctgag cgccgagcgc 2220

gtgcgcggcg acaacaagga gtacgagtac agcgtggagt gccaggagga cagcgcctgc 2280

cccgccgccg aggagagcct gcccatcgag gtgatggtgg acgccgtgca caagctgaag 2340

tacgagaact acaccagcag cttcttcatc cgcgacatca tcaagcccga cccacccaag 2400

aacctgcagc tgaagcccct gaagaacagc cgccaggtgg aggtgagctg ggagtacccc 2460

gacacctgga gcacccccca cagctacttc agcctgacct tctgcgtgca ggtgcagggc 2520

aagagcaagc gcgagaagaa ggaccgcgtg ttcaccgaca agaccagcgc caccgtgatc 2580

tgccgcaaga atgcgagcat cagcgtgcgc gcccaggacc gctactacag cagcagctgg 2640

agcgagtggg ccagcgtgcc ctgcagcgga ggtggcggtg gaggcagccg caacctgccc 2700

gtggccaccc ccgaccccgg catgttcccc tgcctgcacc acagccagaa cctgctgcgc 2760

gccgtgagca acatgctgca gaaggcccgc cagaccctgg agttctaccc ctgcaccagc 2820

gaggagatcg accacgagga catcaccaag gacaagacca gcaccgtgga ggcctgcctg 2880

cccctggagc tgaccaagaa cgagagctgt ctgaacagca gagagactag cttcatcact 2940

aacggcagct gcctagccag ccgcaagacc agcttcatga tggccctgtg cctgagcagc 3000

atctacgagg acctgaagat gtaccaggtg gagttcaaga ccatgaacgc caagctgctg 3060

atggacccca agcgccagat cttcctggac cagaacatgc tggccgtgat cgacgagctg 3120

atgcaggccc tgaacttcaa cagcgagacc gtgccccaga agagcagcct ggaggagccc 3180

gacttctaca agaccaagat caagctgtgc atcctgctgc acgccttccg catccgcgcc 3240

gtgaccatcg accgcgtgat gagctacctg aacgcatcag ttaacggcag cggcgccacc 3300

aacttcagcc tgctgaagca ggccggcgac gtggaggaga accccggccc ctctagagcc 3360

accatggaca tgcgagttcc tgcccaactg cttggccttc ttttgctctg gctgccagga 3420

gccaagtgcc aggtgcagct ggtggagagt ggaggtggcg tggtacagcc cggccgcagc 3480

ctgcgcctgg actgcaaggc cagcggcatc accttcagca acagcggcat gcactgggtg 3540

cgccaggccc ccggcaaggg cctggagtgg gtggccgtga tctggtacga cggcagcaag 3600

cgctactacg ccgacagcgt gaagggccgc ttcaccatca gccgcgacaa cagcaagaac 3660

accctgttcc tgcagatgaa cagcctgcgc gccgaggaca ccgccgtgta ctactgcgcc 3720

accaacgacg actactgggg ccagggcacc ctggtgaccg tgagcagcgg tggcggtggc 3780

tcgggcggtg gtgggtcggg tggcggcgga tctgagatcg tgctgaccca gagccccgcc 3840

accctgagcc tgagccccgg cgagcgcgcc accctgagct gccgcgccag ccagagcgtg 3900

agcagctacc tggcctggta ccagcagaag ccaggacagg ctccacgact gctaatctat 3960

gacgccagca accgcgccac cggcatcccc gcccgcttca gcggcagcgg cagcggcacc 4020

gacttcaccc tgaccatcag cagcctggag cccgaggact tcgccgtgta ctactgccag 4080

cagagcagca actggccccg caccttcggc cagggcacca aggtggagat caag 4134

<210> 52
<211> 726
<212> DNA
<213> artificial sequence

<400> 52
gatattcaga tgacacagac tactagctct ctgagcgcaa gtttgggcga ccgcgtcaca 60

atctcttgtc gagctagtca agacattagt aagtacctga attggtacca acagaaacca 120

gacggtacag tgaagctgct catttaccac acaagcagac tccacagcgg cgtgccttcc 180

cggttcagtg gctccgggag cggtaccgat tatagcctta cgatctccaa cctcgaacag 240

gaagacatcg ctacatactt ctgtcagcaa ggtaacaccc tcccctacac gttcggggga 300

ggcacaaagt tggagattac cggaggcggc ggttctggtg gcggtgggag cggaggcggt 360

gggagcgaag tcaaactcca ggaatcaggc cctggccttg tcgcccctag tcaaagtctg 420

agcgttacgt gcactgtctc aggtgtttca cttcctgact atggggtatc atggataaga 480

cagcctccac gaaaaggtct tgagtggctc ggagtcatat ggggtagtga gactacttac 540

tataacagcg ccctgaaatc aagattgacc ataatcaaag ataatagtaa gagtcaggta 600

tttttgaaaa tgaactcttt gcaaactgat gataccgcca tttattactg cgctaaacat 660

tactattatg gcggttccta tgcgatggac tattggggcc aaggaacgtc cgtaacggtt 720

tccagt 726

<210> 53
<211> 242
<212> PRT
<213> artificial sequence

<400> 53

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Gly Gly Gly Ser
            100                 105                 110

Gly Gly Gly Gly Ser Gly Gly Gly Gly Ser Glu Val Lys Leu Gln Glu
        115                 120                 125

Ser Gly Pro Gly Leu Val Ala Pro Ser Gln Ser Leu Ser Val Thr Cys
    130                 135                 140

Thr Val Ser Gly Val Ser Leu Pro Asp Tyr Gly Val Ser Trp Ile Arg
145                 150                 155                 160

Gln Pro Pro Arg Lys Gly Leu Glu Trp Leu Gly Val Ile Trp Gly Ser
                165                 170                 175

Glu Thr Thr Tyr Tyr Asn Ser Ala Leu Lys Ser Arg Leu Thr Ile Ile
            180                 185                 190

Lys Asp Asn Ser Lys Ser Gln Val Phe Leu Lys Met Asn Ser Leu Gln
        195                 200                 205

Thr Asp Asp Thr Ala Ile Tyr Tyr Cys Ala Lys His Tyr Tyr Tyr Gly
    210                 215                 220

Gly Ser Tyr Ala Met Asp Tyr Trp Gly Gln Gly Thr Ser Val Thr Val
225                 230                 235                 240

Ser Ser

<210>  54
<211>  1395
<212>  DNA
<213>  artificial sequence

<400>  54
gacatccaga tgacacagac tacatcctcc ctgtctgcct ctctgggaga cagagtcacc      60

atcagttgca gggcaagtca ggacattagt aaatatttaa attggtatca gcagaaacca     120

gatggaactg ttaaactcct gatctaccat acatcaagat tacactcagg agtcccatca     180

aggttcagtg gcagtgggtc tggaacagat tattctctca ccattagcaa cctggagcaa     240

gaagatattg ccacttactt ttgccaacag ggtaatacgc ttccgtacac gttcggaggg 300

gggaccaagc tggagatcac aggtggcggt ggctcgggcg gtggtgggtc gggtggcggc 360

ggatctgagg tgaaactgca ggagtcagga cctggcctgg tggcgccctc acagagcctg 420

tccgtcacat gcactgtctc aggggtctca ttacccgact atggtgtaag ctggattcgc 480

cagcctccac gaaagggtct ggagtggctg ggagtaatat ggggtagtga aaccacatac 540

tataattcag ctctcaaatc cagactgacc atcatcaagg acaactccaa gagccaagtt 600

ttcttaaaaa tgaacagtct gcaaactgat gacacagcca tttactactg tgccaaacat 660

tattactacg gtggtagcta tgctatggac tactggggcc aaggaacctc agtcaccgtc 720

tcctcaacca cgacgccagc gccgcgacca ccaacaccgg cgcccaccat cgcgtcgcag 780

cccctgtccc tgcgcccaga ggcgtgccgg ccagcggcgg ggggcgcagt gcacacgagg 840

gggctggact tcgcctgtga tatctacatc tgggcgccct tggccgggac ttgtggggtc 900

cttctcctgt cactggttat caccctttac tgcaaacggg gcagaaagaa actcctgtat 960

atattcaaac aaccatttat gagaccagta caaactactc aagaggaaga tggctgtagc 1020

tgccgatttc cagaagaaga agaaggagga tgtgaactga gagtgaagtt cagcaggagc 1080

gcagacgccc ccgcgtacaa gcagggccag aaccagctct ataacgagct caatctagga 1140

cgaagagagg agtacgatgt tttggacaag agacgtggcc gggaccctga gatgggggga 1200

aagccgagaa ggaagaaccc tcaggaaggc ctgtacaatg aactgcagaa agataagatg 1260

gcggaggcct acagtgagat tgggatgaaa ggcgagcgcc ggaggggcaa ggggcacgat 1320

ggcctttacc agggtctcag tacagccacc aaggacacct acgacgccct tcacatgcag 1380

gccctgcccc ctcgc 1395

<210> 55
<211> 465
<212> PRT
<213> artificial sequence

<400> 55

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Gly Gly Gly Ser
            100                 105                 110

Gly Gly Gly Gly Ser Gly Gly Gly Gly Ser Glu Val Lys Leu Gln Glu
        115                 120                 125

Ser Gly Pro Gly Leu Val Ala Pro Ser Gln Ser Leu Ser Val Thr Cys
    130                 135                 140

Thr Val Ser Gly Val Ser Leu Pro Asp Tyr Gly Val Ser Trp Ile Arg
145                 150                 155                 160

Gln Pro Pro Arg Lys Gly Leu Glu Trp Leu Gly Val Ile Trp Gly Ser
                165                 170                 175

Glu Thr Thr Tyr Tyr Asn Ser Ala Leu Lys Ser Arg Leu Thr Ile Ile
            180                 185                 190

Lys Asp Asn Ser Lys Ser Gln Val Phe Leu Lys Met Asn Ser Leu Gln
        195                 200                 205

Thr Asp Asp Thr Ala Ile Tyr Tyr Cys Ala Lys His Tyr Tyr Tyr Gly
    210                 215                 220

Gly Ser Tyr Ala Met Asp Tyr Trp Gly Gln Gly Thr Ser Val Thr Val
225                 230                 235                 240

Ser Ser Thr Thr Thr Pro Ala Pro Arg Pro Pro Thr Pro Ala Pro Thr
                245                 250                 255

Ile Ala Ser Gln Pro Leu Ser Leu Arg Pro Glu Ala Cys Arg Pro Ala
            260                 265                 270

Ala Gly Gly Ala Val His Thr Arg Gly Leu Asp Phe Ala Cys Asp Ile
        275                 280                 285

Tyr Ile Trp Ala Pro Leu Ala Gly Thr Cys Gly Val Leu Leu Leu Ser
    290                 295                 300

Leu Val Ile Thr Leu Tyr Cys Lys Arg Gly Arg Lys Lys Leu Leu Tyr
305                 310                 315                 320

Ile Phe Lys Gln Pro Phe Met Arg Pro Val Gln Thr Thr Gln Glu Glu
                325                 330                 335

Asp Gly Cys Ser Cys Arg Phe Pro Glu Glu Glu Glu Gly Gly Cys Glu
            340                 345                 350

Leu Arg Val Lys Phe Ser Arg Ser Ala Asp Ala Pro Ala Tyr Lys Gln
        355                 360                 365

Gly Gln Asn Gln Leu Tyr Asn Glu Leu Asn Leu Gly Arg Arg Glu Glu
    370                 375                 380

Tyr Asp Val Leu Asp Lys Arg Arg Gly Arg Asp Pro Glu Met Gly Gly
385                 390                 395                 400

Lys Pro Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465

<210>  56
<211>  714
<212>  DNA
<213>  artificial sequence

<400>  56
gaggtgcagc tggtggagag cggaggcggt ctggtgcagc ccggaggtag cctacgtctg      60

agctgcgctg caagcggcta ctcaatcacc aacgactacg cctggaactg ggtgcgccag     120

gcacctggca agggcctgga gtgggtgggc tacatcagct acagcggcta caccacctac     180

aaccccagcc tgaagagccg cttcaccatc agccgcgaca ccagcaagaa caccctgtac     240

ctgcagatga acagcctgcg cgccgaggac accgccgtgt actactgcgc tcgctggact     300

agcggtctgg actactgggg acagggtact ctggtgaccg tgagcagcgg tggcggtggc     360

tcgggaggtg gagggtcggg tggcggcgga tctgacatcc agatgaccca gagccccagc     420

agcctgagcg ccagcgtggg cgaccgcgtg accatcacct gcaaggccag cgacctgatc     480

cacaactggc tggcctggta ccagcagaag cccggcaagg cccccaagct gctgatctac 540

ggcgccacca gcctggagac cggcgtgccc agccgcttca gcggcagcgg cagcggcacc 600

gacttcaccc tgaccatcag cagcctgcag cccgaggact tcgccaccta ctactgccag 660

cagtactgga ccaccccctt caccttcggc cagggcacca aggtggagat caag 714

<210> 57
<211> 238
<212> PRT
<213> artificial sequence

<400> 57

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Ile Thr Asn Asp
            20                  25                  30

Tyr Ala Trp Asn Trp Val Arg Gln Ala Pro Gly Lys Gly Leu Glu Trp
        35                  40                  45

Val Gly Tyr Ile Ser Tyr Ser Gly Tyr Thr Thr Tyr Asn Pro Ser Leu
    50                  55                  60

Lys Ser Arg Phe Thr Ile Ser Arg Asp Thr Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Trp Thr Ser Gly Leu Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Asp Ile Gln Met Thr Gln Ser Pro Ser Ser Leu Ser Ala
    130                 135                 140

Ser Val Gly Asp Arg Val Thr Ile Thr Cys Lys Ala Ser Asp Leu Ile
145                 150                 155                 160

His Asn Trp Leu Ala Trp Tyr Gln Gln Lys Pro Gly Lys Ala Pro Lys
                165                 170                 175

Leu Leu Ile Tyr Gly Ala Thr Ser Leu Glu Thr Gly Val Pro Ser Arg
            180                 185                 190

Phe Ser Gly Ser Gly Ser Gly Thr Asp Phe Thr Leu Thr Ile Ser Ser
        195                 200                 205

Leu Gln Pro Glu Asp Phe Ala Thr Tyr Tyr Cys Gln Gln Tyr Trp Thr
    210                 215                 220

Thr Pro Phe Thr Phe Gly Gln Gly Thr Lys Val Glu Ile Lys
225                 230                 235

<210>  58
<211>  756
<212>  DNA
<213>  artificial sequence

<400>  58
gacattgtta tgacgcagag ccctgattcc ctcgccgtgt cactgggcga gagggcgact        60

atcaactgta aaagcagcca atcactgctc tatagccgaa accagaaaaa ctacctcgcg       120

tggtaccaac agaaaccagg gcagccacct aagctgctca ttttttgggc aagtacgcgc       180

gaatctggcg tgcctgacag gttttctggg agtggttttg gcaccgattt tacattgaca       240

atttccagct tgcaagcaga agatgtcgct gtgtactatt gtcaacagta cttcagctat       300

cctcttacgt ttgggcaagg aaccaaggtg gagatcaaag gtggcggtgg ctcgggcggt       360

ggtgggtcgg gtggcggcgg atctcaggtc cagctcgtcc agtccggggc tgaggttaag       420

aagccgggtg cttctgtgaa agtgtcatgc aaaacgagcc gatatacctt tacggaatat       480

acgatacact gggtgaggca agcgccgggt cagaggttgg aatggatcgg tgggatcaac       540

cctaataatg ggatacctaa ctataatcag aaatttaaag gccgggttac tattaccgtg       600

gatacgtccg cgtcaacagc ctacatggaa ctctcctcac tcaggagcga agatactgcg       660

gtgtattact gtgcccgccg ccgcattgcg tatggatacg atgaagggca cgcgatggac       720

tactggggac aaggtacact tgtgacggtc tcatca                                 756

<210>  59
<211>  252
<212>  PRT
<213>  artificial sequence

<400>  59

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln

35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys Gly Gly Gly Gly Ser Gly Gly Gly Gly Ser Gly Gly Gly Gly Ser
        115                 120                 125

Gln Val Gln Leu Val Gln Ser Gly Ala Glu Val Lys Lys Pro Gly Ala
    130                 135                 140

Ser Val Lys Val Ser Cys Lys Thr Ser Arg Tyr Thr Phe Thr Glu Tyr
145                 150                 155                 160

Thr Ile His Trp Val Arg Gln Ala Pro Gly Gln Arg Leu Glu Trp Ile
                165                 170                 175

Gly Gly Ile Asn Pro Asn Asn Gly Ile Pro Asn Tyr Asn Gln Lys Phe
            180                 185                 190

Lys Gly Arg Val Thr Ile Thr Val Asp Thr Ser Ala Ser Thr Ala Tyr
        195                 200                 205

Met Glu Leu Ser Ser Leu Arg Ser Glu Asp Thr Ala Val Tyr Tyr Cys
    210                 215                 220

Ala Arg Arg Arg Ile Ala Tyr Gly Tyr Asp Glu Gly His Ala Met Asp
225                 230                 235                 240

Tyr Trp Gly Gln Gly Thr Leu Val Thr Val Ser Ser
                245                 250

<210>  60
<211>  111
<212>  DNA
<213>  artificial sequence

<400>  60
cggagggacc agaggctgcc ccccgatgcc cacaagcccc ctgggggagg cagtttccgg        60

acccccatcc aagaggagca ggccgacgcc cactccaccc tggccaagat c                111

<210>  61
<211>  37
<212>  PRT
<213>  artificial sequence

<400>  61

Arg Arg Asp Gln Arg Leu Pro Pro Asp Ala His Lys Pro Pro Gly Gly
1               5                   10                  15

Gly Ser Phe Arg Thr Pro Ile Gln Glu Glu Gln Ala Asp Ala His Ser
            20                  25                  30

Thr Leu Ala Lys Ile
        35

<210>  62
<211>  5
<212>  PRT
<213>  artificial sequence

<400>  62

Asn Ser Gly Met His
1               5

<210>  63
<211>  17
<212>  PRT
<213>  artificial sequence

<400>  63

Val Ile Trp Tyr Asp Gly Ser Lys Arg Tyr Tyr Ala Asp Ser Val Lys
1               5                   10                  15

Gly

<210>  64
<211>  4
<212>  PRT
<213>  artificial sequence

<400>  64

Asn Asp Asp Tyr
1

<210>  65
<211>  11
<212>  PRT
<213>  artificial sequence

<400>  65

Arg Ala Ser Gln Ser Val Ser Ser Tyr Leu Ala
1               5                   10


<210>  66
<211>  7
<212>  PRT
<213>  artificial sequence

<400>  66

Asp Ala Ser Asn Arg Ala Thr
1               5


<210>  67
<211>  9
<212>  PRT
<213>  artificial sequence

<400>  67

Gln Gln Ser Ser Asn Trp Pro Arg Thr
1               5


<210>  68
<211>  5
<212>  PRT
<213>  artificial sequence

<400>  68

Asp Ser Trp Ile His
1               5


<210>  69
<211>  17
<212>  PRT
<213>  artificial sequence

<400>  69

Trp Ile Ser Pro Tyr Gly Gly Ser Thr Tyr Tyr Ala Asp Ser Val Lys
1               5                   10                  15


Gly


<210>  70
<211>  9
<212>  PRT
<213>  artificial sequence

<400>  70

Arg His Trp Pro Gly Gly Phe Asp Tyr

1               5


<210>  71
<211>  11
<212>  PRT
<213>  artificial sequence

<400>  71

Arg Ala Ser Gln Asp Val Ser Thr Ala Val Ala
1               5                   10


<210>  72
<211>  7
<212>  PRT
<213>  artificial sequence

<400>  72

Ser Ala Ser Phe Leu Tyr Ser
1               5


<210>  73
<211>  9
<212>  PRT
<213>  artificial sequence

<400>  73

Gln Gln Tyr Leu Tyr His Pro Ala Thr
1               5


<210>  74
<211>  5
<212>  PRT
<213>  artificial sequence

<400>  74

Glu Tyr Thr Ile His
1               5


<210>  75
<211>  17
<212>  PRT
<213>  artificial sequence

<400>  75

Asn Ile Asn Pro Asn Asn Gly Gly Thr Thr Tyr Asn Gln Lys Phe Glu
1               5                   10                  15


Asp



<210>  76

<211>  6
<212>  PRT
<213>  artificial sequence

<400>  76

Gly Trp Asn Phe Asp Tyr
1               5



<210>  77
<211>  11
<212>  PRT
<213>  artificial sequence

<400>  77

Lys Ala Ser Gln Asp Val Gly Thr Ala Val Asp
1               5                   10



<210>  78
<211>  7
<212>  PRT
<213>  artificial sequence

<400>  78

Trp Ala Ser Thr Arg His Thr
1               5



<210>  79
<211>  9
<212>  PRT
<213>  artificial sequence

<400>  79

Gln Gln Tyr Asn Ser Tyr Pro Leu Thr
1               5



<210>  80
<211>  5
<212>  PRT
<213>  artificial sequence

<400>  80

Asp Tyr Glu Met His
1               5



<210>  81
<211>  17
<212>  PRT
<213>  artificial sequence

<400>  81

Ala Leu Asp Pro Lys Thr Gly Asp Thr Ala Tyr Ser Gln Lys Phe Lys
1               5                   10                  15

Gly

<210> 82
<211> 6
<212> PRT
<213> artificial sequence

<400> 82

Phe Tyr Ser Tyr Thr Tyr
1               5

<210> 83
<211> 16
<212> PRT
<213> artificial sequence

<400> 83

Arg Ser Ser Gln Ser Leu Val His Ser Asn Arg Asn Thr Tyr Leu His
1               5                   10                  15

<210> 84
<211> 7
<212> PRT
<213> artificial sequence

<400> 84

Lys Val Ser Asn Arg Phe Ser
1               5

<210> 85
<211> 9
<212> PRT
<213> artificial sequence

<400> 85

Ser Gln Asn Thr His Val Pro Pro Thr
1               5

<210> 86
<211> 5
<212> PRT
<213> artificial sequence

<400> 86

Asp Tyr Gly Val Ser
1               5

<210> 87
<211> 16

<212>  PRT
<213>  artificial sequence

<400>  87

Val Ile Trp Gly Ser Glu Thr Thr Tyr Tyr Asn Ser Ala Leu Lys Ser
1               5                   10                  15


<210>  88
<211>  12
<212>  PRT
<213>  artificial sequence

<400>  88

His Tyr Tyr Tyr Gly Gly Ser Tyr Ala Met Asp Tyr
1               5                   10


<210>  89
<211>  11
<212>  PRT
<213>  artificial sequence

<400>  89

Arg Ala Ser Gln Asp Ile Ser Lys Tyr Leu Asn
1               5                   10


<210>  90
<211>  7
<212>  PRT
<213>  artificial sequence

<400>  90

His Thr Ser Arg Leu His Ser
1               5


<210>  91
<211>  9
<212>  PRT
<213>  artificial sequence

<400>  91

Gln Gln Gly Asn Thr Leu Pro Tyr Thr
1               5


<210>  92
<211>  18
<212>  PRT
<213>  artificial sequence

<400>  92

Glu Gly Arg Gly Ser Leu Leu Thr Cys Gly Asp Val Glu Glu Asn Pro
1               5                   10                  15

Gly Pro

<210> 93
<211> 6
<212> PRT
<213> artificial sequence

<400> 93

Asn Asp Tyr Ala Trp Asn
1               5

<210> 94
<211> 16
<212> PRT
<213> artificial sequence

<400> 94

Tyr Ile Ser Tyr Ser Gly Tyr Thr Thr Tyr Asn Pro Ser Leu Lys Ser
1               5                   10                  15

<210> 95
<211> 7
<212> PRT
<213> artificial sequence

<400> 95

Trp Thr Ser Gly Leu Asp Tyr
1               5

<210> 96
<211> 11
<212> PRT
<213> artificial sequence

<400> 96

Lys Ala Ser Asp Leu Ile His Asn Trp Leu Ala
1               5                   10

<210> 97
<211> 7
<212> PRT
<213> artificial sequence

<400> 97

Gly Ala Thr Ser Leu Glu Thr
1               5

<210> 98
<211> 9
<212> PRT

<213>  artificial sequence

<400>  98

Gln Gln Tyr Trp Thr Thr Pro Phe Thr
1               5


<210>  99
<211>  5
<212>  PRT
<213>  artificial sequence

<400>  99

Glu Tyr Thr Ile His
1               5


<210>  100
<211>  17
<212>  PRT
<213>  artificial sequence

<400>  100

Gly Ile Asn Pro Asn Asn Gly Ile Pro Asn Tyr Asn Gln Lys Phe Lys
1               5                   10                  15


Gly



<210>  101
<211>  15
<212>  PRT
<213>  artificial sequence

<400>  101

Arg Arg Ile Ala Tyr Gly Tyr Asp Glu Gly His Ala Met Asp Tyr
1               5                   10                  15


<210>  102
<211>  17
<212>  PRT
<213>  artificial sequence

<400>  102

Lys Ser Ser Gln Ser Leu Leu Tyr Ser Arg Asn Gln Lys Asn Tyr Leu
1               5                   10                  15


Ala



<210>  103
<211>  7
<212>  PRT

<213>  artificial sequence

<400>  103

Trp Ala Ser Thr Arg Glu Ser
1               5


<210>  104
<211>  9
<212>  PRT
<213>  artificial sequence

<400>  104

Gln Gln Tyr Phe Ser Tyr Pro Leu Thr
1               5


<210>  105
<211>  115
<212>  PRT
<213>  artificial sequence

<400>  105

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Glu Met His Trp Val Arg Gln Ala Pro Gly Gln Gly Leu Glu Trp Met
        35                  40                  45


Gly Ala Leu Asp Pro Lys Thr Gly Asp Thr Ala Tyr Ser Gln Lys Phe
    50                  55                  60


Lys Gly Arg Val Thr Leu Thr Ala Asp Lys Ser Thr Ser Thr Ala Tyr
65                  70                  75                  80


Met Glu Leu Ser Ser Leu Thr Ser Glu Asp Thr Ala Val Tyr Tyr Cys
                85                  90                  95


Thr Arg Phe Tyr Ser Tyr Thr Tyr Trp Gly Gln Gly Thr Leu Val Thr
            100                 105                 110


Val Ser Ser
        115


<210>  106
<211>  112
<212>  PRT
<213>  artificial sequence

<400>  106

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110


<210>  107
<211>  115
<212>  PRT
<213>  artificial sequence

<400>  107

Glu Val Gln Leu Val Gln Ser Gly Pro Glu Val Lys Lys Pro Gly Ala
1               5                   10                  15

Thr Val Lys Ile Ser Cys Lys Thr Ser Gly Tyr Thr Phe Thr Glu Tyr
            20                  25                  30

Thr Ile His Trp Val Lys Gln Ala Pro Gly Lys Gly Leu Glu Trp Ile
        35                  40                  45

Gly Asn Ile Asn Pro Asn Asn Gly Gly Thr Thr Tyr Asn Gln Lys Phe
    50                  55                  60

Glu Asp Lys Ala Thr Leu Thr Val Asp Lys Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Ala Gly Trp Asn Phe Asp Tyr Trp Gly Gln Gly Thr Leu Leu Thr
            100                 105                 110

Val Ser Ser
        115

<210>  108
<211>  107
<212>  PRT
<213>  artificial sequence

<400>  108

Asp Ile Gln Met Thr Gln Ser Pro Ser Ser Leu Ser Thr Ser Val Gly
1               5                   10                  15

Asp Arg Val Thr Leu Thr Cys Lys Ala Ser Gln Asp Val Gly Thr Ala
            20                  25                  30

Val Asp Trp Tyr Gln Gln Lys Pro Gly Pro Ser Pro Lys Leu Leu Ile
        35                  40                  45

Tyr Trp Ala Ser Thr Arg His Thr Gly Ile Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Asp Tyr Tyr Cys Gln Gln Tyr Asn Ser Tyr Pro Leu
                85                  90                  95

Thr Phe Gly Pro Gly Thr Lys Val Asp Ile Lys
            100                 105

<210>  109
<211>  113
<212>  PRT
<213>  artificial sequence

<400>  109

<210>  110
<211>  107
<212>  PRT
<213>  artificial sequence

<400>  110

<210>  111
<211>  118
<212>  PRT
<213>  artificial sequence

<400>  111

<210>  112
<211>  107
<212>  PRT
<213>  artificial sequence

<400>  112

<210>  113
<211>  120
<212>  PRT
<213>  artificial sequence

<400>  113

Glu Val Lys Leu Gln Glu Ser Gly Pro Gly Leu Val Ala Pro Ser Gln
1               5                   10                  15

Ser Leu Ser Val Thr Cys Thr Val Ser Gly Val Ser Leu Pro Asp Tyr
            20                  25                  30

Gly Val Ser Trp Ile Arg Gln Pro Pro Arg Lys Gly Leu Glu Trp Leu
        35                  40                  45

Gly Val Ile Trp Gly Ser Glu Thr Thr Tyr Tyr Asn Ser Ala Leu Lys
    50                  55                  60

Ser Arg Leu Thr Ile Ile Lys Asp Asn Ser Lys Ser Gln Val Phe Leu
65                  70                  75                  80

Lys Met Asn Ser Leu Gln Thr Asp Asp Thr Ala Ile Tyr Tyr Cys Ala
                85                  90                  95

Lys His Tyr Tyr Tyr Gly Gly Ser Tyr Ala Met Asp Tyr Trp Gly Gln
            100                 105                 110

Gly Thr Ser Val Thr Val Ser Ser
        115                 120


<210>  114
<211>  107
<212>  PRT
<213>  artificial sequence

<400>  114

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln

65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr
            100                 105

<210>  115
<211>  116
<212>  PRT
<213>  artificial sequence

<400>  115

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Ile Thr Asn Asp
            20                  25                  30

Tyr Ala Trp Asn Trp Val Arg Gln Ala Pro Gly Lys Gly Leu Glu Trp
        35                  40                  45

Val Gly Tyr Ile Ser Tyr Ser Gly Tyr Thr Thr Tyr Asn Pro Ser Leu
    50                  55                  60

Lys Ser Arg Phe Thr Ile Ser Arg Asp Thr Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Trp Thr Ser Gly Leu Asp Tyr Trp Gly Gln Gly Thr Leu Val
            100                 105                 110

Thr Val Ser Ser
        115

<210>  116
<211>  107
<212>  PRT
<213>  artificial sequence

<400>  116

Asp Ile Gln Met Thr Gln Ser Pro Ser Ser Leu Ser Ala Ser Val Gly
1               5                   10                  15

Asp Arg Val Thr Ile Thr Cys Lys Ala Ser Asp Leu Ile His Asn Trp
            20                  25                  30

Leu Ala Trp Tyr Gln Gln Lys Pro Gly Lys Ala Pro Lys Leu Leu Ile
        35                  40                  45

Tyr Gly Ala Thr Ser Leu Glu Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Trp Thr Thr Pro Phe
                85                  90                  95

Thr Phe Gly Gln Gly Thr Lys Val Glu Ile Lys
            100                 105

<210>  117
<211>  124
<212>  PRT
<213>  artificial sequence

<400>  117

Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120

<210>  118
<211>  113

<212>  PRT
<213>  artificial sequence

<400>  118

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys



<210>  119
<211>  119
<212>  PRT
<213>  artificial sequence

<400>  119

<210>  120
<211>  107
<212>  PRT
<213>  artificial sequence

<400>  120

<210>  121
<211>  120
<212>  PRT
<213>  artificial sequence

<400>  121

<210>  122
<211>  107
<212>  PRT
<213>  artificial sequence

<400>  122

<210>  123
<211>  122
<212>  PRT
<213>  artificial sequence

<400>  123

Glu Val Gln Leu Gln Gln Ser Gly Pro Glu Leu Val Lys Pro Gly Ala
1               5                   10                  15

Ser Met Lys Ile Ser Cys Lys Ala Ser Gly Tyr Ser Phe Thr Gly Tyr
            20                  25                  30

Thr Met Asn Trp Val Lys Gln Ser His Gly Lys Asn Leu Glu Trp Met
        35                  40                  45

Gly Leu Ile Asn Pro Tyr Lys Gly Val Ser Thr Tyr Asn Gln Lys Phe
    50                  55                  60

Lys Asp Lys Ala Thr Leu Thr Val Asp Lys Ser Ser Ser Thr Ala Tyr
65                  70                  75                  80

Met Glu Leu Leu Ser Leu Thr Ser Glu Asp Ser Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Thr Leu Thr Val Phe Ser
        115                 120


<210>  124
<211>  107
<212>  PRT
<213>  artificial sequence

<400>  124

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Arg Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Lys Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Trp

85                  90                  95

Thr Phe Ala Gly Gly Thr Lys Leu Glu Ile Lys
            100                 105

<210>  125
<211>  528
<212>  PRT
<213>  artificial sequence

<400>  125

Met Ala Leu Pro Val Thr Ala Leu Leu Leu Pro Leu Ala Leu Leu Leu
1               5                   10                  15

His Ala Ala Arg Pro Asp Ile Gln Met Thr Gln Thr Thr Ser Ser Leu
            20                  25                  30

Ser Ala Ser Leu Gly Asp Arg Val Thr Ile Ser Cys Arg Ala Ser Gln
        35                  40                  45

Asp Ile Ser Lys Tyr Leu Asn Trp Tyr Gln Gln Lys Pro Asp Gly Thr
    50                  55                  60

Val Lys Leu Leu Ile Tyr His Thr Ser Arg Leu His Ser Gly Val Pro
65                  70                  75                  80

Ser Arg Phe Ser Gly Ser Gly Ser Gly Thr Asp Tyr Ser Leu Thr Ile
                85                  90                  95

Ser Asn Leu Glu Gln Glu Asp Ile Ala Thr Tyr Phe Cys Gln Gln Gly
            100                 105                 110

Asn Thr Leu Pro Tyr Thr Phe Gly Gly Gly Thr Lys Leu Glu Ile Thr
        115                 120                 125

Gly Gly Gly Gly Ser Gly Gly Gly Gly Ser Gly Gly Gly Gly Ser Glu
    130                 135                 140

Val Lys Leu Gln Glu Ser Gly Pro Gly Leu Val Ala Pro Ser Gln Ser
145                 150                 155                 160

Leu Ser Val Thr Cys Thr Val Ser Gly Val Ser Leu Pro Asp Tyr Gly
                165                 170                 175

Val Ser Trp Ile Arg Gln Pro Pro Arg Lys Gly Leu Glu Trp Leu Gly
            180                 185                 190

Val Ile Trp Gly Ser Glu Thr Thr Tyr Tyr Asn Ser Ala Leu Lys Ser

195                 200                 205

Arg Leu Thr Ile Ile Lys Asp Asn Ser Lys Ser Gln Val Phe Leu Lys
    210                 215                 220

Met Asn Ser Leu Gln Thr Asp Asp Thr Ala Ile Tyr Tyr Cys Ala Lys
225                 230                 235                 240

His Tyr Tyr Tyr Gly Gly Ser Tyr Ala Met Asp Tyr Trp Gly Gln Gly
                245                 250                 255

Thr Ser Val Thr Val Ser Ser Gly Gly Gly Glu Ala Ala Ala Lys Glu
            260                 265                 270

Ala Ala Ala Lys Glu Ala Ala Ala Lys Ala Gly Gly Glu Val Gln Leu
        275                 280                 285

Gln Gln Ser Gly Pro Glu Leu Val Lys Pro Gly Ala Ser Met Lys Ile
    290                 295                 300

Ser Cys Lys Ala Ser Gly Tyr Ser Phe Thr Gly Tyr Thr Met Asn Trp
305                 310                 315                 320

Val Lys Gln Ser His Gly Lys Asn Leu Glu Trp Met Gly Leu Ile Asn
                325                 330                 335

Pro Tyr Lys Gly Val Ser Thr Tyr Asn Gln Lys Phe Lys Asp Lys Ala
            340                 345                 350

Thr Leu Thr Val Asp Lys Ser Ser Ser Thr Ala Tyr Met Glu Leu Leu
        355                 360                 365

Ser Leu Thr Ser Glu Asp Ser Ala Val Tyr Tyr Cys Ala Arg Ser Gly
    370                 375                 380

Tyr Tyr Gly Asp Ser Asp Trp Tyr Phe Asp Val Trp Gly Gln Gly Thr
385                 390                 395                 400

Thr Leu Thr Val Phe Ser Gly Gly Gly Gly Ser Gly Gly Gly Gly Ser
                405                 410                 415

Gly Gly Gly Gly Ser Asp Ile Gln Met Thr Gln Thr Thr Ser Ser Leu
            420                 425                 430

Ser Ala Ser Leu Gly Asp Arg Val Thr Ile Ser Cys Arg Ala Ser Gln
        435                 440                 445

Asp Ile Arg Asn Tyr Leu Asn Trp Tyr Gln Gln Lys Pro Asp Gly Thr
    450                 455                 460

Val Lys Leu Leu Ile Tyr Tyr Thr Ser Arg Leu His Ser Gly Val Pro
465                 470                 475                 480

Ser Lys Phe Ser Gly Ser Gly Ser Gly Thr Asp Tyr Ser Leu Thr Ile
                485                 490                 495

Ser Asn Leu Glu Gln Glu Asp Ile Ala Thr Tyr Phe Cys Gln Gln Gly
            500                 505                 510

Asn Thr Leu Pro Trp Thr Phe Ala Gly Gly Thr Lys Leu Glu Ile Lys
        515                 520                 525

<210>  126
<211>  507
<212>  PRT
<213>  artificial sequence

<400>  126

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Gly Gly Gly Gly Ser
            100                 105                 110

Gly Gly Gly Gly Ser Gly Gly Gly Gly Ser Glu Val Gln Leu Val Glu
        115                 120                 125

Ser Gly Gly Gly Leu Val Gln Pro Gly Gly Ser Leu Arg Leu Ser Cys
    130                 135                 140

Ala Ala Ser Gly Phe Asn Ile Lys Asp Thr Tyr Ile His Trp Val Arg
145                 150                 155                 160

Gln Ala Pro Gly Lys Gly Leu Glu Trp Val Ala Arg Ile Tyr Pro Thr
                165                 170                 175

Asn Gly Tyr Thr Arg Tyr Ala Asp Ser Val Lys Gly Arg Phe Thr Ile
            180                 185                 190

Ser Ala Asp Thr Ser Lys Asn Thr Ala Tyr Leu Gln Met Asn Ser Leu
        195                 200                 205

Arg Ala Glu Asp Thr Ala Val Tyr Tyr Cys Ser Arg Trp Gly Gly Asp
    210                 215                 220

Gly Phe Tyr Ala Met Asp Val Trp Gly Gln Gly Thr Leu Val Thr Val
225                 230                 235                 240

Ser Ser Gly Gly Gly Glu Ala Ala Ala Lys Glu Ala Ala Ala Lys Glu
                245                 250                 255

Ala Ala Ala Lys Ala Gly Gly Glu Val Gln Leu Gln Gln Ser Gly Pro
            260                 265                 270

Glu Leu Val Lys Pro Gly Ala Ser Met Lys Ile Ser Cys Lys Ala Ser
        275                 280                 285

Gly Tyr Ser Phe Thr Gly Tyr Thr Met Asn Trp Val Lys Gln Ser His
    290                 295                 300

Gly Lys Asn Leu Glu Trp Met Gly Leu Ile Asn Pro Tyr Lys Gly Val
305                 310                 315                 320

Ser Thr Tyr Asn Gln Lys Phe Lys Asp Lys Ala Thr Leu Thr Val Asp
                325                 330                 335

Lys Ser Ser Ser Thr Ala Tyr Met Glu Leu Leu Ser Leu Thr Ser Glu
            340                 345                 350

Asp Ser Ala Val Tyr Tyr Cys Ala Arg Ser Gly Tyr Tyr Gly Asp Ser
        355                 360                 365

Asp Trp Tyr Phe Asp Val Trp Gly Gln Gly Thr Thr Leu Thr Val Phe
    370                 375                 380

Ser Gly Gly Gly Gly Ser Gly Gly Gly Gly Ser Gly Gly Gly Gly Ser
385                 390                 395                 400

Asp Ile Gln Met Thr Gln Thr Thr Ser Ser Leu Ser Ala Ser Leu Gly
                405                 410                 415

Asp Arg Val Thr Ile Ser Cys Arg Ala Ser Gln Asp Ile Arg Asn Tyr
            420                 425                 430

Leu Asn Trp Tyr Gln Gln Lys Pro Asp Gly Thr Val Lys Leu Leu Ile
        435                 440                 445

Tyr Tyr Thr Ser Arg Leu His Ser Gly Val Pro Ser Lys Phe Ser Gly
    450                 455                 460

Ser Gly Ser Gly Thr Asp Tyr Ser Leu Thr Ile Ser Asn Leu Glu Gln
465                 470                 475                 480

Glu Asp Ile Ala Thr Tyr Phe Cys Gln Gln Gly Asn Thr Leu Pro Trp
                485                 490                 495

Thr Phe Ala Gly Gly Thr Lys Leu Glu Ile Lys
            500                 505

<210>  127
<211>  5
<212>  PRT
<213>  artificial sequence

<400>  127

Gly Tyr Thr Met Asn
1               5

<210>  128
<211>  17
<212>  PRT
<213>  artificial sequence

<400>  128

Leu Ile Asn Pro Tyr Lys Gly Val Ser Thr Tyr Asn Gln Lys Phe Lys
1               5                   10                  15

Asp

<210>  129
<211>  13
<212>  PRT
<213>  artificial sequence

<400>  129

Ser Gly Tyr Tyr Gly Asp Ser Asp Trp Tyr Phe Asp Val

1                   5                   10


<210>  130
<211>  11
<212>  PRT
<213>  artificial sequence

<400>  130

Arg Ala Ser Gln Asp Ile Arg Asn Tyr Leu Asn
1               5                   10


<210>  131
<211>  7
<212>  PRT
<213>  artificial sequence

<400>  131

Tyr Thr Ser Arg Leu His Ser
1               5


<210>  132
<211>  9
<212>  PRT
<213>  artificial sequence

<400>  132

Gln Gln Gly Asn Thr Leu Pro Trp Thr
1               5


<210>  133
<211>  7
<212>  PRT
<213>  artificial sequence

<400>  133

Ser Gly Asp Tyr Tyr Trp Thr
1               5


<210>  134
<211>  16
<212>  PRT
<213>  artificial sequence

<400>  134

His Ile Tyr Tyr Ser Gly Asn Thr Asn Tyr Asn Pro Ser Leu Lys Ser
1               5                   10                  15


<210>  135
<211>  9
<212>  PRT
<213>  artificial sequence

<400>  135

Asp Arg Val Thr Gly Ala Phe Asp Ile
1               5



<210>  136
<211>  11
<212>  PRT
<213>  artificial sequence

<400>  136

Gln Ala Ser Gln Asp Ile Ser Asn Tyr Leu Asn
1               5                   10



<210>  137
<211>  7
<212>  PRT
<213>  artificial sequence

<400>  137

Asp Ala Ser Asn Leu Glu Thr
1               5



<210>  138
<211>  9
<212>  PRT
<213>  artificial sequence

<400>  138

Gln His Phe Asp His Leu Pro Leu Ala
1               5



<210>  139
<211>  5
<212>  PRT
<213>  artificial sequence

<400>  139

Asp Thr Tyr Ile His
1               5



<210>  140
<211>  17
<212>  PRT
<213>  artificial sequence

<400>  140

Arg Ile Tyr Pro Thr Asn Gly Tyr Thr Arg Tyr Ala Asp Ser Val Lys
1               5                   10                  15


Gly

<210>  141
<211>  11
<212>  PRT
<213>  artificial sequence

<400>  141

Trp Gly Gly Asp Gly Phe Tyr Ala Met Asp Tyr
1               5                   10


<210>  142
<211>  11
<212>  PRT
<213>  artificial sequence

<400>  142

Arg Ala Ser Gln Asp Val Asn Thr Ala Val Ala
1               5                   10


<210>  143
<211>  7
<212>  PRT
<213>  artificial sequence

<400>  143

Ser Ala Ser Phe Leu Tyr Ser
1               5


<210>  144
<211>  14
<212>  PRT
<213>  artificial sequence

<400>  144

Gln Gln His Tyr Thr Thr Pro Pro Thr Phe Gly Gln Gly Thr
1               5                   10


<210>  145
<211>  129
<212>  DNA
<213>  artificial sequence

<400>  145
agtaagagga gcaggctcct gcacagtgac tacatgaaca tgactccccg ccgccccggg      60

cccacccgca agcattacca gccctatgcc ccaccacgcg acttcgcagc ctatcgctcc     120

ggcggaggg                                                             129


<210>  146
<211>  43
<212>  PRT
<213>  artificial sequence

<400>  146

Ser Lys Arg Ser Arg Leu Leu His Ser Asp Tyr Met Asn Met Thr Pro
1               5                   10                  15

Arg Arg Pro Gly Pro Thr Arg Lys His Tyr Gln Pro Tyr Ala Pro Pro
            20                  25                  30

Arg Asp Phe Ala Ala Tyr Arg Ser Gly Gly Gly
        35                  40

<210>  147
<211>  162
<212>  DNA
<213>  artificial sequence

<400>  147
cacctttgga gaagacagtg gcgaccaaga cgattttctg ccttagagca agggattcac      60

cctccgcagg ctcagagcaa gatagaggag ctggagcaag aaccggagcc ggagccggag     120

ccggaaccgg agcccgagcc cgagcccgag ccggagcagc tc                        162

<210>  148
<211>  54
<212>  PRT
<213>  artificial sequence

<400>  148

His Leu Trp Arg Arg Gln Trp Arg Pro Arg Arg Phe Ser Ala Leu Glu
1               5                   10                  15

Gln Gly Ile His Pro Pro Gln Ala Gln Ser Lys Ile Glu Glu Leu Glu
            20                  25                  30

Gln Glu Pro Glu Pro Glu Pro Glu Pro Glu Pro Glu Pro Glu Pro Glu
        35                  40                  45

Pro Glu Pro Glu Gln Leu
    50

<210>  149
<211>  10
<212>  DNA
<213>  artificial sequence

<400>  149
gggaatttcc                                                              10

<210>  150
<211>  11
<212>  DNA

<213>  artificial sequence

<400>  150
ggggactttc c                                                            11


<210>  151
<211>  52
<212>  DNA
<213>  artificial sequence

<400>  151
gggaatttcc ggggactttc cgggaatttc cggggacttt ccgggaattt cc               52


<210>  152
<211>  68
<212>  DNA
<213>  artificial sequence

<400>  152
ggggactttc cgcttgggga ctttccgctg gggactttcc gctggggact ttccgctggg       60

gactttcc                                                                68


<210>  153
<211>  32
<212>  DNA
<213>  artificial sequence

<400>  153
tagagggtat ataatggaag ctcgacttcc ag                                     32


<210>  154
<211>  46
<212>  DNA
<213>  artificial sequence

<400>  154
gggtcgaggt aggcgtgtac ggtgggaggc ctatataagc agagct                      46


<210>  155
<211>  182
<212>  DNA
<213>  artificial sequence

<400>  155
agcttgaata aaatgaatat tagaagctgt tagaataaga gaaaatgaca gaggaaaact       60

gaaagggaga actgaaagtg ggaaattcct ctgaggcaga aaggaccatc ccttataaat      120

agcacaggcc atgaaggaag atcattctca ctgcagcctt tgacagcctt tgcctcatct      180

tg                                                                     182


<210>  156
<211>  256
<212>  DNA
<213>  artificial sequence

<400> 156
ggggactttc cgcttgggga ctttccgctg gggactttcc gctggggact ttccgctggg 60

gactttccga attcagcttg aataaaatga atattagaag ctgttagaat aagagaaaat 120

gacagaggaa aactgaaagg gagaactgaa agtgggaaat tcctctgagg cagaaaggac 180

catcccttat aaatagcaca ggccatgaag gaagatcatt ctcactgcag cctttgacag 240

cctttgcctc atcttg 256